(19) Europäisches Patentamt
European Patent Office
Office européen des brevets

(11) EP 1 482 033 A1

(12) # EUROPEAN PATENT APPLICATION
published in accordance with Art. 158(3) EPC

(43) Date of publication:
**01.12.2004 Bulletin 2004/49**

(21) Application number: **03707055.4**

(22) Date of filing: **25.02.2003**

(51) Int Cl.$^7$: **C12N 15/09**, C12N 9/42, C12P 1/02, D21C 3/00

(86) International application number:
**PCT/JP2003/002058**

(87) International publication number:
**WO 2003/070940 (28.08.2003 Gazette 2003/35)**

(84) Designated Contracting States:
**AT BE BG CH CY CZ DE DK EE ES FI FR GB GR HU IE IT LI LU MC NL PT SE SI SK TR**
Designated Extension States:
**AL LT LV MK RO**

(30) Priority: **25.02.2002 JP 2002048675**

(71) Applicant: **Oji Paper Co., Ltd.**
**Tokyo 104-0061 (JP)**

(72) Inventors:
- **TSUKAMOTO, Akira**
  **Edogawa-ku, Tokyo 134-0088 (JP)**
- **NAKAGAME, Seiji**
  **Chiba-shi, Chiba 261-0001 (JP)**
- **KABUTO, Mari**
  **Chiba-shi, Chiba 261-0001 (JP)**
- **SUGIURA, Jun**
  **Kawasaki-shi, Kanagawa 216-0033 (JP)**
- **SAKAGUCHI, Hisako**
  **Chiba-shi, Chiba 261-0001 (JP)**
- **FURUJYO, Atsushi**
  **Suzuka-shi, Mie 513-0806 (JP)**

(74) Representative: **Schrell, Andreas, Dr. et al**
**Gleiss & Grosse**
**Leitzstrasse 45**
**70469 Stuttgart (DE)**

(54) **CELLULOSE DIGESTING ENZYME GENE AND UTILIZATION OF THE GENE**

(57) A method for treating woodchips, comprising the steps of: preparing a DNA encoding an antisense RNA substantially complementary to the whole or a part of a transcription product of a cellulolytic enzyme gene derived from Basidiomycete; preparing a vector comprising (a) the above DNA, or (b) a recombinant DNA comprising the above DNA and a DNA fragment having a promoter activity, wherein the above DNA binds to the above DNA fragment such that an antisense RNA of the cellulolytic enzyme gene is generated as a result of transcription; transforming host cells with the above vector, so as to prepare the host cells having a suppressed cellulolytic enzyme activity; and inoculating the above host cells having a suppressed cellulolytic enzyme activity into woodchips to treat them.

EP 1 482 033 A1

## Description

Technical Field

The present invention relates to a method for treating woodchips using an antisense gene of a gene encoding cellulolytic enzyme.

Background Art

Pulp produced in the paper and pulp industry is divided into mechanical pulp and chemical pulp in terms of a production method thereof.

Mechanical pulp is produced by physically grinding wood fibers with mechanical energy. Since such mechanical pulp contains almost all wood components, it can be produced at a high yield, and thereby, a thin paper with high opacity can be produced. However, mechanical pulp has disadvantages that a high electric power is required for grinding and that the produced paper hardly has paper strength.

Microorganisms, which solve the above problems and do not decrease pulp yield, have widely been screened. For example, there has been a report that when alder first refined thermomechanical pulp (TMP) was treated with a Basidiomycete, *Phanerochaete chrysosporium*, in the presence of glucose, and it was then subjected to the second refining, energy necessary for refining was reduced by 25% to 30% (Bar-Lev and T. K. Kirk, Tappi J., 65, 111, 1982). Moreover, when aspen lumbers were treated with *Phanerochaete chrysosporium* and *Dichomitus squalens*, the obtained paper had paper strength higher than that of a control (Myers., Tappi J., 105, 1988). Akamathu et al. have cultured 10 strains of white-rot fungi including *Coriolus hirsutus* on poplar lumbers, so as to examine pulp yield, refining energy, and pulp strength. As a result, they have found that, among the used strains, *Coriolus hirsutus* was preferable as a fungus used in pretreatment of chips because this fungus caused decrease in refining energy and increase in the degree of crystallinity. However, at the same time, *Coriolus hirsutus* caused a yield of about 7% (Akamathu et al., Mokuzai gakkaishi, 30 (8) 697-702, 1984). Moreover, using 10 types of white-rot fungi preliminarily selected from 61 types (85 strains) of white-rot fungi, Nishibe et al. have microbially decomposed secondary refined TMP from *Pterocarya rhoifolia* wood pieces and softwood. Thereafter, they have selectively performed delignification. They have selected *Coprinus cinereus* and *Phanerochaete chrysosporium*, which caused less degradation of pulp fibers, and they have shown that these fungi could control reduction in paper strength in the presence of glucose and urea. However, when pulp was treated with the above fungi at 30°C for 14 days, decreases in pulp yield were 6.3% and 9.7%, respectively. When *Coriolus hirsutus* was used, decrease in yield was 7.5% (Nishibe et al., Japan Tappi, 42 (2), 1988). Kashino et al. have screened a white-rot fungus IZU-154 from the nature, and lignin has been selectively decomposed with *Phanerochaete chrysosporium* or *Trametes versicolor.* They have confirmed that when a hardwood tree was thus treated for 7 days, refining energy was decreased by 1/2 to 2/3. In addition, they have also confirmed that pulp strength was approximately two times increased. When a softwood was treated for 10 to 14 days, refining energy was decreased by 1/3, and increase in pulp strength was also observed. When a medium was added, the same results could be obtained for 7 days (Kashino et al., Tappi J., 76 (12), 167, 1993). Furthermore, recently, a lignin consortium consisting of research institutes and several pulp and paper companies, including USDA Forest Products Laboratory as a center, has been established in the U.S.A. The lignin consortium has screened a strain, which has a high ability to decompose lignin but a low ability to decompose cellulose, and as a result, the consortium has newly isolated *Ceriporiopsis subvermispora* from the nature. The consortium has studied reduction in a power used for mechanical pulp, using this strain. The consotium has reported that this strain enabled reduction in nearly 40% of the energy necessary for producing TMP, for example, and that decrease in yield was approximately 3% to 5% in this case. The consortium has also reported that there were no feared adverse effects on paper strength, but that such strength rather increased. The USDA Forest Products Laboratory has already constructed a pilot plant, and verification tests of the isolated *Ceriporiopsis subvermispora* are carried out therein. Assuming the U.S. plants, it is also considered that a treatment with microorganisms is carried out in a chip yard in a plant (Scott et al., Tappi J., 81. 12. 153, 1998). In such a case, since the inside of a pile conserving chips has a high temperature, strains having effects even at a high temperature are required. However, since the isolated *Ceriporiopsis subvermispora* has effects only at a temperature of 32°C or lower, this fungus is inadequate for practical use.

In addition, microorganisms that have been obtained by the previous screening do not necessarily have high selectivity with respect to lignin decomposition. Since they decompose not only lignin but also cellulose, they result in decrease in pulp yield or paper strength. Thus, it is further desired to obtain or produce microorganisms having enhanced selectivity with respect to lignin decomposition, that is, microorganisms having a suppressed ability to decompose cellulose as well as having an excellent ability to decompose lignin.

Ander et al. have produced a mutant having enhanced selectivity with respect to lignin decomposition. They have introduced a mutation in *Sporotrichum pulverulentum* by UV radiation, so that they have developed a strain Ce144 having low cellulase activity. Birch wood pieces were decomposed by both a wild type strain and the above cellulase-deficient Cel44. As a result, it was found that the former decomposed well lignin and xylan, whereas the latter decomposed well lignin and xylan but hardly decomposed glucan (Ander and Eriksson, Svensk Papperstidning, 18, 643, 1975). Birch lumbers were treated with this Ce144 for 6 weeks, and thereafter, mechanical pulp was produced therefrom. As a result, it was found that paper strength was increased (Ander and Eriksson, Svensk Papperetidning, 18, 641, 1975). Moreover, there has been a report that when an experiment was carried out using birch lumbers and pine lumbers, energy necessary for fibrillation and refining of fibers was 30% reduced by increasing treating time (Eriksson and Vallander., Svensk Papperstid, 85, R33, 1982). They have also produced Ce126 with a low cellulase activity from *Phlebia radiata*. Chips and pulp made from pine lumbers were treated with this Ce126, and mechanical pulp was then produced therefrom. As a result, it was found that paper strength was not improved in both cases but that decrease in refining energy was observed. In addition, reduction in the weight was 2% or less (Samuelsson et al., Svensk Papperstid, 8, 221, 1980).

As stated above, mutant strains with a low cellulose activity have been produced, and the use of such mutant strains in a treatment of mechanical pulp has been considered. However, since these mutant strains are mutagenetically treated by ultraviolet radiation, they have problems that their growth rate is slow and it takes a long time to decompose pulp. Accordingly, it is desired to produce a mutant strain, which has a normal growth rate, but has only its cellulolytic activity of which is suppressed.

On the other hand, chemical pulp is obtained by a production method comprising solving lignin from lumbers using chemicals, so as to obtain cellulose and hemicellulose. At present, Kraft pulp involving delignification with sodium hydroxide and sodium sulfate has become mainstream. As with mechanical pulp, Kraft pulp is also treated with microorganisms and subjected to delignification before cooking, whereby reduction of production energy and improvement of quality of pulp are attempted.

For example, there has been a report that when red oak lumbers or aspen lumbers are treated with *Phanerochaete chrysosporium* for 30 days, yield is improved at the same Ka value and beating energy is thereby reduced, and tensile strength and bursting strength are increased (Oriaran et al., Tappi, 73, 147, 1990). Likewise, increase in bursting strength and tearing strength has been reported from an experiment using *Phanerochaete chrysosporium* (Chen et al., Wood Fiber Sci., 27. 198, 1995). Molina et al. have reported that when radiata pine was treated with *Trametes versicolor* and *Pleurotus ostreatus*, production energy could be 11% to 14% reduced. In the case of *Trametes versicolor,* however, decrease in pulp strength was observed (Molina, 50th Appita Annual General Conference, pp. 57-63, 1996; Molina, 51st Annual General Conference, pp. 199-206). Bajpai et al. have reported from an experiment using *Ceriporiopsis subvermispora* that using the microorganism, active alkali could be reduced by 18%, cooking time could be reduced by 33%, and sulfur degree in white liquor could be reduced by 30% (P. Bajpai et al. J. Pulp and Paper Science: 27 (7), 235-239,2001).

As stated above, a treatment of Kraft pulp with microorganisms improves cooking, thereby resulting in reduction in energy. However, in some cases, such a treatment with microorganisms might decrease yield or paper strength. Accordingly, as in the case of mechanical pulp, for the practical use of a treatment with microorganisms, achievement or production of microorganisms with enhanced selectivity with respect to lignin decomposition is required, which has an excellent ability to decompose lignin but has a suppressed ability to decompose cellulose.

Among such cellulolytic enzymes (cellulose-decomposing enzymes), an enzyme generically called cellulase hydrolyzes the bond of β-1,4-glucan (cellulose) or a derivative thereof to β-1,4-glucopyranosyl. This enzyme is widely distributed in higher plants, microorganisms such as fungi or bacteria, mollusks, etc. It has been known that cellulase is broadly divided into what is called endoglucanase (CMCase) hydrolyzing the β-1,4-glucopyranosyl bond of a cellulose main chain in an endo-manner, and into what is called exoglucanase (avicelase) eliminating mainly cellobiose residues from the terminus of a cellulose main chain. These hydrolytic enzymes synergistically act on cellulose, so that the cellulose substrate reduces its molecular weight to generate cellobiose, and further, due to involvement of β-glucosidase, it is decomposed into glucose units.

Moreover, cellobiose dehydrogenase is oxidoreductase, which oxidizes cellobiose or cellooligosaccharide to generate cellobionolactone and, at the same time, also reduces quinone, metal complexes of such as iron, phenoxy radical, or oxygen. This enzyme is generated at the same time with cellulase when microorganisms decompose cellulose (Eriksson et al., FEBS Lett., 49, 282-285, 1974). In addition, this enzyme releases the inhibition of cellulase activity due to cellobiose, that is, inhibition of products due to cellobiose (Igarashi et al., Eur. J. Biochem., 253, 101, 1998). From these facts, it is considered that cellobiose dehydrogenase is conjugated with cellulase to promote decomposition of cellulose. Furthermore, since cellobiose dehydrogenase causes a Fenton reaction, which generates a hydroxyl radical strongly decomposing cellulose, it is considered that this enzyme is highly associated with decomposition of cellulose. This inference is supported also from the production of mutant strains having suppressed cellobiose dehydrogenase activity by Dumonceaux et al., and analysis results of the properties thereof. Dumonceaux et al. have produced a cellobiose dehydrogenase-deficient strain by homologous recombination, using an antibiotic ferromycin as an index. They have reported that the growth rate of the cellobiose dehydrogenase-deficient strain is almost the same as that of a wild type stain when amorphous cellulose is used as a carbon source, but that the growth rate of the cellobiose dehydrogenase-deficient strain becomes significantly slow when it is cultured on crystalline cellulose, and that the cellobiose dehydrogenase-deficient strain decomposes lignin contained in unbleached pulp from broad-leaved trees or synthetic lignin $^{14}$C-DHP at a level equivalent to a wild type strain (Dumonceaux, Enzyme and Microb. 29, 478-489, 2001).

According to the general introduction regarding cellobiose dehydrogenase or the like (G. Henriksson et al., J. Biotechnol. 78 (2000) 93-113), examples of microorganisms producing this enzyme may include wood rotting fungi such as *Phanerochaete chrysosporium*, *Trametes versicolor, Schizophyllum commune, Coneophora puteana*, *Myceliophtore thermophila*, or *Fumicola insolens.*

In addition, with regard to a gene encoding cellobiose dehydrogenase (hereinafter referred to as a cellobiose dehydrogenase gene), in the case of *Phanerochaete chrysosporium* for example, cDNA of the K3 strain (Raices et al., FEBS Letters, 69, 233-238, 1995), and the cDNA (Li et al., Appl. Environ. Microbiol., 62(4), 1329-1335, 1996) and chromosomal DNA of the OGC101 strain have been cloned (Li et al. Appl. Environ. Microbiol., 63(2), 796-799, 1997). Moreover, with regard to *Trametes versicolor* (T. J. Dumonceaux et al., Gene. 210. 211-219 (1998)) and *Pycnoporus cinnabarinus* (S. M. Moukha et al., Gene, 234, 23-33, 1999) also, the presence of cellobiose dehydrogenase genes has been reported.

As stated above, cellobiose dehydrogenase and genes encoding the enzyme have been disclosed. However, there have been no reports regarding clarification of cellulolytic enzymes including cellobiose dehydrogenase derived from *Coriolus hirsutus* as a typical example, or cellulolytic enzyme genes, that are necessary for achievement or production of microorganisms with enhanced selectivity with respect to lignin decomposition, used in a treatment with microorganisms in the production of mechanical pulp or chemical pulp, and regarding genetic recombination techniques of applying the above genes. Further, no effective methods for treating pulp using a transformant obtained by such genetic recombination have been disclosed.

On the other hand, use of cellulolytic enzymes has attracted keen interest for a long time. For example, use of cellulolytic enzymes in various ways, such as addition of the enzymes into household detergents, reforming of cellulose polymeric materials such as fibers due to surface treatment with the enzymes, deinking treatment from waste papers, or food processing, has been studied. Thus, a method for producing a large quantity of cellulolytic enzyme has been required. As an attempt to produce a large quantity of cellobiose dehydrogenase, there has been a report that D-glycerylaldehyde-3-phosphate dehydrogenase as a structurally functioning promoter was ligated upstream of the cellobiose dehydrogenase-1 gene of *Phanerochaete chrysosporium* (Li., Biochem. Biophys. Res. Commun., 270, 141-146, 2000). However, since *Phanerochaete chrysosporium* is designated as a destructive fungus in Japan, it cannot be used. Hence, it is desired to develop a technique of producing a large quantity of cellulolytic enzymes including cellobiose dehydrogenase as a typical example, using harmless microorganisms, but so far there have been no reports regarding the above techniques of using cellulolytic enzyme genes such as a cellobiose dehydrogenase gene derived from *Coriolus hirsutus.*

Disclosure of the Invention

It is an object of the present invention to provide a method for treating woodchips using an antisense gene of a gene encoding cellulolytic enzyme.

In order to solve the above-described problems, the present inventors have intensively studied and widely screened for fungi producing cellulolytic enzymes including cellobiose dehydrogenase as a typical example. As a result, they have found that *Coriolus hirsutus* produces cellulolytic enzymes. Moreover, the present inventors have also succeeded in cloning genes encoding such cellulolytic enzymes. Furthermore, they have developed a method for controlling the expression of the above gene using an antisense gene thereof. They have produced pulp from woodchips according to the above method and have succeeded in controlling yield or reduction in paper strength, thereby completing the present invention.

That is to say, the present invention provide the following features:

(1) A method for treating woodchips, comprising the steps of:

preparing a DNA encoding an antisense RNA substantially complementary to the whole or a part of a transcription product of a cellulolytic enzyme gene derived from Basidiomycete;
preparing a vector comprising (a) the above DNA, or (b) a recombinant DNA comprising the above DNA and a DNA fragment having a promoter activity, wherein the above DNA binds to the above DNA fragment such that an antisense RNA of the cellulolytic enzyme gene is generated as a result of transcription;
transforming host cells with the above vector, so as to prepare the host cells having a suppressed cellulolytic enzyme activity; and

inoculating the above host cells having a suppressed cellulolytic enzyme activity into woodchips to treat them.

(2) The method according to (1), wherein the cellulolytic enzyme gene comprises one or more genes selected from the group consisting of respective genes encoding a cellobiose dehydrogenase, cellobiohydrolase I, cellobiohydrolase II, an endoglucanase belonging to glycolytic enzyme family 61, an endoglucanase belonging to glycolytic enzyme family 12, an endoglucanase belonging to glycolytic enzyme family 5, and an endoglucanase belonging to glycolytic enzyme family 9.

(3) The method according to (2), wherein the cellobiose dehydrogenase gene is an isolated cellobiose dehydrogenase gene comprising any one of the following nucleotide sequences (a) to (c):

(a) a nucleotide sequence as shown in SEQ ID No. 1 or 3;
(b) a nucleotide sequence hybridizing with a nucleotide sequence comprising a nucleotide sequence complementary to the nucleotide sequence according to (a) under stringent conditions, and encoding a protein having a cellobiose dehydrogenase enzyme activity; and
(c) a nucleotide sequence comprising deletion, substitution or addition of one or more nucleotides with respect to SEQ ID No. 1 or 3, and encoding a protein having a cellobiose dehydrogenase enzyme activity.

(4) The method according to (2), wherein the cellobiohydrolase I gene is an isolated cellobiohydrolase I gene comprising any one of the following nucleotide sequences (a) to (c):

(a) a nucleotide sequence as shown in SEQ ID No. 7, 9, or 11;
(b) a nucleotide sequence hybridizing with a nucleotide sequence comprising a nucleotide sequence complementary to the nucleotide sequence according to (a) under stringent conditions, and encoding a protein having a cellobiohydrolase I gene enzyme activity; and
(c) a nucleotide sequence comprising deletion, substitution or addition of one or more nucleotides with respect to SEQ ID No. 7, 9 or 11, and encoding a protein having a cellobiohydrolase I gene enzyme activity.

(5) The method according to (2), wherein the cellobiohydrolase II gene is an isolated cellobiohydrolase II gene comprising any one of the following nucleotide sequences (a) to (c):

(a) a nucleotide sequence as shown in SEQ ID No. 14;
(b) a nucleotide sequence hybridizing with a nucleotide sequence comprising a nucleotide sequence complementary to the nucleotide sequence according to (a) under stringent conditions, and encoding a protein having a cellobiohydrolase II gene enzyme activity; and
(c) a nucleotide sequence comprising deletion, substitution or addition of one or more nucleotides with respect to SEQ ID No. 14, and encoding a protein having a cellobiohydrolase II gene enzyme activity.

(6) The method according to (2), wherein the endoglucanase gene belonging to glycolytic enzyme family 61 is an isolated endoglucanase gene belonging to glycolytic enzyme family 61 comprising any one of the following nucleotide sequences (a) to (c):

(a) a nucleotide sequence as shown in SEQ ID No. 18;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 61; and
(c) a nucleotide sequence comprising deletion, substitution or addition of one or more nucleotides with respect to SEQ ID No. 18, and encoding a protein having an activity of endoglucanase enzyme belonging to glycolytic enzyme family 61.

(7) The method according to (2), wherein the endoglucanase gene belonging to glycolytic enzyme family 12 is an isolated endoglucanase gene belonging to glycolytic enzyme family 12 comprising any one of the following nucleotide sequences (a) to (c):

(a) a nucleotide sequence as shown in SEQ ID No. 20;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 12; and
(c) a nucleotide sequence comprising deletion, substitution or addition of one or more nucleotides with respect to SEQ ID No. 20, and encoding a protein having an activity of endoglucanase enzyme belonging to glycolytic enzyme family 12.

(8) The method according to (2), wherein the endoglucanase gene belonging to glycolytic enzyme family 5 is an isolated endoglucanase gene belonging to glycolytic enzyme family 5 comprising any one of the following nucleotide sequences (a) to (c):

(a) a nucleotide sequence as shown in SEQ ID No. 24;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 5; and
(c) a nucleotide sequence comprising deletion, substitution or addition of one or more nucleotides with respect to SEQ ID No. 24, and encoding a protein having an activity of endoglucanase enzyme belonging to glycolytic enzyme family 5.

(9) The method according to (2), wherein the endoglucanase gene belonging to glycolytic enzyme family 9 is an isolated endoglucanase gene belonging to glycolytic enzyme family 9 comprising any one of the following nucleotide sequences (a) to (c):

(a) a nucleotide sequence as shown in SEQ ID No. 28;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 9; and
(c) a nucleotide sequence comprising deletion, substitution or addition of one or more nucleotides with respect to SEQ ID No. 28, and encoding a protein having an activity of endoglucanase enzyme belonging to glycolytic enzyme family 9.

(10) The method according to any one of (1) to (9), wherein Basidiomycete is *Coriolus hirsutus* or *Phanerochaete chrysosporium*.
(11) The method according to any one of (1) to (10), wherein host cells are *Coriolus hirsutus.*
(12) A woodchip obtained by the method according to any one of (1) to (11).
(13) A method for producing a pulp using the woodchip according to (12).
(14) A pulp obtained by the method according to (13).

This specification includes part or all of the contents as disclosed in the specification and/or drawings of Japanese Patent Application No. 2002-48675, which is a priority document of the present application.

Brief Description of the Drawings

Figure 1 is a view showing analysis results of a reaction of acting the cellobiose dehydrogenase of the present invention on cellobiose;
Figure 2 is a view showing optimal pH of the cellobiose dehydrogenase of the present invention. In the figure, ♦ represents glycine-HCl, ■ represents acetic acid, and ▲ represents phosphoric acid;
Figure 3 is a view showing pH stability of the cellobiose dehydrogenase of the present invention. In the figure, ♦ represents glycine-HCl, ■ represents acetic acid, and ▲ represents phosphoric acid;
Figure 4 is a view showing an optimal reaction temperature of the cellobiose dehydrogenase of the present invention; and
Figure 5 is a view showing heat stability of the cellobiose dehydrogenase of the present invention.

Description of Sequence Listing

SEQ ID No. 5 is a probe used in plaque hybridization.
SEQ ID No. 6 is a probe used in plaque hybridization.
SEQ ID No. 13 is a probe used in plaque hybridization.

SEQ ID No. 16 is a primer used in a PCR reaction.
SEQ ID No. 17 is a primer used in a PCR reaction.
SEQ ID No. 22 is a primer used in a PCR reaction.
SEQ ID No. 23 is a primer used in a PCR reaction.
SEQ ID No. 26 is a primer used in a PCR reaction.
SEQ ID No. 27 is a primer used in a PCR reaction.
SEQ ID No. 30 is a primer used in a PCR reaction.
SEQ ID No. 31 is a primer used in a PCR reaction.
SEQ ID No. 32 is a primer used in a PCR reaction.
SEQ ID No. 33 is a primer used in a PCR reaction.
SEQ ID No. 34 is a primer used in a PCR reaction.
SEQ ID No. 35 is a primer used in a PCR reaction.
SEQ ID No. 36 is a primer used in a PCR reaction.
SEQ ID No. 37 is a primer used in a PCR reaction.
SEQ ID No. 38 is a primer used in a PCR reaction.
SEQ ID No. 39 is a primer used in a PCR reaction.
SEQ ID No. 40 is a primer used in a PCR reaction.
SEQ ID No. 41 is a primer used in a PCR reaction.
SEQ ID No. 42 is a primer used in a PCR reaction.
SEQ ID No. 43 is a primer used in a PCR reaction.
SEQ ID No. 44 is a primer used in a PCR reaction.
SEQ ID No. 45 is a primer used in a PCR reaction.
SEQ ID No. 46 is a primer used in a PCR reaction.
SEQ ID No. 47 is a primer used in a PCR reaction.
SEQ ID No. 48 is a primer used in a PCR reaction.
SEQ ID No. 49 is a primer used in a PCR reaction.
SEQ ID No. 50 is a primer used in a PCR reaction.
SEQ ID No. 51 is a primer used in a PCR reaction.
SEQ ID No. 52 is a primer used in a PCR reaction.

Mode for Carrying out the Invention

The present invention will be described further in detail below.

The present invention provides a method for treating woodchips, which comprises the steps of: preparing DNA encoding antisense RNA substantially complementary to the whole or a part of a transcription product of a cellulolytic enzyme gene derived from Basidiomycete; preparing a vector comprising (a) the above DNA, or (b) recombinant DNA comprising the above DNA and a DNA fragment having a promoter activity, wherein the above DNA binds to the above DNA fragment such that antisense RNA of a cellulolytic enzyme gene is generated as a result of transcription; performing transformation with the above vector, so as to prepare host cells having a suppressed cellulolytic enzyme activity; and inoculating the above host cells having a suppressed cellulolytic enzyme activity into woodchips, thereby treating them.

In the present specification, any type of Basidiomycete can be used, as long as it has an ability to decompose cellulose. In particular, *Coriolus hirsutus* whose Japanese name is Aragekawaratake is preferable.

Moreover, a cellulolytic enzyme gene is not particularly limited, as long as the enzyme can decompose cellulose. Preferred examples of such a cellulolytic enzyme gene may include a cellobiose dehydrogenase gene, a cellobiohydrolase I gene, a cellobiohydrolase II gene, and an endoglucanase gene. More specifically, various genes described in 1 to 7 below can be used. It is to be noted that these genes can be used singly, but they may be used in combination of one or more types.

1. An isolated cellobiose dehydrogenase gene comprising any one of the following nucleotide sequences (a) to (c):

   (a) a nucleotide sequence as shown in SEQ ID No. 1 or 3;
   (b) a nucleotide sequence hybridizing with a nucleotide sequence having a nucleotide sequence complementary to the nucleotide sequence according to (a) under stringent conditions, and encoding a protein having a cellobiose dehydrogenase enzyme activity; and
   (c) a nucleotide sequence comprising deletion, substitution or addition of one or more nucleotides with respect to SEQ ID No. 1 or 3, and encoding a protein having a cellobiose dehydrogenase enzyme activity.

2. An isolated cellobiohydrolase I gene comprising any one of the following nucleotide sequences (a) to (c):

(a) a nucleotide sequence as shown in SEQ ID No. 7, 9, or 11;
(b) a nucleotide sequence hybridizing with a nucleotide sequence having a nucleotide sequence complementary to the nucleotide sequence according to (a) under stringent conditions, and encoding a protein having a cellobiohydrolase I gene enzyme activity; and
(c) a nucleotide sequence comprising deletion, substitution or addition of one or more nucleotides with respect to SEQ ID No. 7, 9 or 11, and encoding a protein having a cellobiohydrolase I gene enzyme activity.

3. An isolated cellobiohydrolase II gene comprising any one of the following nucleotide sequences (a) to (c):

(a) a nucleotide sequence as shown in SEQ ID No. 14;
(b) a nucleotide sequence hybridizing with a nucleotide sequence having a nucleotide sequence complementary to the nucleotide sequence according to (a) under stringent conditions, and encoding a protein having a cellobiohydrolase II gene enzyme activity; and
(c) a nucleotide sequence comprising deletion, substitution or addition of one or more nucleotides with respect to SEQ ID No. 14, and encoding a protein having a cellobiohydrolase II gene enzyme activity.

4. An isolated endoglucanase gene belonging to glycolytic enzyme family 61 comprising any one of the following nucleotide sequences (a) to (c):

(a) a nucleotide sequence as shown in SEQ ID No. 18;
(b) a nucleotide sequence hybridizing with a nucleotide sequence having a nucleotide sequence complementary to the nucleotide sequence according to (a) under stringent conditions, and encoding a protein having an activity of endoglucanase enzyme belonging to glycolytic enzyme family 61; and
(c) a nucleotide sequence comprising deletion, substitution or addition of one or more nucleotides with respect to SEQ ID No. 18, and encoding a protein having an activity of endoglucanase enzyme belonging enzyme to glycolytic enzyme family 61.

5. An isolated endoglucanase gene belonging to glycolytic enzyme family 12 comprising any one of the following nucleotide sequences (a) to (c):

(a) a nucleotide sequence as shown in SEQ ID No. 20;
(b) a nucleotide sequence hybridizing with a nucleotide sequence having a nucleotide sequence complementary to the nucleotide sequence according to (a) under stringent conditions, and encoding a protein having an activity of endoglucanase enzyme belonging to glycolytic enzyme family 12; and
(c) a nucleotide sequence comprising deletion, substitution or addition of one or more nucleotides with respect to SEQ ID No. 20, and encoding a protein having an activity of endoglucanase enzyme belonging to glycolytic enzyme family 12.

6. An isolated endoglucanase gene belonging to glycolytic enzyme family 5 comprising any one of the following nucleotide sequences (a) to (c):

(a) a nucleotide sequence as shown in SEQ ID No. 24;
(b) a nucleotide sequence hybridizing with a nucleotide sequence having a nucleotide sequence complementary to the nucleotide sequence according to (a) under stringent conditions, and encoding a protein having an activity of endoglucanase enzyme belonging to glycolytic enzyme family 5; and
(c) a nucleotide sequence comprising deletion, substitution or addition of one or more nucleotides with respect to SEQ ID No. 24, and encoding a protein having an activity of endoglucanase enzyme belonging to glycolytic enzyme family 5.

7. An isolated endoglucanase gene belonging to glycolytic enzyme family 9 comprising any one of the following nucleotide sequences (a) to (c):

(a) a nucleotide sequence as shown in SEQ ID No. 28;
(b) a nucleotide sequence hybridizing with a nucleotide sequence having a nucleotide sequence complementary to the nucleotide sequence according to (a) under stringent conditions, and encoding a protein having an activity of endoglucanase enzyme belonging to glycolytic enzyme family 9; and

(c) a nucleotide sequence comprising deletion, substitution or addition of one or more nucleotides with respect to SEQ ID No. 28, and encoding a protein having an activity of endoglucanase enzyme belonging to glycolytic enzyme family 9.

The cellobiose dehydrogenase gene described in 1. above can be obtained by the following procedure.

Chromosomal DNA is prepared from *Coriolus hirsutus* by a common method of extracting chromosomal DNA, such as the method of Yelton et al. (Proc. Natl. Acad. Sci. USA, 81, 1470 (1984)). Subsequently, the obtained chromosomal DNA is treated with suitable restriction enzymes such as *Sau*3AI for partial decomposition, and the resultant product is fractionated by sucrose density gradient ultracentrifugation, so as to obtain DNA fragments with a size from 10 kbp to 25 kbp. The thus obtained DNA fragment is ligated to phage DNA, which has been treated with restriction enzymes generating the same cohesive termini. EMBL3 (A-M, Frishauf et al., J. Mol. Biol. 170, 827 (1983)) λ phage DNA is an example of such phage DNA. The obtained DNA fragment-ligated phage is subjected to *in vitro* packaging, and the resultant product is used as a chromosomal DNA library. For subcloning, a commonly used cloning vector, and preferably an *Escherichia coli* vector can be used. For example, a pUC plasmid such as pUC18 (C.Yanisch-Perron, et al., Gene, 33, 103 (1985)) can be used. A cloning vector is not limited to the above example, but commercially available cloning vector or known vectors described in publications can also be used.

In order to isolate a cellobiose dehydrogenase gene from the above-described chromosomal gene library, cellobiose dehydrogenase which has been obtained from *Coriolus hirsutus* and purified, is completely digested with lysyl endopeptidase, and the digest is then subjected to amino acid sequencing. Thereafter, using synthetic DNA probes produced based on a nucleotide sequence estimated from the obtained amino acid sequence, plaque hybridization is carried out, so as to select clones containing cellobiose dehydrogenase genes. A DNA fragment containing a cellobiose dehydrogenase gene is isolated from the selected clones. Thereafter, a restriction map thereof is prepared, and a sequence thereof is determined. The sequence can be determined by inserting the above fragment containing a cellobiose dehydrogenase gene into a suitable cloning vector (e.g., a pUC vector such as pUC19), and then applying the method of Sanger et al. (Proc. Natl. Acad. Sci. USA, 74, 5463 (1977)).

According to the above-described procedure, nucleotide sequences shown in SEQ ID NOS: 1 and 3 encoding cellobiose dehydrogenase derived from *Coriolus hirsutus* have been determined. A gene having a 3,420 bp nucleotide sequence as shown in SEQ ID No. 1 is a structural gene of a 7,207 bp cellobiose dehydrogenase genomic gene derived from *Coriolus hirsutus,* which was named as cellobiose dehydrogenase 1 gene. A gene having a 3,480 bp nucleotide sequence as shown in SEQ ID No. 3 is a structural gene of a 5,345 bp cellobiose dehydrogenase genomic gene derived from *Coriolus hirsutus,* which was named as cellobiose dehydrogenase 2 gene.

A structural gene portion of the cellobiose dehydrogenase 1 gene shown in SEQ ID No. 1 consists of 16 exons and 15 introns (intervening sequence).

More specifically, exon 1 is located between 129 and 177, intron 1 is located between 178 and 239, exon 2 is located between 240 and 498, intron 2 is located between 499 and 557, exon 3 is located between 558 and 667, intron 3 is located between 668 and 716, exon 4 is located between 717 and 833, intron 4 is located between 834 and 885, exon 5 is located between 886 and 1028, intron 5 is located between 1029 and 1077, exon 6 is located between 1078 and 1242, intron 6 is located between 1243 and 1301, exon 7 is located between 1302 and 1374, intron 7 is located between 1375 and 1425, exon 8 is located between 1426 and 1480, intron 8 is located between 1481 and 1534, exon 9 is located between 1535 and 2165, intron 9 is located between 2166 and 2223, exon 10 is located between 2224 and 2351, intron 10 is located between 2352 and 2407, exon 11 is located between 2408 and 2456, intron 11 is located between 2457 and 2509, exon 12 is located between 2510 and 2598, intron 12 is located between 2599 and 2653, exon 13 is located between 2654 and 2799, intron 13 is located between 2800 and 2859, exon 14 is located between 2860 and 2930, intron 14 is located between 2931 and 2995, exon 15 is located between 2996 and 3100, intron 15 is located between 3101 and 3157, and exon 16 is located between 3158 and 3274. In addition, a region represented by nucleotide No. 3275 and forward is a 3'-nontranslation region including a terminator.

Moreover, it was found that an amino acid sequence estimated from analysis of the nucleotide sequence is an amino acid sequence consisting of 768 amino acid residues, which is shown in SEQ ID No. 2.

On the other hand, a structural gene portion of the cellobiose dehydrogenase 2 gene shown in SEQ ID No. 3 consists of 16 exons and 15 introns.

More specifically, exon 1 is located between 159 and 207, intron 1 is located between 208 and 269, exon 2 is located between 270 and 528, intron 2 is located between 529 and 587, exon 3 is located between 588 and 697, intron 3 is located between 698 and 746, exon 4 is located between 747 and 863, intron 4 is located between 864 and 915, exon 5 is located between 916 and 1058, intron 5 is located between 1059 and 1107, exon 6 is located between 1108 and 1272, intron 6 is located between 1273 and 1331, exon 7 is located between 1332 and 1404, intron 7 is located between 1405 and 1455, exon 8 is located between 1456 and 1510, intron 8 is located between 1511 and 1564, exon 9 is located between 1565 and 2195, intron 9 is located between 2196 and 2253, exon 10 is located between 2254 and 2381, intron 10 is located between 2382 and 2437, exon 11 is located between 2438 and 2486, intron 11 is located between 2487 and 2539, exon 12 is located between 2540 and 2628, intron 12 is located between 2629 and 2683, exon 13 is located between 2684 and 2829, intron 13 is located between 2830 and 2887, exon 14 is located between 2888 and 2958, intron 15 is located between 2959 and 3025, exon 15 is located between 3026 and 3130, intron 15 is located between 3131 and 3208, and exon 16 is located between 3209 and 3325. A region represented by nucleotide No. 3326 and forward is a 3'-nontranslation region including a terminator.

Moreover, it was found that an amino acid sequence estimated from analysis of the nucleotide sequence is an amino acid sequence consisting of 768 amino acid residues, which is shown in SEQ ID No. 4.

Furthermore, in the cellobiose dehydrogenase 1 gene, a cellobiose dehydrogenase gene portion ranging from nucleotide 129 to nucleotide 3274 of a nucleotide sequence as shown in SEQ ID No. 1 is useful. In the cellobiose dehydrogenase 2 gene, a cellobiose dehydrogenase gene portion ranging from nucleotide 159 to nucleotide 3325 of a nucleotide sequence as shown in SEQ ID No. 3 is particularly useful.

A DNA fragment of the cellobiose dehydrogenase gene derived from *Coriolus hirsutus* can be obtained from the DNA fragment containing the above-described cellobiose dehydrogenase chromosomal gene by PCR. As primers used in PCR, sequences consisting of approximately 10 to 50 nucleotides, and preferably consisting of approximately 15 to 30 nucleotides, which are obtained based on the above-described nucleotide sequences shown in SEQ ID NOS: 1 and 3 and sequences complementary thereto, can be used as a sense primer and an antisense primer. For example, a sense primer shown in SEQ ID No. 31 and an antisense primer shown in SEQ ID No. 32 can be used (refer to Example 12).

It is to be noted that transformed *Escherichia coli* strains, *Escherichia coli* JM109/pCHCDH1 and *Escherichia coli* JM109/pCHCDH2, which have genome DNA containing the sequence of the cellobiose dehydrogenase gene derived from *Coriolus hirsutus,* were deposited with the National Institute of Advanced Industrial Science and Technology, an Independent Administrative Institution under the Ministry of Economy, Trade and Industry (the AIST Tsukuba Central 6, Higashi 1-1-1, Tsukuba, Ibaraki, Japan) under accession Nos. FERM BP-8278 and FERM B-8279, respectively, on February 8, 2002. A DNA comprising the nucleotide sequence as shown in SEQ ID No. 1 or 3 contained in these deposited strains is also included in the present invention.

The cellobiohydrolase I gene described in 2. above can be obtained by the following procedure.

Plaque hybridization is carried out in the same manner as in preparation of the gene described 1. above, so as to prepare clones containing cellobiohydrolase I genes. A DNA fragment containing the cellobiohydrolase I gene is isolated from the selected clones, followed by preparation of a restriction map thereof, and determination of a sequence thereof. Such sequencing can be carried out by inserting a DNA fragment containing the cellobiohydrolase I gene into a suitable cloning vector (e.g., a pUC vector such as pUC19), and then applying the method of Sanger et al. (as described above).

According to the above-described procedure, nucleotide sequences shown in SEQ ID NOS: 7, 9, and 11 encoding cellobiohydrolase I derived from *Coriolus hirsutus* have been determined. The thus determined nucleotide sequences were named as cellobiohydrolase I-1 gene, cellobiohydrolase I-2 gene, and cellobiohydrolase I-3 gene, respectively.

Moreover, it was found that amino acid sequences estimated from analysis of the above nucleotide sequences were an amino acid sequence as shown in SEQ ID No. 8 consisting of 456 amino acid residues, an amino acid sequence as shown in SEQ ID No. 10 consisting of 456 amino acid residues, and an amino acid sequence as shown in SEQ ID No. 12 consisting of 457 amino acid residues, respectively.

DNA fragments of the above-described cellobiohydrolase I-1 to I-3 genes can be obtained from DNA fragments containing chromosomal genes of the above-described cellobiohydrolase I-1 to I-3 by PCR. As PCR primers, sequences consisting of approximately 10 to 50 nucleotides, and preferably consisting of approximately 15 to 30 nucleotides, which are obtained based on the above-described nucleotide sequences (SEQ ID NOS: 7, 9, and 11) and sequences complementary thereto, can be used as a sense primer and an antisense primer.

The cellobiohydrolase II gene described in 3. above can be obtained by the following procedure.

Plaque hybridization is carried out in the same manner as in preparation of the gene described 1. above, so as to prepare clones containing cellobiohydrolase II genes. A DNA fragment containing the cellobiohydrolase II gene is isolated from the selected clones, followed by preparation of a restriction map thereof, and determination of a sequence thereof. Such sequencing can be carried out by inserting a DNA fragment containing the cellobiohydrolase II gene into a suitable cloning vector (e.g., a pUC vector such as pUC19), and then applying the method of Sanger et al. (as described above).

According to the above-described procedure, a nucleotide sequence as shown in SEQ ID No. 14 encoding cellobiohydrolase II derived from *Coriolus hirsutus* has been determined. Moreover, it was found that an amino acid sequence estimated from analysis of the above nucleotide sequence was an amino acid sequence as shown in SEQ ID No. 15 consisting of 453 amino acid residues.

A DNA fragment of the above-described cellobiohydrolase II gene can be obtained from a DNA fragment containing a cellobiohydrolase II chromosomal gene by PCR. As PCR primers, sequences consisting of approximately 10 to 50 nucleotides, and preferably consisting of approximately 15 to 30 nucleotides, which are obtained based on the above-described nucleotide sequence and a sequence complementary thereto, can be used as a sense primer and an antisense primer.

The endoglucanase gene belonging to glycolytic enzyme family 61 described in 4. above can be obtained by the following procedure.

mRNA is recovered from cell bodies obtained by growing *Coriolus hirsutus* on woodchips, and a cDNA library is then produced according to common methods. In order to isolate an endoglucanase gene belonging to glycolytic enzyme family 61 from the obtained cDNA library, plaques are formed on an appropriate agar medium, and several plaques are randomly isolated therefrom. Thereafter, a cDNA portion derived from *Coriolus hirsutus* is amplified with two types of suitable primers, and a nucleotide sequence thereof is analyzed, so as to isolate a DNA fragment containing an endoglucanase gene belonging to glycolytic enzyme family 61.

According to the above-described procedure, a nucleotide sequence as shown in SEQ ID No. 18 encoding engoglucanase belonging to glycolytic enzyme family 61 has been determined. Moreover, it was found that an amino acid sequence estimated from analysis of the above nucleotide sequence is an amino acid sequence as shown in SEQ ID No. 19 consisting of 374 amino acid residues.

A DNA fragment of an endoglucanase gene belonging to glycolytic enzyme family 61 can be obtained from a DNA fragment containing the above-described endoglucanase gene belonging to glycolytic enzyme family 61 by PCR. As PCR primers, sequences consisting of approximately 10 to 50 nucleotides, and preferably consisting of approximately 15 to 30 nucleotides, which are obtained based on the nucleotide sequence as shown in SEQ ID No. 18 and a sequence complementary thereto, can be used as a sense primer and an antisense primer.

Moreover, the endoglucanase gene belonging to glycolytic enzyme family 12 described in 5. above can be obtained by the following procedure.

Plaques are randomly isolated from a cDNA library in the same manner as in preparation of the gene described in 4. above. Thereafter, a cDNA portion derived from *Coriolus hirsutus* is amplified, and a nucleotide sequence thereof is analyzed, so as to isolate an endoglucanase gene belonging to glycolytic enzyme family 12.

According to the above-described procedure, a nucleotide sequence as shown in SEQ ID No. 20 encoding engoglucanase belonging to glycolytic enzyme family 12 has been determined. Moreover, it was found that an amino acid sequence estimated from analysis of the above nucleotide sequence is an amino acid sequence as shown in SEQ ID No. 21 consisting of at least 215 amino acid residues.

A DNA fragment of an endoglucanase gene belonging to glycolytic enzyme family 12 derived from *Coriolus hirsutus* can be obtained from a DNA fragment containing the above-described endoglucanase gene belonging to glycolytic enzyme family 12 by PCR. As PCR primers, sequences consisting of approximately 10 to 50 nucleotides, and preferably consisting of approximately 15 to 30 nucleotides, which are obtained based on the nucleotide sequence as shown in SEQ ID No. 20 and a sequence complementary thereto, can be used as a sense primer and an antisense primer.

Furthermore, the endoglucanase gene belonging to glycolytic enzyme family 5 described in 6. above can be obtained by the following procedure.

A Basidiomycete, *Phanerochaete chrysosporium*, is cultured in an appropriate medium, and chromosomal DNA is then recovered by the method of Yelton et al. described in 1. above. With respect to the obtained chromosomal DNA, two suitable PCR primers are produced, which are predicted from the database of the choromosomal DNA of *Phanerochaete chrysosporium*. Thereafter, a DNA fragment of interest is amplified by conventional methods, so as to obtain an endoglucanase gene belonging to glycolytic enzyme family 5.

According to the above-described procedure, a nucleotide sequence as shown in SEQ ID No. 24 encoding engoglucanase belonging to glycolytic enzyme family 5 derived from *Phanerochaete chrysosporium* has been determined. Moreover, it was found that an amino acid sequence estimated from analysis of the above nucleotide sequence is an amino acid sequence as shown in SEQ ID No. 25 consisting of 386 amino acid residues.

A DNA fragment of an endoglucanase gene belonging to glycolytic enzyme family 5 derived from *Phanerochaete chrysosporium* can be obtained from a DNA fragment containing the above-described endoglucanase gene belonging to glycolytic enzyme family 5 by PCR. As PCR primers, sequences consisting of approximately 10 to 50 nucleotides, and preferably consisting of approximately 15 to 30 nucleotides, which are obtained based on the nucleotide sequence as shown in SEQ ID No. 24 and a sequence complementary thereto, can be used as a sense primer and an antisense primer.

Still further, the endoglucanase gene belonging to glycolytic enzyme family 9 described in 7. above can be obtained by the following procedure.

Clones containing endoglucanase genes belonging to glycolytic enzyme family 9 are prepared using suitable PCR primers in the same manner as in preparation of the gene described in 6. above. A DNA fragment containing the endoglucanase gene belonging to glycolytic enzyme family 9 is isolated from the selected clones, followed by preparation of a restriction map thereof, and determination of a sequence thereof. Such sequencing can be carried out by inserting a DNA fragment containing the endoglucanase gene belonging to glycolytic enzyme family 9 into a suitable cloning vector (e.g., a pUC vector such as pUC19), and then applying the method of Sanger et al. (as described above).

According to the above-described procedure, a nucleotide sequence as shown in SEQ ID No. 28 encoding engoglucanase belonging to glycolytic enzyme family 9 derived from *Phanerochaete chrysosporium* has been determined. Moreover, it was found that an amino acid sequence estimated from analysis of the above nucleotide sequence is an amino acid sequence as shown in SEQ ID No. 29 consisting of 592 amino acid residues.

A DNA fragment of an endoglucanase gene belonging to glycolytic enzyme family 9 can be obtained from a DNA fragment containing the above-described endoglucanase gene belonging to glycolytic enzyme family 9 by PCR. As PCR primers, sequences consisting of approximately 10 to 50 nucleotides, and preferably consisting of approximately 15 to 30 nucleotides, which are obtained based on the nucleotide sequence as shown in SEQ ID No. 28 and a sequence complementary thereto, can be used as a sense primer and an antisense primer.

In the first step of the present method, there is prepared DNA encoding antisense RNA substantially complementary to the whole or a part of a transcription product of a cellulolytic enzyme gene derived from the above-described Basidiomycete.

In the present specification, the term "antisense RNA" is used to mean a nucleotide sequence comprising a sequence substantially complementary to the whole or a part of mRNA as a transcription product of the above cellulolytic enzyme gene, wherein, in a case where it exists in a cell, it binds to the mRNA of a cellulolytic enzyme gene that is complementary thereto, and it thereby inhibits the translation of the cellulolytic enzyme gene and suppresses its expression.

Moreover, the term "substantially" is used herein to mean that as long as the antisense RNA binds to mRNA to form a double strand and it inhibits the translation of mRNA into a protein, the sequence may comprise a mutation such as deletion, substitution, or addition.

The length of the above sequence may be appropriately determined, as long as it is capable of suppressing the expression of any one of the cellulolytic enzyme genes of the present invention. It is not necessarily the same as the length of the entire nucleotide sequence of a cellulolytic enzyme gene. For example, when the expression of a cellulolytic enzyme gene is suppressed, the sequence preferably contains a nucleotide sequence, which encodes amino acids at positions 80 and 128 in SEQ ID NOS: 2 and 4 that are heme-binding sites, and amino acids between positions 236 and 241 in SEQ ID NOS: 2 and 4 that correspond to a flavine adenine dinucleotide (FAD)-binding site.

Preparation of antisense RNA and use of the sequence are carried out by conventional methods known to a person skilled in the art. More specifically, the antisense RNA of a cellulolytic enzyme gene can be obtained by performing PCR to obtain an exon portion of the nucleotide sequence of the cellulolytic enzyme gene, or by digesting the cellulolytic enzyme gene with appropriate restriction enzymes. Moreover, the antisense RNA can also be obtained from the cDNA of the cellulolytic enzyme gene. Furthermore, this antisense RNA may be synthetic RNA that is artificially produced on the basis of the information on the nucleotide sequence of the cellulolytic enzyme gene.

In the second step of the present method, there is prepared a vector comprising (a) DNA encoding the antisense RNA as obtained above, or (b) recombinant DNA comprising DNA encoding the antisense RNA as obtained above and a DNA fragment having a promoter activity, wherein the above DNA ligates to the above DNA fragment such that antisense RNA of a cellulolytic enzyme gene is generated as a result of transcription.

In the present specification, the expression "such that antisense RNA of a cellulolytic enzyme gene is generated as a result of transcription" is used to mean that when DNA encoding antisense RNA is transcribed into mRNA under the action of a promoter in a host, antisense RNA capable of binding to mRNA from the cellulolytic enzyme gene of the present invention to form a double strand and thereby suppressing the expression of the cellulolytic enzyme gene can be generated.

In order that DNA binds to a DNA fragment such that antisense RNA is generated, DNA encoding antisense RNA may be ligated to the downstream of a DNA fragment having a promoter sequence in an antisense direction (reverse direction), and it may be then transcribed into mRNA by the action of the promoter. The obtained mRNA is antisense RNA of the nucleotide sequence of a cellulolytic enzyme gene.

A promoter gene is not particularly limited as long as it is a gene fragment having a function as a promoter, but any types of genes can be used as a promoter gene. Examples of a promoter gene may include a GPD promoter and a ras gene promoter. These promoter genes can be obtained by a known genomic cloning method or PCR method, based on sequences registered in gene banks, sequences described in publications, etc. Otherwise, with regard to deposited genes, those obtainable as a result of request for fumishment can also be used.

A gene containing a promoter sequence and a cellulolytic enzyme gene or DNA encoding the antisense RNA of the above gene can be subjected to introduction of a restriction site, a blunt-end treatment, or a sticky-end treatment, if necessary, and then, they can be ligated to each other using suitable DNA ligase. As recombinant DNA techniques including cloning, a ligation reaction, PCR, or the like, those described in, for example, J. Sambrook et al., Molecular Cloning, A Laboratory Manual, Second Edition, Cold Spring Harbor Laboratory Press, 1989, and Short Protocols In Molecular Biology, Third Edition, A Compendium of Methods from Current Protocols in Molecular Biology, John Wiley & Sons, Inc. can be used.

The type of a vector is not particularly limited. It is selected depending on the type of a host transformed with the vector. As a vector, those capable of autonomously replicating in prokaryotic or eukaryotic host cells or capable of homologously recombining in a chromosome can be used. Examples of such a vector may include plasmids, viruses including phages, and cosmids. A vector may appropriately contain a selective marker, a replication origin, a terminator, a polylinker, an enhancer, a ribosome-binding site, etc. Various vectors used for prokaryotes and eukaryotes, such as bacteria, Mycomycetes, yeasts, animals, or plants, are commercially available, or these vectors are described in publications or the like. Using these vectors, DNA or recombinant DNA encoding antisense RNA of the cellobiose dehydrogenase gene of the present invention, can be introduced into a vector.

DNA can be introduced using the technique described in, for example, J. Sambrook et al. (as described above). In order to introduce DNA or recombinant DNA encoding antisense RNA of the cellobiose dehydrogenase gene of the present invention into a vector, as stated above, it should be introduced into a vector such that the antisense RNA of the cellobiose dehydrogenase gene of the present invention is generated as a result of transcription.

In the third step of the present method, transformation is performed with the above vector, so as to prepare host cells having a suppressed cellulolytic enzyme activity.

Any host cells can be used herein, as long as the cells can exhibit a promoter activity in the expression of DNA encoding antisense RNA of the cellobiose dehydrogenase gene of the present invention. Examples of such host cells may include not only fungi such as Basidiomycetes, Eumycetes, or yeasts, but also other eukaryotes (animal cells, plant cells, insect cells, algae, etc.) and prokaryocytes (bacteria, Schizophyceae, etc.). Of these, preferred host cells are Basidiomycetes, and more preferably *Coriolus hirsutus.* More specifically, for example, an auxotrophic mutant strain OJI-1078 (FERM BP-4210) described later in examples, which lacks ornithine carbamoyltransferase of *Coriolus hirsutus,* can be used as a host.

Examples of a transformation method may include the calcium chloride/PEG method, the calcium phosphate method, the lithium acetate method, the electroporation, the protoplast method, the spheroplast method, the lipofection, and the Agrobacterium method, but are not limited thereto.

In the fourth step of the present method, the above-described host cells having a suppressed cellulolytic enzyme activity are inoculated on woodchips, and the woodchips are thereby treated.

In the present specification, the term "woodchips" are used to mean chips obtained by mechanically fragmentating lumbers to a size between 2 and 3 cm. Any types of woodchips can be used herein, as long as they can be obtained from trees including conifers such as Pinus, Cryptomeria japonica, Abies, Picea, Douglas fir or Pinus radiata, and broad-leaved trees such as Fagales, Betulaceae, alders, maples, Eucalyptus, Populus, Acacia, lauans or rubber trees, and they can be used as a material for pulp or the like.

Woodchips are treated with host cells having a suppressed cellulolytic enzyme activity. If the host cells having a suppressed cellulolytic enzyme activity can sufficiently grow therein, woodchips can directly be used without subjecting to a pretreatment. However, if the host cells having a suppressed cellulolytic enzyme activity could more easily grow due to a pretreatment whereby other microorganisms are killed, such a pretreatment is preferably carried out to perform disinfection using autoclave or steaming.

A temperature at which woodchips are treated with host cells having a suppressed cellulolytic enzyme activity is preferably between 10°C and 60°C, and more preferably between 20°C and 30°C. The water content in woodchips is set at 20% to 80%, and preferably at 30% to 50%. Air does not need to be supplied into woodchips, if the host cells having a suppressed cellulolytic enzyme activity can sufficiently grow without air supply. However, in general, 0.001 to 1 L/(1 min.) of air may be supplied to 1 L of woodchips (hereinafter, air supply unit L/(1 min.) is referred to as vvm), and preferably, 0.01 vvm to 0.1 vvm of air may be supplied to the above volume of woodchips.

The amount of the host cells having a suppressed cellulolytic enzyme activity inoculated into woodchips can be appropriately determined, unless it reduces pulp yield or paper strength.

The host cells having a suppressed cellulolytic enzyme activity may be degraded disintegrated with sterilized water, and they may be then inoculated on woodchips and cultured therein. Otherwise, a medium may be added to woodchips, so as to treat them. Any medium can be used herein, as long as the host cells having a suppressed cellulolytic enzyme activity can grow therein. Examples of such a medium may include carbon sources such as glucose, cellobiose, or amorphous cellulose. In addition, nitrogen sources such as yeast extract, peptone, various types of amino acid, soybean, corn steep liquor, or nitrogen compounds such as various types of inorganic nitrogen may also be used. Moreover, various types of salts, vitamin, mineral, and the like may be appropriately used, if necessary.

In the case of woodchips treated with the host cells having a suppressed cellulolytic enzyme activity, reduction in refining energy or increase in paper strength is observed in the production of mechanical pulp such as thermomechanical pulp (TMP), ground pulp (GP), or refiner ground pulp (RGP), and improvement of cooking or increase in paper strength is achieved in the production of chemical pulp such as kraft pulp or sulfite pulp.

In addition, the host cells having a suppressed cellulolytic enzyme activity used in the method of the present invention are also useful in the production of cellulolytic enzymes comprising the cultivation in a suitable medium and the recovery of the generated cellulolytic enzymes.

In this case, when cellulolytic enzyme is expressed or translated in a fusion form with a signal peptide, it is generated in the form of secretion, and it can be directly isolated from a medium. In contrast, when cellulolytic enzyme is generated in the form of nonsecretion, cells may be separated and then disintegrated by a treatment such as ultrasonification or homogenizing to obtain a cell extract, and cellulolytic enzyme may be isolated from the extract. Isolation and purification of the enzymes can be carried out by applying methods such as solvent extraction, salting out, desalination, organic solvent deposition, ultrafiltration, ion exchange, hydrophobic interaction, HPLC, gel filtration and affinity chromatography, electrophoresis, or chromatofocusing, singly or in combination of several methods.

For example, a GPD promoter, a ras promoter are ligated upstream of the cellulolytic enzyme gene of the present invention, followed by applying a recombinant DNA technique, so as to produce cellulolytic enzymes in large quantity. The above promoters can be prepared, for example, from recombinant *Escherichia coli, E. coli* JM109/pCHGP (FERM P-15015) containing a GPD promoter gene derived from *Coriolus hirsutus,* and from recombinant *Escherichia coli,* E. coli DH5α/pCHRAS (FERM P-17352) containing a ras promoter gene derived from *Coriolus hirsutus*.

A protein (cellobiose dehydrogenase) encoded by the cellobiose dehydrogenase gene used in the method of the present invention has the following physicochemical properties.

(1) Action

The activity of cellobiose dehydrogenase was measured by the method described in Method in Enzymology (Wood et al, Vol.160, Academic press, INC. Calfornia). That is to say, dichlorophenolindophenol (manufactured by Sigma Chemical Company) and cellobiose (manufactured by Kanto Kagaku) were dissolved in a 50 mM acetate buffer solution of pH 5 such that the concentrations of both components became 0.33 mM and 0.67 mM, respectively. Thereafter, cellobiose dehydrogenase was added to the obtained buffer solution, followed by a reaction at 37°C. After the reaction was started, absorbance (optical length: 1 cm) at 550 nm, the maximum absorption wavelength of dichlorophenolindophenol, was continuously measured. As a result, the results shown in Figure 1 were obtained.

As shown in Figure 1, decrease of dichlorophenolindophenol was observed as a result of the reduction reaction of cellobiose dehydrogenase. From the facts that when either cellobiose or cellobiose dehydrogenase was eliminated from the reaction system, decrease of dichlorophenolindophenol was not observed, and that although cytochrome C or Mn(III)-malonic acid complex was used instead of dichlorophenolindophenol, the same reduction reaction was observed, it is clear that the present enzyme is cellobiose dehydrogenase.

(2) Method of measuring titer

The activity of cellobiose dehydrogenase was measured as follows. A solution was produced by mixing 250 ul of 0.67 mM dichlorophenolindophenol (manufactured by Sigma Chemical Company), 100 ul of 3.33 mM cellobiose (manufactured by Kanto Kagaku), and 100 ul of a 250 mM acetate buffer solution of pH 5, and thereafter, 50 ul of a test solution was added to the mixed solution, followed by reaction at 37°C. After initiation of the reaction, absorbance (optical length: 1 cm) at 550 nm (molar absorption coefficient: 3965 L/mol/cm) as the maximum absorption wavelength of dichlorophenolindophenol, was continuously measured. With regard to the activity unit of cellobiose dehydrogenase, the amount of enzyme necessary for reducing 1 umol dichlorophenolindophenol per minute under the above conditions was defined as 1 unit (unit: U).

(3) Substrate specificity

Cellobiose dehydrogenase acts not only on cellobiose and cellooligosaccharide, but also on Avicel containing cellulose and hardwood tree Kraft pulp.

(4) Optimal pH and stable pH range

Optimal reaction pH and pH stability were measured using a glycine-HCl buffer solution (pH 2 to 4), an acetate buffer solution (pH 4 to 6), and a phosphate buffer solution (pH 6 to 8). Enzyme activity was measured at the above each pH. The results are shown in Figure 2.

From Figure 2, it was found that the optimal pH for the enzyme reaction was between pH 4 and pH 6. In addition, the enzyme was incubated at 4°C for 24 hours in 50 mM each buffer solution, and then enzyme activity was measured. The results are shown in Figure 3. The present enzyme was stable between pH 2 and pH 5.

(5) Range of temperatures suitable for action

The activity of the enzyme was measured while changing a reaction temperature. The results are shown in Figure 4. The present enzyme showed a high activity in a temperature range between 20°C and 40°C. In addition, the enzyme was incubated in a 50 mM acetate buffer solution (pH 5) at a certain temperature for 30 minutes, and then, enzyme activity was measured. The results are shown in Figure 5.

From Figure 5, it was found that the enzyme maintained approximately 80% or more activity after a treatment at 40°C for 30 minutes and that it maintained approximately 30% or more activity even after a treatment at 50°C for 30 minutes.

(6) Isoelectric point

Isoelectric focusing was carried out using PRECOAT manufactured by SERVA Electrophoresis GmbH at pH 3 to pH 10. As a result, it was found that the isoelectric point was 4.2.

(7) Molecular weight

The molecular weight was measured by SDS polyacrylamide gel electrophoresis. As a result, it was found that the molecular weight was approximately 91,700.

(8) Influence by metal ions or inhibitors

Various substances such as metal salts or inhibitors were added to an enzyme solution such that the concentration became 1 mM, followed by incubation at 4°C overnight. Thereafter, the same types of metal salts were added also into the reaction solution such that the concentration became 1 mM, and enzyme activity was then measured. The results are shown in Table 1. From Table 1, it was found that the enzyme was weakly inhibited by sodium azide and EDTA and was strongly inhibited by $Hg^{2+}$ and SDS.

Table 1

| Metal salts (1 mM) | Relative activity (%) |
|---|---|
| None | 100 |
| $FeSO_4$ | 0 |
| $ZnSO_4$ | 72 |
| $CuSO_4$ | 82 |
| $BaCl_2$ | 71 |
| $MgCl_2$ | 64 |
| $CaCl_2$ | 65 |
| $CoCl_2$ | 53 |
| $MnCl_2$ | 35 |
| $AlCl_2$ | 23 |
| $HgCl_2$ | 0 |
| $NiCl_2$ | 60 |
| $LiCl_2$ | 45 |
| $ZnCl_2$ | 32 |
| $CuCl_2$ | 53 |
| $NaN_3$ | 46 |
| EDTA | 45 |
| SDS | 0 |

In contrast, there have been the following reports regarding the previously known cellobiose dehydrogenase.

Henriksson et al. have reported cellobiose dehydrogenase derived from *Phanerochaete chrysosporium*, having an optimal reaction pH of 5.0, an isoelectric point of about 4.2, and a molecular weight of 89,000 (Eur. J. Biochem., 196 (1991) 101-106). However, the optimal reaction temperature of this cellobiose dehydrogenase is 50°C.

Roy et al. have reported cellobiose dehydrogenase derived from *Trametes versicolor,* having an optimal reaction pH of 5.0, an isoelectric point of about 4.2, and a molecular weight of 97,000 (Appl. Environ. Microbiol., 62 (1996) 4417-4427). However, the optimal reaction temperature of this cellobiose dehydrogenase is 50°C.

Fang et al. have reported cellobiose dehydrogenase derived from *Schizophyllum commune* (Arch. Biochem. Biophys., 353-1 (1998) 37-46). However, the optimal reaction pH of this cellobiose dehydrogenase is 4.5 and the molecular weight thereof is 102,000. They have described neither its optimal temperature nor its isoelectric point.

Schmidhalter, Canevascini, et al., have reported cellobiose dehydrogenase derived from *Conephora puteana*, having an isoelectric point of about 3.9 (Arch. Biochem. Biophys., 300-2 (1993) 559-563). However, the optimal reaction pH of this cellobiose dehydrogenase is 4.0 and the molecular weight thereof is 111,000. They have not described an optimal reaction temperature.

Canevascini et al. have reported cellobiose dehydrogenase derived from *Myceliophtore thermophila*, having an isoelectric point of about 4.1 and a molecular weight of 91,000 (Eur. J. Biochem., 198 (1991) 43-52). However, the optimal reaction pH of this cellobiose dehydrogenase is 7.0, and its reaction optimal temperature is not described.

Shou et al. have reported cellobiose dehydrogenase derived from *Humicola insolens,* having an isoelectric point of about 4.0 and a molecular weight of 92,000 (Biochem. J., 330 (1991) 565-571). The optimal reaction pH of this cellobiose dehydrogenase is 7.0, and the reaction optimal temperature thereof is 65°C.

As stated above, cellobiose dehydrogenase encoded by the cellobiose dehydrogenase gene used in the method of the present invention differs from the known cellobiose dehydrogenases in terms of optimal temperature, optimal pH, molecular weight, and isoelectric point. Accordingly, it is considered that this cellobiose dehydrogenase is a novel cellobiose dehydrogenase. Physiochemical properties of the known cellobiose dehydrogenases are shown in Table 2.

Table 2

| | Molecular weight (kD) | Isoelectric point | Optimal pH | Optimal temperature (°C) | |
|---|---|---|---|---|---|
| *Phanerochaete chrysosporium* | 89 | 4.2 | 5.0 | 50 | Eur. J. Biochem., 196(1991) 101-106 |
| *Trametes versicolor* | 97 | 4.2 | 5.0 | 50 | Appl. Environ. Microbiol., 62 (1996) 4417-4427 |
| *Schizophyllum commune* | 102 | Not described | 4.5 | Not described | Arch. Biochem. Biophys., 353-1 (1998) 37-46 |
| *Conephora puteana* | 111 | 3.9 | 4.0 | Not described | Arch. Biochem. Biophys., 300-2 (1993) 559-563 |
| *Myceliophtore thermophila* | 91 | 4.1 | 7.0 | Not described | Eur. J. Biochem., 198(1991) 43-52 |
| *Humicola insolens* | 92 | 4.0 | 7.0 | 65 | Biochem. J., 330 (1991) 565-571 |

*Coriolus hirsutus* having an ability to produce cellobiose dehydrogenase is not particularly limited. For example, a *Coriolus hirsutus* IFO4917 strain can be used.

Cellobiose dehydrogenase derived from *Coriolus hirsutus* can be obtained, for example, by culturing *Coriolus hirsutus* producing cellobiose dehydrogenase in a medium, and collecting cellobiose dehydrogenase from the obtained culture. A medium with any compositions can be used as the above medium for culturing *Coriolus hirsutus,* as long as the above fungi can proliferate therein. As a nutrient for a medium, those usually used in the culture of *Coriolus hirsutus* can be widely used. Any carbon sources may be used herein as long as it can be assimilated. Glucose, pulp, crystalline cellulose, etc. can be used as such a carbon source. Any available nitrogen compounds may be used herein as a nitrogen source. For example, yeast extract, peptone, various types of amino acid, soybean, corn steep liquor, various types of inorganic nitrogen can be used as such a nitrogen source. In addition, various types of salts, vitamin, mineral, or the like can be appropriately used, if necessary.

Culture temperature and pH can be appropriately determined within a range where *Coriolus hirsutus* can proliferate. For example, the culture temperature is between 20°C and 55°C, and more preferably between 25°C and 30°C. pH is between 3 and 9, and preferably between 4 and 6.

Cellobiose dehydrogenase derived from *Coriolus hirsutus* is produced as a secretion product in a culture solution as a result of the culture of *Coriolus hirsutus* under the above-described conditions. Accordingly, the present enzyme is collected from a culture product obtained as a result of culture. A solution collected from the culture product can be directly used as a cellobiose dehydrogenase crude enzyme solution. However, cellobiose dehydrogenase can also be concentrated or consolidated by salting out, ultrafiltration, or freeze drying. Moreover, cellobiose dehydrogenase can be purified by ammonium sulfate fractionation, molecular weight fractionation by gel filtration, various types of ion exchange resin, hydroxyapatite, hydrophobic chromatogram, isoelectric fractionation, or the like. These methods can be repeated, and further, the methods can be used in combination of other purification methods, if necessary.

EXAMPLES

The present invention will be more specifically described in the following examples. However, these examples are not intended to limit the scope of the present invention.

[Example 1] Preparation of chromosomal DNA library derived from *Coriolus hirsutus*

A *Coriolus hirsutus* IFO 4917 strain was cultured in an agar plate medium, and an agar section with a diameter of 5 mm was cut out of the culture using a cork borer. It was then inoculated into 200 ml of a glucose-peptone medium (which contained 2% glucose, 0.5% polypeptone, 0.2% yeast extract, $KH_2PO_4$, and 0.05% $MgSO_4$, and which was adjusted to pH 4.5 with phosphoric acid), followed by rotary shaking at 28°C for 7 days. After completion of the culture, cell bodies were collected and then washed with 1 L of sterilized water. Thereafter, the cell bodies were frozen with liquid nitrogen.

5 g of the frozen cell bodies were crushed in a mortar. The crushed cell bodies were transferred into a centrifuge tube, and then, 10 ml of a lytic buffer solution (100 mM Tris (pH 8), 100 mM EDTA, 100 mM NaCl, and proteinase K added such that it became 100 μg/ml) was added thereto, followed by incubation at 55°C for 3 hours. After completion of the incubation, a phenol treatment and a chloroform treatment were carried out. Ethanol was gradually added to a water phase. When DNA was deposited, chromosomal DNA was taken up and then suspended in a TE solution.

100 μg of the obtained chromosomal DNA was partially decomposed with restriction enzyme *Sau*3AI, and then fractionated by 5% to 20% sucrose density gradient ultracentrifugation (30,000 rpm, 18 hours), so as to collect a fragmental fraction with a size between 20 and 40 kbp. This fragmental fraction was ligated to phage λ EMBL3-*Bam* arm manufactured by Toyobo Co., Ltd., using T4 DNA ligase. The obtained phage DNA was packaged with Gigapack Gold manufactured by STRATAGENE. Thereafter, *Escherichia coli* P2329 was infected with the obtained product, so as to obtain a chromosomal DNA library.

[Example 2] Isolation of cellobiose dehydrogenase gene from chromosomal DNA library

Clones containing cellobiose dehydrogenase genes were selected from the above chromosomal DNA library by plaque hybridization. A series of operations were carried out according to a conventional method (Sambrook et al., Molecular Cloning A Laboratory Manual/2nd Edition (1989)). A probe used in the plaque hybridization was obtained by labeling the 3'-terminus of a synthetic oligomer having the following sequence with fluorescein, using an oligo DNA labeling kit manufactured by Amersham.

5'-TA(T/C)GA(A/G)AA(T/C)AA(A/G)ATT(T/C/A)TT(T/C/A/G)-3' (SEQ ID No. 5)

As a result, 4 positive clones could be selected from approximately 40,000 plaques. Recombinant phage DNA was prepared from the positive clones by conventional methods, and it was then digested with various types of restriction enzymes, followed by Southern hybridization using the above synthetic DNA. As a result, two different clones which hybridize with probes, were observed as DNA bands with sizes of 5.3 kbp and 7.2 kbp in a fragment obtained by digestion with restriction enzyme *Xho*I.

The above DNA fragments with sizes of 7.2 kbp and 5.3 kbp were cut out by agarose gel electrophoresis, and they were then subcloned into the *Xho*I site of an *Escherichia coli* vector pBluescriptII SK+. Thereafter, an *Escherichia coli* JM109 strain was transformed with the vector. The subcloned DNA was prepared in large quantity, and it was then purified by ultracentrifugation (50,000 rpm, 16 hours, 15°C), followed by sequencing. The nucleotide sequences were determined using a sequencing kit manufactured by United States Biochemical.

The nucleotide sequences are shown in SEQ ID NOS: 1 and 3. It was found that the cellobiose dehydrogenase gene derived from *Coriolus hirsutus* was fragmentated by 15 introns within the range of the above nucleotide sequences. In addition, it was confirmed that amino acid sequences (SEQ ID NOS: 2 and 4) estimated from the nucleotide sequences had high similarity to those of cellobiose dehydrogenase genes that had been reported so far.

[Example 3] Isolation of cellobiohydrolase I-1 gene from chromosomal DNA library

Plaque hybridization was carried out in the same manner as in Example 2. A probe used herein was obtained by labeling with fluorescein the 3'-terminus of a synthetic oligomer having the following sequence prepared based on the nucleotide sequence of the cellobiohydrolase I gene isolated from other organisms, using an oligo DNA labeling kit manufactured by Amersham.

5'-GA(T/C)ATCAAGTT(T/C)ATC(A/G)ATGG-3' (SEQ ID No. 6)

As a result, 2 positive clones could be selected from approximately 40,000 plaques. Recombinant phage DNA was prepared from the positive clones by conventional methods, and it was then digested with various types of restriction enzymes, followed by Southern hybridization using the above synthetic DNA. As a result, a clone as which hybridizes with probe was observed as a single DNA band of 3.9-kbp, in a fragment obtained by digestion with restriction enzymes *Pst*I and *Nhe*I.

The above 3.9-kbp DNA fragment was cut out by agarose gel electrophoresis, and it was then subcloned into the *Pst*I-*Spe*I site of an *Escherichia coli* vector pBluescriptsII SK-. Thereafter, an *Escherichia coli* JM109 strain was transformed with the vector, so as to obtain a plasmid pCHCBHI26 containing a cellobiohydrolase I-1 gene derived from *Coriolus hirsutus.* The nucleotide sequence of the subcloned DNA fragment was determined.

The nucleotide sequence is shown in SEQ ID No. 7. It was found that the cellobiohydrolase I-1 gene derived from *Coriolus hirsutus* was fragmentated by 2 introns within the range of the above nucleotide sequence. In addition, an amino acid sequence estimated from the nucleotide sequence is shown in SEQ ID No. 8.

[Example 4] Isolation of cellobiohydrolase I-2 gene from chromosomal DNA library

Plaque hybridization was carried out in the same manner as in Example 2. A probe used herein was obtained by labeling with fluorescein the 3'-terminus of the synthetic oligomer having the nucleotide sequence as shown in SEQ ID No. 6 used in Example 3, using an oligo DNA labeling kit manufactured by Amersham.

As a result, 3 positive clones could be selected from approximately 40,000 plaques. Recombinant phage DNA was prepared from the positive clones by conventional methods, and it was then digested with various types of restriction enzymes, followed by Southern hybridization using the above synthetic DNA. As a result, a clone which hybridizes with a probe was observed as a single DNA band of 4.2-kbp, in a fragment obtained by digestion with restriction enzyme *Sal*I.

The above 4.2-kbp DNA fragment was cut out by agarose gel electrophoresis, and it was then subcloned into the *Sal*I site of an *Escherichia coli* vector pUC19. Thereafter, an *Escherichia coli* JM109 strain was transformed with the vector, so as to obtain a plasmid pCHCBHI27 containing a cellobiohydrolase I-2 gene derived from *Coriolus hirsutus.* The nucleotide sequence of the subcloned DNA fragment was determined.

The nucleotide sequence is shown in SEQ ID No. 9. It was found that the cellobiohydrolase I-2 gene derived from *Coriolus hirsutus* was fragmentated by 2 introns within the range of the above nucleotide sequence. In addition, an amino acid sequence estimated from the nucleotide sequence is shown in SEQ ID No: 10.

[Example 5] Isolation of cellobiohydrolase I-3 gene from chromosomal DNA library

Plaque hybridization was carried out in the same manner as in Example 2. A probe used herein was obtained by labeling with fluorescein the 3'-terminus of the synthetic oligomer having the nucleotide sequence as shown in SEQ ID No. 6 used in Example 3, using an oligo DNA labeling kit manufactured by Amersham.

As a result, 2 positive clones could be selected from approximately 40,000 plaques. Recombinant phage DNA was prepared from the positive clones by conventional methods, and it was then digested with various types of restriction enzymes, followed by Southern hybridization using the above synthetic DNA. As a result, a clone which hybridizes with a probe was observed as a single DNA band of 4.6-kbp, in a fragment obtained by digestion with restriction enzymes *Eco*RI and *Bam*HI.

The above 4.6-kbp DNA fragment was cut out by agarose gel electrophoresis, and it was then subcloned into the *Eco*RI-*Bam*HI site of an *Escherichia coli* vector pUC19. Thereafter, an *Escherichia coli* JM109 strain was transformed with the vector, so as to obtain a plasmid pCHCBHI31 containing a cellobiohydrolase I-3 gene derived from *Coriolus hirsutus*. The nucleotide sequence of the subcloned DNA fragment was determined.
The nucleotide sequence is shown in SEQ ID No. 11. It was found that the cellobiohydrolase I-3 gene derived from *Coriolus hirsutus* was fragmentated by 2 introns within the range of the above nucleotide sequence. In addition, an amino acid sequence estimated from the nucleotide sequence is shown in SEQ ID No. 12.

[Example 6] Isolation of cellobiohydrolase II gene from chromosomal DNA library

Plaque hybridization was carried out in the same manner as in Example 2. A probe used herein was obtained by labeling with fluorescein the 3'-terminus of a synthetic oligomer having the following sequence prepared based on the nucleotide sequence of a cellobiohydrolase II gene isolated from other organisms, using an oligo DNA labeling kit manufactured by Amersham.

5'-CAGTGGGGIGACTGGTGCAAC-3' (SEQ ID No. 13)

As a result, 8 positive clones could be selected from approximately 100,000 plaques. Recombinant phage DNA was prepared from the positive clones by conventional methods, and it was then digested with various types of restriction enzymes, followed by Southern hybridization using the above synthetic DNA. As a result, a clone which hybridized with a probe was observed as a single DNA band of 5.0-kbp, in a fragment obtained by digestion with restriction enzymes *Eco*RV and *Nco*I.
In order to recover the above DNA fragment, a 5.0-kbp DNA fragment obtained by digesting with restriction enzyme *Nco*I, smoothing with a Klenow fragment, and further digesting with *Eco*RV, was cut out by agarose gel electrophoresis. Thereafter, it was then subcloned into the *Sma*I site of an *Escherichia coli* vector pUC19, so as to obtain a plasmid pCHCBHII containing a cellobiohydrolase II gene derived from *Coriolus hirsutus*. Thereafter, an *Escherichia coli* JM109 strain was transformed with this plasmid. The nucleotide sequence of the subcloned DNA fragment was determined.
The nucleotide sequence is shown in SEQ ID No. 14. It was found that the cellobiohydrolase II gene derived from *Coriolus hirsutus* was fragmentated by 6 introns within the range of the above nucleotide sequence. In addition, an amino acid sequence estimated from the nucleotide sequence is shown in SEQ ID No. 15.

[Example 7] Preparation of *Coriolus hirsutus* cDNA library

6 g dry weight of *Eucalyptus globulus* chips were placed in a glass schale with a diameter of 9.5 cm, and were then sterilized at 121°C for 15 minutes. A medium was prepared by adding 20 ml of a peptone medium (which contained 1.0% polypeptone, 0.2% yeast extract, $KH_2PO_4$, and 0.05% $MgSO_4$, and which was adjusted to pH 4.5 with phosphoric acid) to the thus treated chips. Thereafter, using a cork borer, agar sections each having a diameter of 5 mm were cut out of the agar plate culture product of a *Coriolus hirsutus* IFO 4917 strain, and the thus obtained 3 agar sections were inoculated into the above obtained medium, followed by static culture at 30°C for 10 days. After completion of the culture, cell bodies were collected and then frozen with liquid nitrogen.
Thereafter, total RNA was collected from the frozen cell bodies by the guanidine-hydrochloric acid method. Subsequently, poly(A)$^+$RNA was prepared using an Oligotex-dT <super> mRNA Purification kit manufactured by Takara Shuzo Co., Ltd. Thereafter, using a cDNA Synthesis kit manufactured by STRATAGENE, cDNA was synthesized, and an *Eco*RI site was attached to the 5'-side thereof and an *Xho*I site was attached to the 3'-side thereof. It was then inserted into the *Eco*RI-*Xho*I site of a λZAPII vector, and using an *in vitro* packaging kit, a cDNA library was produced.

[Example 8] Isolation of endoglucanase gene belonging to glycolytic enzyme family 61 from *Coriolus hirsutus* cDNA library

A cDNA library solution produced in Example 7 was appropriately diluted such that plaques could be isolated on a schale, and then, an *Escherichia coli* XL1 Blue MRF' strain was infected with the solution, followed by culture at 37°C overnight, so that plaques were formed. A single plaque obtained as above was suspended in an SM buffer. Using universal sequencing primers, an M13(-20) primer (GTAAAACGACGGCCAGT, SEQ ID No. 16) and an M13 reverse primer (GGAAACAGCTATGACCATG, SEQ ID No. 17), a PCR reaction was carried out to amplify a cDNA fragment. The nucleotide sequences of the thus obtained cDNA fragments were randomly analyzed. As a result, a cDNA gene encoding endoglucanase belonging to glycolytic enzyme family 61 was discovered. The nucleotide sequence is shown in SEQ ID No. 18, and an amino acid sequence estimated therefrom is shown in SEQ ID No. 19.

[Example 9] Isolation of endoglucanase gene belonging to glycolytic enzyme family 12 from *Coriolus hirsutus* cDNA library

A cDNA library solution produced in Example 7 was appropriately diluted such that plaques could be isolated on a schale, and then, an *Escherichia coli* XL1 Blue MRF' strain was infected with the solution, followed by culture at 37°C overnight, so that plaques were formed. A single plaque obtained as above was suspended in an SM buffer. Using universal sequencing primers, an M13(-20) primer (GTAAAACGACGGCCAGT, SEQ ID No. 16) and an M13 reverse primer (GGAAACAGCTATGACCATG, SEQ ID No. 17), a PCR reaction was carried out to amplify a cDNA fragment. The nucleotide sequences of the thus obtained cDNA fragments were randomly analyzed. As a result, a cDNA gene encoding endoglucanase belonging to glycolytic enzyme family 12 was discovered. The nucleotide sequence is shown in SEQ ID No. 20, and an amino acid sequence estimated therefrom is shown in SEQ ID No. 21.

[Example 10] Isolation of endoglucanase gene belonging to glycolytic enzyme family 5 from basidiomycete *Phanerochaete chrysosporium* chromosomal DNA

Using a cork borer, agar sections each having a diameter of 5 mm were cut out of the agar plate culture product of a *Phanerochaete chrysosporium* ATCC 34541 strain, and the 5 sections were inoculated into 100 ml of a glucose-peptone medium (which contained 2% glucose, 0.5% polypeptone, 0.2% yeast extract, $KH_2PO_4$, and 0.05% $MgSO_4$, and which was adjusted to pH 4.5 with phosphoric acid), followed by shaking culture at 30°C for 5 days. After completion of the culture, cell bodies were collected and then frozen with liquid nitrogen. 5 g of the frozen cell bodies were crushed in a mortar. The crushed cell bodies were transferred into a centrifuge tube, and then, 10 ml of a lytic buffer solution (100 mM Tris (pH 8), 100 mM EDTA, 100 mM NaCl, and proteinase K added such that it became 100 μg/ml) was added thereto, followed by incubation at 55°C for 3 hours. After completion of the incubation, a phenol treatment and a chloroform treatment were carried out. Ethanol was gradually added to a water phase. When DNA was deposited, chromosomal DNA was taken up and then suspended in a TE solution, so as to produce a *Phanerochaete chrysosporium* chromosomal DNA solution.

Using two DNA primers described below, a PCR reaction was performed on the above *Phanerochaete chrysosporium* chromosomal DNA solution, so as to obtain an endoglucanase chromosomal gene belonging to glycolytic enzyme family 5.

5'-ATGAAGTTACTTCTTGCTCTC-3' (SEQ ID No. 22)

5'-TCACAGGAAGGGTTCGAGTGC-3' (SEQ ID No. 23)

The obtained nucleotide sequence is shown in SEQ ID No. 24. It was found that the endoglucanase gene belonging to glycolytic enzyme family 5 derived from *Phanerochaete chrysosporium* was fragmentated by 15 introns within the range of the above nucleotide sequence. In addition, an amino acid sequence estimated from the nucleotide sequence is shown in SEQ ID No. 25.

[Example 11] Isolation of endoglucanase gene belonging to glycolytic enzyme family 9 from basidiomycete *Phanerochaete chrysosporium* chromosomal DNA

Using two primers described below, a PCR reaction was performed on the *Phanerochaete chrysosporium* chromosomal DNA solution prepared in Example 10, so as to obtain an endoglucanase gene belonging to glycolytic enzyme family 9.

5'-ATGATACCTCTCCGCTCTGC-3' (SEQ ID No. 26)

5'-TATCTTCCTGATGCGATTCC-3' (SEQ ID No. 27)

The obtained nucleotide sequence is shown in SEQ ID No. 28. It was found that the endoglucanase gene belonging to glycolytic enzyme family 9 derived from *Phanerochaete chrysosporium* was fragmentated by 7 introns within the range of the above nucleotide sequence. In addition, an amino acid sequence estimated from the nucleotide sequence is shown in SEQ ID No. 29.

[Example 12] Construction of expression vector for *Coriolus hirsutus*-derived cellobiose dehydrogenase gene, using *Coriolus hirsutus*-derived glyceraldehyde-3-phosphate dehydrogenase promoter

A structural gene region of a cellobiose dehydrogenase gene was ligated downstream of a promoter of *Coriolus hirsutus,* whereby the original cellobiose dehydrogenase gene was substituted by a glyceraldehyde-3-phosphate dehydrogenase gene promoter region, so as to obtain a cellobiose dehydrogenase gene expression vector.

More specifically, a glyceraldehyde-3-phosphate dehydrogenase chromosomal gene was digested with *Eco*RI and *Bam*HI, so as to obtain a 3.8-kbp DNA fragment (fragment 1). The fragment 1 was ligated to the *Eco*RI-*Bam*HI site of a phage vector M13mp18, using a T4 DNA ligase. An *Escherichia coli* JM109 strain was transformed therewith, so as to prepare single-stranded phage DNA.

Subsequently, a DNA primer shown in SEQ ID No. 30 was synthesized, and it was then annealed to the above single-stranded phage DNA. Then, only a promoter region of a GPD gene was synthesized by the primer extension method, and it was then digested with restriction enzyme *Eco*RI (manufactured by Takara Shuzo Co., Ltd.), so as to prepare a 0.9-kbp DNA fragment (fragment 2).

5'-CATGGTGTGTGGTGGATG-3' (SEQ ID No. 30)

On the other hand, in order to extract only a gene region encoding mature enzyme of cellobiose dehydrogenase, using a plasmid pCHCDH1 as a template, a PCR reaction was carried out with primers shown in SEQ ID NOS: 31 and 32 for elongation, so as to obtain a DNA fragment with a size of approximately 3.5 kbp (fragment 3)

5'-AAGTTCAAGAGTCTCCTGT-3' (SEQ ID No. 31)

5'-GGTACAGTACTTATCTGTAT-3' (SEQ ID No. 32)

An *Escherichia coli* vector pUC18 was digested with restriction enzymes *Eco*RI and *Sma*I (Takara Shuzo Co., Ltd.), and the above two types of DNA fragments were mixed and ligated to each other using T4 DNA ligase. Thereafter, an *Escherichia coli* JM109 strain was transformed therewith. A plasmid, into which the above two types of DNA fragments, fragments 2 and 3, had been simultaneously inserted, was isolated from the ampicillin-resistant transformed strain. The plasmid was named as pGPCDH1.

[Example 13] Construction of plasmid having antisense sequence of cellobiose dehydrogenase gene

A 0.9-kb glyceraldehyde-3-phosphate dehydrogenase gene promoter region fragment 2 was obtained by the same method as in Example 12. The obtained fragment 2 was ligated to the *Eco*RI-*Sma*I site of pUC18, using T4 DNA ligase, and an *Escherichia coli* JM109 strain was transformed therewith. A plasmid into which the above fragment 2 had been inserted was isolated from the ampicillin-resistant transformed strain. The plasmid was named as pCHGP1. The plasmid pCHGP1 was digested with restriction enzyme *Nco*I-*Xba*I, so as to prepare a vector portion (fragment 4).

Moreover, using two primers described below (SEQ ID NOS: 33 and 34), a PCR reaction was performed on the plasmid pCHCDH1 containing the cellobiose dehydrogenase 1 gene derived from *Coriolus hirsutus,* so as to amplify an approximately 650-bp DNA fragment containing the $8^{th}$ exon of the cellobiose dehydrogenase gene derived from *Coriolus hirsutus.* Thereafter, the amplified product was digested with restriction enzymes *Xba*I and *Nco*I to obtain a DNA fragment (fragment 5).

5'-TCTAGATTTACTGGTACCCCAACAACAATG-3' (SEQ ID No. 33)

5'-CCATGGGTTGATCGACGGGTTGTCAGACACG-3' (SEQ ID No. 34)

The above fragment 4 was mixed with the above fragment 5, and they were ligated to each other with T4 DNA ligase. Thereafter, an *Escherichia coli* JM109 strain was transformed therewith. A plasmid into which the above fragment 5 had been inserted was isolated from the ampicillin-resistant transformed strain. The plasmid was named as pGPantiCDH1. The plasmid pGPantiCDH1 was digested with restriction enzymes *Xba*I and *Hind*III, so as to prepare a vector portion (fragment 6).

Subsequently, using two primers described below (SEQ ID NOS: 35 and 36), a PCR reaction was performed on a plasmid pBSMPOG1 (FERM P-14933) containing a manganese peroxidase gene derived from *Coriolus hirsutus,* so as to amplify the C-terminal nontranslation region of manganese peroxidase. The amplified product was digested with restriction enzymes *Xba*I and *Hind*III to obtain an approximately 1-kb DNA fragment (fragment 7).

5'-TCTAGAGTCACCTCCGT-3' (SEQ ID No. 35)

5'-AAGCTTGGGTACTGTG-3' (SEQ ID No. 36)

The above fragment 6 was mixed with the above fragment 7, and they were ligated to each other with T4 DNA ligase. Thereafter, an *Escherichia coli* JM109 strain was transformed therewith. A plasmid in which the above DNA fragment 7 had been inserted in a forward direction was isolated from the ampicillin-resistant transformed strain. The plasmid was named as pGPCDHAM.

[Example 14] Construction of plasmid having antisense sequence of cellobiohydrolase I gene derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the plasmid pCHCBHI26 obtained in Example 3, which contained the cellobiohydrolase I gene derived from *Coriolus hirsutus*, so as to amplify an approximately 750-bp DNA fragment. Thereafter, the amplified product was digested with restriction enzymes *Nco*I and *Xba*I to obtain a DNA fragment (fragment 9).

5'-TCTAGAGCCAACCTCGAGGGGTGG-3' (SEQ ID No. 37)

5'-CCATGGGAACGTCGAGCCGATGGG-3' (SEQ ID No. 38)

The above fragment 8 was mixed with the above fragment 9, and they were ligated to each other with T4 DNA ligase. Thereafter, an *Escherichia coli* JM109 strain was transformed therewith. A plasmid into which the above DNA fragment 9 had been inserted was isolated from the ampicillin-resistant transformed strain. The plasmid was named as pGPCBHI26AM.

[Example 15] Construction of plasmid having antisense sequence of cellobiohydrolase I gene derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the plasmid pCHCBHI27 obtained in Example 4, which contained the cellobiohydrolase I gene derived from *Coriolus hirsutus*, so as to amplify an approximately 750-bp DNA fragment. Thereafter, the amplified product was digested with restriction enzymes *Nco*I and *Xba*I to obtain a DNA fragment (fragment 10).

5'-TCTAGAGCCAACGTCCTCGGCTGG-3' (SEQ ID No. 39)

5'-CCATGGGTAGGTCGAGCCGATGGG-3' (SEQ ID No. 40)

The above fragment 8 was mixed with the above fragment 10, and they were ligated to each other with T4 DNA ligase. Thereafter, an *Escherichia coli* JM109 strain was transformed therewith. A plasmid into which the above DNA fragment 10 had been inserted was isolated from the ampicillin-resistant transformed strain. The plasmid was named as pGPCBHI27AM.

[Example 16] Construction of plasmid having antisense sequence of cellobiohydrolase I gene derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the plasmid pCHCBHI31 obtained in Example 5, which contained the cellobiohydrolase I gene derived from *Coriolus hirsutus,* so as to amplify an approximately 750-bp DNA fragment. Thereafter, the amplified product was digested with restriction enzymes *Nco*I and *Xba*I to obtain a DNA fragment (fragment 11).

5'-TCTAGAGCCAACGTCCTCGGCTGG-3' (SEQ ID No. 41)

5'-CCATGGAGCGTAGGTCGAGCCAATG-3' (SEQ ID No. 42)

The above fragment 8 was mixed with the above fragment 11, and they were ligated to each other with T4 DNA ligase. Thereafter, an *Escherichia coli* JM109 strain was transformed therewith. A plasmid into which the above DNA fragment 11 had been inserted was isolated from the ampicillin-resistant transformed strain. The plasmid was named as pGPCBHI31AM.

[Example 17] Construction of plasmid having antisense sequence of cellobiohydrolase II gene derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the plasmid pCHCBHII obtained in Example 6, which contained the cellobiohydrolase I gene derived from *Coriolus hirsutus,* so as to amplify an approximately 600-bp DNA fragment. Thereafter, the amplified product was digested with restriction enzymes *Nco*I and *Xba*I to obtain a DNA fragment (fragment 12).

5'-TCTAGAATCTACCTGAGCCCTTAC-3' (SEQ ID No. 43)

5'-CCATGGCTCACTAGTGGCGAGACC-3' (SEQ ID No. 44)

The above fragment 8 was mixed with the above fragment 12, and they were ligated to each other with T4 DNA ligase. Thereafter, an *Escherichia coli* JM109 strain was transformed therewith. A plasmid into which the above DNA fragment 12 had been inserted was isolated from the ampicillin-resistant transformed strain. The plasmid was named as pGPCBHIIAM.

[Example 18] Construction of plasmid having antisense sequence of endoglucanase gene belonging to family 61 derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the endoglucanase cDNA gene belonging to glycolytic enzyme family 61 derived from *Coriolus hirsutus* obtained in Example 12, so as to amplify an approximately 600-bp DNA fragment. Thereafter, the amplified product was digested with restriction enzymes *Nco*I and *Xba*I to obtain a DNA fragment (fragment 13).

5'-TCTAGAGCTCACGGTTTCATTCATG-3' (SEQ ID No. 45)

5'-CCATGGGGTGTAGAGCCCCGGAATG-3' (SEQ ID No. 46)

The above fragment 8 was mixed with the above fragment 13, and they were ligated to each other with T4 DNA ligase. Thereafter, an *Escherichia coli* JM109 strain was transformed therewith. A plasmid into which the above DNA fragment 13 had been inserted was isolated from the ampicillin-resistant transformed strain. The plasmid was named as pGPEG61AM.

[Example 19] Construction of plasmid having antisense sequence of endoglucanase gene belonging to family 12 derived from *Coriolus hirsutus*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the endoglucanase cDNA gene belonging to glycolytic enzyme family 12 derived from *Coriolus hirsutus* obtained in Example 9, so as to amplify an approximately 700-bp DNA fragment. Thereafter, the amplified product was digested with restriction enzymes *Nco*I and *Xba*I to obtain a DNA fragment (fragment 13).

5'-TCTAGAGCGGGCCCGTACTCGCTC-3' (SEQ ID No. 47)

5'-CCATGGGTAATGTGATTCCTGTCG-3' (SEQ ID No. 48)

The above fragment 8 was mixed with the above fragment 13, and they were ligated to each other with T4 DNA ligase. Thereafter, an *Escherichia coli* JM109 strain was transformed therewith. A plasmid into which the above DNA fragment 13 had been inserted was isolated from the ampicillin-resistant transformed strain. The plasmid was named as pGPEG12AM.

[Example 20] Construction of plasmid having antisense sequence of endoglucanase gene belonging to family 5 derived from *Phanerochaete chrysosporium*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the endoglucanase gene belonging to glycolytic enzyme family 5 derived from *Phanerochaete chrysosporium* obtained in Example 10, so as to amplify an approximately 600-bp DNA fragment. Thereafter, the amplified product was digested with restriction enzymes *Nco*I and *Xba*I to obtain a DNA fragment (fragment 15).

5'-TCTAGAATGAAGTACTTCTTGCTC-3' (SEQ ID No. 49)

5'-CCATGGCGTTTGGCGTACCGTCTG-3' (SEQ ID No. 50)

The above fragment 8 was mixed with the above fragment 15, and they were ligated to each other with T4 DNA ligase. Thereafter, an *Escherichia coli* JM109 strain was transformed therewith. A plasmid into which the above DNA fragment 14 had been inserted was isolated from the ampicillin-resistant transformed strain. The plasmid was named as pGPPCEG5AM.

[Example 21] Construction of plasmid having antisense sequence of endoglucanase gene belonging to family 9 derived from *Phanerochaete chrysosporium*

The plasmid pGPCDHAM obtained in Example 13 was digested with restriction enzymes *Nco*I and *Xba*I, so as to prepare a DNA fragment for vector portion (fragment 8). Subsequently, using two primers described below, a PCR reaction was performed on the endoglucanase gene belonging to glycolytic enzyme family 9 derived from *Phanerochaete chrysosporium* obtained in Example 11, so as to amplify an approximately 500-bp DNA fragment. Thereafter, the amplified product was digested with restriction enzymes *Nco*I and *Xba*I to obtain a DNA fragment (fragment 16).

5'-TCTAGACCCCGGTACAGACGCCGC-3' (SEQ ID No. 51)

5'-CCATGGGATGTTAGGAATGATCTG-3' (SEQ ID No. 52)

The above fragment 8 was mixed with the above fragment 16, and they were ligated to each other with T4 DNA ligase. Thereafter, an *Escherichia coli* JM109 strain was transformed therewith. A plasmid into which the above DNA fragment 15 had been inserted was isolated from the ampicillin-resistant transformed strain. The plasmid was named as pGPPCEG9AM.

[Example 22] Method for transforming *Coriolus hirsutus*

a. Cultivation of monokaryon strain

Approximately 30 glass beads each having a diameter of about 6 mm were placed in a 500 ml-volume Erlenmeyer flask. 100 ml of an SMY medium (1% sucrose, 1% malt extract, and 0.4% yeast extract) was dispensed into the above flask and then sterilized. Thereafter, an agar section having a diameter of 5 mm was cut out of an agar plate medium containing a *Coriolus hirsutus* OJI-1078 strain, using a cork borer. The section was then inoculated into the above SMY medium, followed by static culture at 28°C for 7 days (preculture). However, in order to fragmentate hypha, the flask was shaken once or twice a day for mixing. Subsequently, 200 ml of an SMY medium was dispensed into a 1 L-volume Erlenmeyer flask, and a rotator was further added thereto, followed by sterilization. Thereafter, the above precultured hypha was collected by filtration with a nylon mesh (with a pore size of 30 µm), and the total amount of the hypha was inoculated into the medium, followed by culture at 28°C. While culturing, the medium was stirred with a stirrer for 2 hours per day, so that the hypha was fragmentated. This culture was carried out for 4 days.

b. Preparation of protoplasts

The above liquid culture hypha was collected by filtration with a nylon mesh (with a pore size of 30 µm), and the collected hypha was then washed with an osmoregulation solution (0.5 M $MgSO_4$, 50 ml maleate buffer (pH 5.6)). Subsequently, 100 mg of wet cell bodies was suspended in 1 ml of a cell-wall-digesting enzyme solution. While the mixture was gently shaken, it was incubated at 28°C for 3 hours, and protoplasts were released. As a cell-wall-digesting enzyme solution, the following commercially available enzyme preparations were used in combination. This is to say, 5 mg of Cellulase ONOZUKA (cellulase ONOZUKA RS manufactured by Yakult) and 10 mg of Yatalase (manufactured by Takara Shuzo Co., Ltd.) were dissolved in 1 mg of the above osmoregulation solution, and the obtained solution was used as an enzyme solution.

c. Purification of protoplasts

Hypha fragments were removed from the above enzyme reaction solution using a nylon mesh (with a pore size of 30 µm). Thereafter, in order to enhance the recovery rate of protoplasts, hypha fragments and protoplasts remaining on the nylon mesh were washed once with the above osmoregulation solution. The obtained protoplast suspension was centrifuged (1,000 k × g, 5 minutes) to remove the supernatant. The residue was resuspended in 4 ml of 1 M sucrose (20 mM MOPS buffer solution, pH 6.3). The obtained suspension was centrifuged again, and the resultant product was washed twice with the above 1M sucrose solution. The precipitate was suspended in 500 µl of a 1M sorbitol solution (20 mM MES, pH 6.4) including 40 mM calcium chloride, and the obtained suspension was used as a protoplast solution. This solution was conserved at 4°C.

The protoplast concentration was determined by direct observation with a speculum, using a hemacytometer. All the centrifugal operations were carried out at 1,000 × g for 5 minutes at room temperature, using a swing rotor.

d. Transformation

The plasmid pGPCDH1 (2 µg) obtained in Example 12 was added to 100 µl of a protoplast solution with a

concentration of $10^6$ cells/100 μl. Moreover, as a selective marker, 0.2 μg of a plasmid pUCR1 containing an ornithine carbamoyltransferase gene derived from *Coriolus hirsutus* (JP Patent Publication (Kokai) No. 6-054691 A (1994); FERM BP-4201) was added to the above mixed solution, followed by cooling on ice for 30 minutes. Subsequently, a PEG solution (50% PEG3400, 20mM MOPS (pH 6.4)) was added thereto in an amount equal to the liquid amount, followed by cooling on ice for 30 minutes. Thereafter, the obtained solution was mixed into a minimal agar medium (1% agar) containing 0.5 M sucrose and leucine, and the mixture was dispersed on a plate. The above plate was cultured at 28°C for several days, so as to obtain a transformant. Thereafter, DNA was prepared from the transformant, and then, it was confirmed by Southern hybridization that a cellobiose dehydrogenase gene expression plasmid pGPCDH1 of interest was incorporated therein.

[Example 23] Preparation of *Coriolus hirsutus* transformant highly secreting and producing cellobiose dehydrogenase

The transformant obtained in Example 22 was inoculated into a 300 ml-volume Erlenmeyer flask containing 100 ml of a glucose-peptone medium (which contained 30 g/l glucose, 10 g/l polypeptone, 1.5 g/l $KH_2PO_4$, 0.5 g/l $MgSO_4$, and 2 mg/l thiamine hydrochloride, and which was adjusted to pH 4.5 with phosphoric acid), followed by shaking culture at 28°C at 100 rpm. Using the above-described cellobiose dehydrogenase activity measurement method, the cellobiose dehydrogenase activity was measured with time. The activity of cellobiose dehydrogenase was 0.02 U/ml in a control, but it was 0.2 U/ml in the transformant.

[Example 24] Selection of transformant having suppressed cellobiose dehydrogenase activity

Transformation was carried out as described in the transformation method in the above Example 22 using the plasmid pGPCDHAM produced in Example 13 instead of the plasmid pGPCDH1. The obtained transformant was inoculated into a 300 ml-volume Erlenmeyer flask containing 100 ml of hardwood oxygen-bleached kraft pulp (LOKP)-peptone medium (which contained 1% LOKP, 0.5% polypeptone, 0.2% yeast extract, 0.15% $KH_2PO_4$, and 0.05% $MgSO_4$, and which was adjusted to pH 4.5 with phosphoric acid), followed by shaking culture at 28°C at 100 rpm. Using the above-described cellobiose dehydrogenase activity measurement method, the cellobiose dehydrogenase activity was measured with time. As a result, it was found that a transformant having at maximum 70% suppressed cellobiose dehydrogenase activity could be obtained.

[Example 25] Treatment of woodchips with transformant having suppressed cellobiose dehydrogenase

The transformant with a suppressed cellobiose dehydrogenase activity selected in Example 24 was cultured at 28°C in a potato dextrose agar medium, and the culture product was conserved at 4°C. 5 sections each having a diameter of 5 mm were cut out form the plate using a cork borer. The 5 sections were then inoculated into a 300 ml-volume Erlenmeyer flask containing 100 ml of a glucose-peptone medium (which contained 2% glucose, 0.5% polypeptone, 0.2% yeast extract, $KH_2PO_4$, and 0.05% $MgSO_4$, and which was adjusted to pH 4.5 with phosphoric acid), followed by shaking culture at 28°C at 100 rpm for 1 week. After completion of the culture, cell bodies were filtrated, and a medium remaining in the cell bodies was washed with sterilized water. The cell bodies were mixed with sterilized water, and they were then crushed with a Waring blender for 15 seconds. Thereafter, the cell bodies were inoculated into 1 kg bone-dry weight of Eucalyptus lumbers, such that the dry weight of the cell bodies became 10 mg. After the inoculation, the mixture was well stirred, such that the cell bodies were distributed uniformly. As a culture, a static culture was carried out at 28°C for 1 week under aeration. Saturated water vapors were aerated whenever necessary, such that the water contents in the chips became 40% to 65%. For aeration, the amount of air discharged was set at 0.01 vvm per chip.

[Example 26] Production of mechanical pulp using transformant having suppressed cellobiose dehydrogenase activity

Woodchips were prepared from radiata pine lumbers, and the woodchips were treated in the same manner as in Example 25. The treated woodchips were beaten using a laboratory refiner (manufactured by Kumagai Riki KOGYO), and Canadian Standard Freeness was set at 200 ml. Thereafter, handsheets used for physical tests of pulp were prepared in accordance with Tappi test method T205om-81, and the physical tests of handmade pulp sheets were carried out in accordance with Tappi test T220om-83. Electric energy used herein was measured using a wattmeter (Hiokidenki model 3133) and an integration counter (model 3141). The yield of chips was obtained by placing 1 kg bone-dry weight of woodchips containing water into a container, measuring the bone-dry weight of the woodchips before and after the treatment, and then calculating the chip yield by the following formula:

(bone-dry weight after treatment)/(bone-dry weight before treatment) × 100

As shown in Table 3 below, use of a transformant having suppressed cellobiose dehydrogenase could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 3

| Effects of woodchip treatment with microorganisms having suppressed cellobiose dehydrogenase activity on mechanical pulp | | | |
|---|---|---|---|
| | Control | Transformant | Wild-type strain |
| Chip yield (%) | 99.8 | 98.9 | 94.7 |
| Refining energy (Kw·h/ton) | 2560 | 1792 | 1840 |
| Tear index ($mN\cdot m^2/g$) | 7.92 | 8.21 | 6.95 |
| Burst index ($kPa\cdot m^2/g$) | 1.35 | 1.52 | 1.21 |

[Example 27] Cooking of woodchips treated with transformant having suppressed cellobiose dehydrogenase activity

Woodchips made from Eucalyptus lumbers were treated in the same manner as in Example 25. Thereafter, 400 g bone-dry weight was weighed from the woodchips. A cooking white liquor was added to the woodchips in an autoclave, such that a liquid ratio of 5, a sulfur degree of 30%, and an effective alkali of 17% (as $Na_2O$) could be achieved. Thereafter, a cooking temperature was set at 150°C, and Kraft cooking was carried out. After completion of the Kraft cooking, a black liquor was separated, and the obtained chips were refined using a high concentration refining machine. Thereafter, the refined chips were subjected to centrifugal dehydration with a filter cloth followed by washing with water, three times. Thereafter, uncooked products were eliminated by screening, and the residue was subjected to centrifugal dehydration, so as to obtain cooked unbleached pulp.

2.0% by mass of NaOH was added to the pulp obtained by the above Kraft cooking, and oxygen gas was then injected therein, followed by a treatment at 100°C under an oxygen gauge pressure of 0.49 MPa (5 $kg/cm^2$) for 60 minutes.

The above obtained pulp was subjected to a 4-steps bleaching treatment consisting of D-E-P-D sequence, as described below. In the first chlorine dioxide treatment (D), pulp was prepared such that the concentration of the pulp became 10% by mass, and 0.4% by mass of chlorine dioxide was added thereto, followed by a treatment at 70°C for 40 minutes. Subsequently, the pulp was washed with ion exchanged water and then dehydrated. The concentration of the pulp was adjusted to 10% by mass, and 1% by mass of sodium hydroxide was added to the pulp, followed by an alkali extraction treatment (E) at 70°C for 90 minutes. Subsequently, the pulp was washed with ion exchanged water and then dehydrated. The concentration of the pulp was adjusted to 10% by mass, and then, 0.5% by mass of hydrogen peroxide and 0.5% by mass of sodium hydroxide were successively added to the pulp, followed by a hydrogen peroxide treatment (P) at 70°C for 120 minutes. Subsequently, the pulp was washed with ion exchanged water and then dehydrated. The concentration of the pulp was adjusted to 10%, and 0.25% by mass of chlorine dioxide was added to the pulp, followed by a chlorine dioxide treatment (D) at 70°C for 180 minutes. Finally, the pulp was washed with ion exchanged water and then dehydrated, so as to obtain bleached pulp with a whiteness degree of 86.0% in accordance with JIS P 8123.

The thus obtained pulp slurry having a pulp concentration of 4% by mass was beaten with a refiner, such that the freeness became 410 ml (CSF).

[Example 28] Measurement of Ka value, preparation of handsheets used for physical tests of pulp, and physical tests of handmade pulp sheets

Kappa value was measured in accordance with JIS P 8211. Handmade sheets used for physical tests of pulp were prepared in accordance with Tappi test method T205-om81, and the physical tests of handmade pulp sheets were carried out in accordance with Tappi test T220om-83. As shown in Table 4 below, when woodchips were treated with a transformant or wild type strain, the Ka value was decreased after cooking, the screened yield was increased, and the screened rejects was decreased. In addition, as shown in Table 5 below, when the transformant was compared with the wild type strain, the transformant did not cause decrease in paper strength.

Table 4

| Effects of woodchip treatment with microorganisms having suppressed cellobiose dehydrogenase activity on cooking | | | |
|---|---|---|---|
| | Control | Transformant | Wild type strain |
| Ka value after cooking | 20.1 | 17.6 | 17.7 |
| Screened yield (%) | 45.7 | 47.8 | 47.3 |
| Screened rejects (%) | 1.20 | 0.65 | 0.84 |

Table 5

| Effects of woodchip treatment with microorganisms having suppressed cellobiose dehydrogenase activity on paper strength Whiteness degree = 86, and CSF (Canadian Shopper Freeness) = 410 ml | | | |
|---|---|---|---|
| | Control | Transformant | Wild type strain |
| PFI (rev) | 2,600 | 2,200 | 2,200 |
| Tear index ($mN\cdot m^2/g$) | 9.4 | 9.3 | 8.5 |
| Breaking length (km) | 8.62 | 8.71 | 7.34 |
| Burst index ($kPa\cdot m^2/g$) | 6.76 | 7.72 | 7.54 |
| Folding endurance (logT) | 2.31 | 2.35 | 2.19 |
| Note) Control means a pulp sheet produced by a woodchip treatment wherein no microorganism treatment was carried out. | | | |

[Example 29] Selection of transformant having suppressed cellobiohydrolase I activity

Transformation was carried out as described in the transformation method in the above Example 22 using the plasmid pGPCBHI26AM produced in Example 14 instead of the plasmid pGPCDH1. The obtained transformant was cultured by the same method as in Example 24. Thereafter, applying a cellobiohydrolase I activity measurement method using 4-methyl-O-umbellifferyl-cellobioside as a substrate, the activity of cellobiohydrolase I was measured with time. As a result, it was found that a transformant having at maximum 60% suppressed cellobiohydrolase I activity could be obtained.

[Example 30] Treatment of woodchips with transformant having suppressed cellobiohydrolase I activity

Woodchips made from Eucalyptus lumbers were treated with the transformant strain having a suppressed cellobiohydrolase I activity selected in Example 29 according to the same method as in Example 25.

[Example 31] Production of mechanical pulp using transformant having suppressed cellobiohydrolase I activity

Woodchips made from radiata pine lumbers were treated in the same manner as in Example 25. The treated woodchips were subjected to the same test as in Example 26. As shown in Table 6 below, use of a transformant with a suppressed cellobiohydrolase I activity could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 6

| Effects of woodchip treatment with microorganisms having suppressed cellobiohydrolase I activity on mechanical pulp | | | |
|---|---|---|---|
| | Control | Transformant | Wild type strain |
| Chip yield (%) | 99.8 | 98.7 | 94.7 |
| Refining energy (Kw·h/ton) | 2560 | 1782 | 1840 |

Table 6 (continued)

| Effects of woodchip treatment with microorganisms having suppressed cellobiohydrolase I activity on mechanical pulp | | | |
|---|---|---|---|
| | Control | Transformant | Wild type strain |
| Tear index (mN·m$^2$/g) | 7.92 | 8.02 | 6.95 |
| Burst index (kPa·m$^2$/g) | 1.35 | 1.50 | 1.21 |

[Example 32] Cooking of woodchips treated with transformant having suppressed cellobiohydrolase I activity

The woodchips treated in Example 30 were cooked by the same method as in Example 27.

[Example 33] Measurement of Ka value, preparation of handsheets used for physical tests of pulp, and physical tests of handmade pulp sheets

Measurement of the kappa value or the like was carried out on the pulp obtained in Example 32 in the same manner as described in Example 28. As shown in Table 7 below, when woodchips were treated with a transformant or wild type strain, the Ka value was decreased after cooking, the Screened yield was increased, and the Screened rejects was decreased. In addition, as shown in Table 8 below, when the transformant was compared with the wild type strain, the transformant did not cause decrease in paper strength.

Table 7

| Effects of woodchip treatment with microorganisms having suppressed cellobiohydrolase I activity on cooking | | | |
|---|---|---|---|
| | Control | Transformant | Wild type strain |
| Ka value after cooking | 20.1 | 17.7 | 17.7 |
| Screened yield (%) | 45.7 | 47.9 | 47.3 |
| Screened rejects (%) | 1.20 | 0.75 | 0.84 |

Table 8

| Effects of woodchip treatment with microorganisms having suppressed cellobiohydrolase I activity on paper strength Whiteness degree = 86, and CSF (Canadian Shopper Freeness) = 410 ml | | | |
|---|---|---|---|
| | Control | Transformant | Wild type strain |
| PFI (rev) | 2,600 | 2,300 | 2,200 |
| Tear index (mN·m$^2$/g) | 9.4 | 9.4 | 8.5 |
| Breaking length (km) | 8.62 | 8.51 | 7.34 |
| Burst index (kPa·m$^2$/g) | 6.76 | 7.82 | 7.54 |
| Folding endurance (logT) | 2.31 | 2.34 | 2.19 |
| Note) Control means a pulp sheet produced by a woodchip treatment wherein no microorganism treatment was carried out. | | | |

[Example 34] Selection of transformant having suppressed cellobiohydrolase I activity

Transformation was carried out as described in the transformation method in the above Example 22 using the plasmid pGPCBHI27AM produced in Example 15 instead of the plasmid pGPCDH1. The obtained transformant was cultured by the same method as in Example 26. Thereafter, applying a cellobiohydrolase I activity measurement method using 4-methyl-O-umbelliferyl-cellobioside as a substrate, the activity of cellobiohydrolase I was measured with time. As a result, it was found that a transformant having at maximum 70% suppressed cellobiohydrolase I activity could be obtained.

[Example 35] Treatment of wood chips with transformant having suppressed cellobiohydrolase I activity

Woodchips made from Eucalyptus lumbers were treated with the transformant strain having a suppressed cellobiohydrolase I activity selected in Example 34 according to the same method as in Example 25.

[Example 36] Production of mechanical pulp using transformant having suppressed cellobiohydrolase I activity

Woodchips made from radiata pine lumbers were treated with the transformant obtained in Example 34 in the same manner as in Example 25. The treated woodchips were subjected to the same test as in Example 26. As shown in Table 9 below, use of a transformant with a suppressed cellobiohydrolase I activity could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 9

| Effects of woodchip treatment with microorganisms having suppressed cellobiohydrolase I activity on mechanical pulp | | | |
|---|---|---|---|
| | Control | Transformant | Wild type strain |
| Chip yield (%) | 99.8 | 98.5 | 94.7 |
| Refining energy (Kw·h/ton) | 2560 | 1752 | 1840 |
| Tear index (mN·$m^2$/g) | 7.92 | 8.11 | 6.95 |
| Burst index (kPa·$m^2$/g) | 1.35 | 1.42 | 1.21 |

[Example 37] Cooking of woodchips treated with transformant having suppressed cellobiohydrolase I activity

Woodchips made from Eucalyptus lumbers were treated in the same manner as in Example 25. Thereafter, the treated woodchips were cooked by the same method as in Example 27.

[Example 38] Measurement of Ka value, preparation of handsheets used for physical tests of pulp, and physical tests of handmade pulp sheets

Measurement of the kappa value or the like was carried out on the pulp obtained in Example 37 in the same manner as described in Example 28. As shown in Table 10 below, when woodchips were treated with a transformant or wild type strain, the Ka value was decreased after cooking, the Screened yield was increased, and the Screened rejects was decreased. In addition, as shown in Table 11 below, when the transformant was compared with the wild type strain, the transformant did not cause decrease in paper strength.

Table 10

| Effects of woodchip treatment with microorganisms having suppressed cellobiohydrolase I activity on cooking | | | |
|---|---|---|---|
| | Control | Transformant | Wild type strain |
| Ka value after cooking | 20.1 | 17.5 | 17.7 |
| Screened yield (%) | 45.7 | 48.2 | 47.3 |
| Screened rejects (%) | 1.20 | 0.45 | 0.84 |

Table 11

| Effects of woodchip treatment with microorganisms having suppressed cellobiohydrolase I activity on paper strength Whiteness degree = 86, and CSF (Canadian Shopper Freeness) = 410 ml | | | |
|---|---|---|---|
| | Control | Transformant | Wild type strain |
| PFI (rev) | 2,600 | 2,300 | 2,200 |
| Tear index (mN·$m^2$/g) | 9.4 | 9.1 | 8.5 |

Table 11 (continued)

| Effects of woodchip treatment with microorganisms having suppressed cellobiohydrolase I activity on paper strength Whiteness degree = 86, and CSF (Canadian Shopper Freeness) = 410 ml | | | |
|---|---|---|---|
| | Control | Transformant | Wild type strain |
| Breaking length (km) | 8.62 | 8.54 | 7.34 |
| Burst index (kPa·$m^2$/g) | 6.76 | 7.75 | 7.54 |
| Folding endurance (logT) | 2.31 | 2.31 | 2.19 |
| Note) Control means a pulp sheet produced by a woodchip treatment wherein no microorganism treatment was carried out. | | | |

[Example 39] Selection of transformant having suppressed cellobiohydrolase I activity

Transformation was carried out as described in the transformation method in the above Example 22 using the plasmid pGPCBHI31 AM produced in Example 16 instead of the plasmid pGPCDH1. The obtained transformant was cultured by the same method as in Example 24. Thereafter, applying a cellobiohydrolase I activity measurement method using 4-methyl-O-umbellifferyl-cellobioside as a substrate, the activity of cellobiohydrolase I was measured with time. As a result, it was found that a transformant having at maximum 70% suppressed cellobiohydrolase I activity could be obtained.

[Example 40] Treatment of woodchips with transformant having suppressed cellobiohydrolase I activity

Woodchips made from Eucalyptus lumbers were treated with the transformant strain having a suppressed cellobiohydrolase I activity selected in Example 39 according to the same method as in Example 25.

[Example 41] Production of mechanical pulp using transformant having suppressed cellobiohydrolase I activity

Woodchips made from radiata pine lumbers were treated with the transformant obtained in Example 39 in the same manner as in Example 25. The treated woodchips were subjected to the same test as in Example 26.

As shown in Table 12 below, use of a transformant with a suppressed cellobiohydrolase I activity could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 12

| Effects of woodchip treatment with microorganisms having suppressed cellobiohydrolase I activity on mechanical pulp | | | |
|---|---|---|---|
| | Control | Transformant | Wild type strain |
| Chip yield (%) | 99.8 | 98.3 | 94.7 |
| Refining energy (Kw·h/ton) | 2560 | 1820 | 1840 |
| Tear index (mN·$m^2$/g) | 7.92 | 8.35 | 6.95 |
| Burst index (kPa·$m^2$/g) | 1.35 | 1.43 | 1.21 |

[Example 42] Cooking of woodchips treated with transformant having suppressed cellobiohydrolase I activity

The woodchips obtained in Example 39 were cooked by the same method as in Example 27.

[Example 43] Measurement of Ka value, preparation of handsheets used for physical tests of pulp, and physical tests of handmade pulp sheets

Measurement of the kappa value or the like was carried out on the pulp obtained in Example 42 in the same manner as described in Example 28. As shown in Table 13 below, when woodchips were treated with a transformant or wild type strain, the Ka value was decreased after cooking, the Screened yield was increased, and the Screened rejects was decreased. In addition, as shown in Table 14 below, when the transformant was compared with the wild type strain, the transformant did not cause decrease in paper strength.

Table 13

| Effects of woodchip treatment with microorganisms having suppressed cellobiohydrolase I activity on cooking | | | |
|---|---|---|---|
| | Control | Transformant | Wild type strain |
| Ka value after cooking | 20.1 | 17.8 | 17.7 |
| Screened yield (%) | 45.7 | 47.4 | 47.3 |
| Screened rejects (%) | 1.20 | 0.63 | 0.84 |

Table 14

| Effects of woodchip treatment with microorganisms having suppressed cellobiohydrolase I activity on paper strength Whiteness degree = 86, and CSF (Canadian Shopper Freeness) = 410 ml | | | |
|---|---|---|---|
| | Control | Transformant | Wild type strain |
| PFI (rev) | 2,600 | 2,200 | 2,200 |
| Tear index (mN·$m^2$/g) | 9.4 | 9.1 | 8.5 |
| Breaking length (km) | 8.62 | 8.61 | 7.34 |
| Burst index (kPa·$m^2$/g) | 6.76 | 7.73 | 7.54 |
| Folding endurance (logT) | 2.31 | 2.35 | 2.19 |
| Note) Control means a pulp sheet produced by a woodchip treatment wherein no microorganism treatment was carried out. | | | |

[Example 44] Selection of transformant having suppressed cellobiohydrolase II activity

Transformation was carried out as described in the transformation method in the above Example 22 using the plasmid pGPCBHIIAM produced in Example 17 instead of the plasmid pGPCDH1. The obtained transformant was cultured by the same method as in Example 23. The cultured cell bodies were sampled periodically, and mRNA was recovered, and the amount of the expression of cellobiohydrolase II gene was measured. As a result, it was found that a transformant could be obtained, which had a suppressed cellobiohydrolase II activity that was approximately 50% less than that of the host cells.

[Example 45] Treatment of woodchips with transformant having suppressed cellobiohydrolase II activity

Woodchips made from Eucalyptus lumbers were treated with the transformant strain having a suppressed cellobiohydrolase II activity selected in Example 44 according to the same method as in Example 25.

[Example 46] Production of mechanical pulp using transformant having suppressed cellobiohydrolase II activity

Woodchips made from radiata pine lumbers were treated with the transformant obtained in Example 44 in the same manner as in Example 25. Analysis of the paper strength or the like was carried out on the treated woodchips according to the method described in Example 26. As shown in Table 15 below, use of a transformant with a suppressed cellobiohydrolase II activity could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 15

| Effects of woodchip treatment with microorganisms having suppressed cellobiohydrolase II activity on mechanical pulp | | | |
|---|---|---|---|
| | Control | Transformant | Wild type strain |
| Chip yield (%) | 99.8 | 98.1 | 94.7 |

Table 15 (continued)

| Effects of woodchip treatment with microorganisms having suppressed cellobiohydrolase II activity on mechanical pulp | | | |
|---|---|---|---|
| | Control | Transformant | Wild type strain |
| Refining energy (Kw·h/ton) | 2560 | 1830 | 1840 |
| Tear index (mN·$m^2$/g) | 7.92 | 8.25 | 6.95 |
| Burst index (kPa·$m^2$/g) | 1.35 | 1.44 | 1.21 |

[Example 47] Cooking of woodchips treated with transformant having suppressed cellobiohydrolase II activity

The woodchips obtained in Example 45 were cooked by the same method as in Example 27.

[Example 48] Measurement of Ka value, preparation of handsheets used for physical tests of pulp, and physical tests of handmade pulp sheets

Measurement of the kappa value or the like was carried out on the pulp obtained in Example 47 in the same manner as described in Example 28. As shown in Table 16 below, when woodchips were treated with a transformant or wild type strain, the Ka value was decreased after cooking, the Screened yield was increased, and the Screened rejects was decreased. In addition, as shown in Table 17 below, when the transformant was compared with the wild type strain, the transformant did not cause decrease in paper strength.

Table 16

| Effects of woodchip treatment with microorganisms having suppressed cellobiohydrolase II activity on cooking | | | |
|---|---|---|---|
| | Control | Transformant | Wild type strain |
| Ka value after cooking | 20.1 | 18.1 | 17.7 |
| Screened yield (%) | 45.7 | 47.1 | 47.3 |
| Screened rejects (%) | 1.20 | 0.86 | 0.84 |

Table 17

| Effects of woodchip treatment with microorganisms having suppressed cellobiohydrolase II activity on paper strength Whiteness degree = 86, and CSF (Canadian Shopper Freeness) = 410 ml | | | |
|---|---|---|---|
| | Control | Transformant | Wild type strain |
| PFI (rev) | 2,600 | 2,300 | 2,200 |
| Tear index (mN·$m^2$/g) | 9.4 | 8.7 | 8.5 |
| Breaking length (km) | 8.62 | 8.45 | 7.34 |
| Burst index (kPa·$m^2$/g) | 6.76 | 7.49 | 7.54 |
| Folding endurance (logT) | 2.31 | 2.32 | 2.19 |
| Note) Control means a pulp sheet produced by a woodchip treatment wherein no microorganism treatment was carried out. | | | |

[Example 49] Selection of transformant having suppressed activity of engoglucanase belonging to glycolytic enzyme family 61

Transformation was carried out as described in the transformation method in the above Example 22 using the plasmid pGPEG61AM produced in Example 18 instead of the plasmid pGPCDH1. The obtained transformant was cultured by the same method as in Example 23. The culture solution was sampled with time, and the carboxymethyl cellulose (CMC)-decomposing activity was measured. As a result, it was found that a transformant could be obtained, which had a suppressed endoglucanase activity that was approximately 50% less than that of the host cells.

[Example 50] Treatment of woodchips with transformant having suppressed activity of endoglucanase belonging to glycolytic enzyme family 61

Woodchips made from Eucalyptus lumbers were treated with the transformant strain having a suppressed activity of endoglucanase belonging to glycolytic enzyme family 61 selected in Example 49 according to the method described in Example 25.

[Example 51] Production of mechanical pulp using transformant having suppressed activity of endoglucanase belonging to glycolytic enzyme family 61

Woodchips made from radiata pine lumbers were treated with the transformant obtained in Example 49 in the same manner as in Example 25. Analysis of the paper strength or the like was carried out on the treated woodchips by the method described in Example 26. As shown in Table 18 below, use of a transformant with a suppressed activity of endoglucanase belonging to glycolytic enzyme family 61 could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 18

| Effects of woodchip treatment with microorganisms having suppressed activity of endoglucanase belonging to glycolytic enzyme family 61 on mechanical pulp | | | |
|---|---|---|---|
| | Control | Transformant | Wild type strain |
| Chip yield (%) | 99.8 | 98.6 | 94.7 |
| Refining energy (Kw·h/ton) | 2560 | 1800 | 1840 |
| Tear index (mN·$m^2$/g) | 7.92 | 8.21 | 6.95 |
| Burst index (kPa·$m^2$/g) | 1.35 | 1.41 | 1.21 |

[Example 52] Cooking of woodchips treated with transformant having suppressed activity of endoglucanase belonging to glycolytic enzyme family 61

The woodchips obtained in Example 50 were cooked by the same method as in Example 27.

[Example 53] Measurement of Ka value, preparation of handsheets used for physical tests of pulp, and physical tests of handmade pulp sheets

Measurement of the kappa value or the like was carried out on the pulp obtained in Example 52 in the same manner as described in Example 28. As shown in Table 19 below, when woodchips were treated with a transformant or wild type strain, the Ka value was decreased after cooking, the Screened yield was increased, and the Screened rejects was decreased. In addition, as shown in Table 20 below, when the transformant was compared with the wild type strain, the transformant did not cause decrease in paper strength.

Table 19

| Effects of woodchip treatment with microorganisms having suppressed activity of endoglucanase belonging to glycolytic enzyme family 61 on cooking | | | |
|---|---|---|---|
| | Control | Transformant | Wild type strain |
| Ka value after cooking | 20.1 | 17.7 | 17.7 |
| Screened yield (%) | 45.7 | 47.6 | 47.3 |
| Screened rejects (%) | 1.20 | 0.73 | 0.84 |

Table 20

| Effects of woodchip treatment with microorganisms having suppressed activity of endoglucanase belonging to glycolytic enzyme family 61 on paper strength<br>Whiteness degree = 86, and CSF (Canadian Shopper Freeness) = 410 ml | | | |
|---|---|---|---|
| | Control | Transformant | Wild type strain |
| PFI (rev) | 2,600 | 2,200 | 2,200 |
| Tear index (mN·m$^2$/g) | 9.4 | 9.5 | 8.5 |
| Breaking length (km) | 8.62 | 8.63 | 7.34 |
| Burst index (kPa·m$^2$/g) | 6.76 | 7.63 | 7.54 |
| Folding endurance (logT) | 2.31 | 2.33 | 2.19 |
| Note) Control means a pulp sheet produced by a woodchip treatment wherein no microorganism treatment was carried out. | | | |

[Example 54] Selection of transformant having suppressed activity of engoglucanase belonging to glycolytic enzyme family 12

Transformation was carried out as described in the transformation method in the above Example 22 using the plasmid pGPEG12AM produced in Example 19 instead of the plasmid pGPCDH1. The obtained transformant was cultured by the same method as in Example 23. The culture solution was sampled with time, and the carboxymethyl cellulose (CMC)-decomposing activity was measured. As a result, it was found that a transformant could be obtained, which had a suppressed endoglucanase activity that was approximately 50% less than that of the host cells.

[Example 55] Treatment of woodchips with transformant having suppressed activity of endoglucanase belonging to glycolytic enzyme family 12

Woodchips made from Eucalyptus lumbers were treated with the transformant strain having a suppressed activity of endoglucanase belonging to glycolytic enzyme family 12 selected in Example 54 according to the method described in Example 25.

[Example 56] Production of mechanical pulp using transformant having suppressed activity of endoglucanase belonging to glycolytic enzyme family 12

Woodchips made from radiata pine lumbers were treated with the transformant obtained in Example 54 in the same manner as in Example 25. Analysis of the paper strength or the like was carried out on the treated woodchips by the method described in Example 26. As shown in Table 21 below, use of a transformant with a suppressed activity of endoglucanase belonging to glycolytic enzyme family 12 could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 21

| Effects of woodchip treatment with microorganisms having suppressed activity of endoglucanase belonging to glycolytic enzyme family 12 on mechanical pulp | | | |
|---|---|---|---|
| | Control | Transformant | Wild type strain |
| Chip yield (%) | 99.8 | 98.5 | 94.7 |
| Refining energy (Kw·h/ton) | 2560 | 1860 | 1840 |
| Tear index (mN·m$^2$/g) | 7.92 | 8.06 | 6.95 |
| Burst index (kPa·m$^2$/g) | 1.35 | 1.43 | 1.21 |

[Example 57] Cooking of woodchips treated with transformant having suppressed activity of endoglucanase belonging to glycolytic enzyme family 12

The woodchips obtained in Example 55 were cooked by the same method as in Example 27.

[Example 58] Measurement of Ka value, preparation of handsheets used for physical tests of pulp, and physical tests of handmade pulp sheets

Measurement of the kappa value or the like was carried out on the pulp obtained in Example 56 in the same manner as described in Example 28. As shown in Table 22 below, when woodchips were treated with a transformant or wild type strain, the Ka value was decreased after cooking, the Screened yield was increased, and the Screened rejects was decreased. In addition, as shown in Table 23 below, when the transformant was compared with the wild type strain, the transformant did not cause decrease in paper strength.

Table 22

| Effects of woodchip treatment with microorganisms having suppressed activity of endoglucanase belonging to glycolytic enzyme family 12 on cooking | | | |
|---|---|---|---|
| | Control | Transformant | Wild type strain |
| Ka value after cooking | 20.1 | 18.2 | 17.7 |
| Screened yield (%) | 45.7 | 47.1 | 47.3 |
| Screened rejects (%) | 1.20 | 0.93 | 0.84 |

Table 23

| Effects of woodchip treatment with microorganisms having suppressed activity of endoglucanase belonging to glycolytic enzyme family 12 on paper strength<br>Whiteness degree = 86, and CSF (Canadian Shopper Freeness) = 410 ml | | | |
|---|---|---|---|
| | Control | Transformant | Wild type strain |
| PFI (rev) | 2,600 | 2,400 | 2,200 |
| Tear index (mN·$m^2$/g) | 9.4 | 9.3 | 8.5 |
| Breaking length (km) | 8.62 | 8.58 | 7.34 |
| Burst index (kPa·$m^2$/g) | 6.76 | 7.63 | 7.54 |
| Folding endurance (logT) | 2.31 | 2.31 | 2.19 |
| Note) Control means a pulp sheet produced by a woodchip treatment wherein no microorganism treatment was carried out. | | | |

[Example 59] Selection of transformant having suppressed activity of engoglucanase belonging to glycolytic enzyme family 5

Transformation was carried out as described in the transformation method in the above Example 22 using the plasmid pGPPCEG5AM produced in Example 20 instead of the plasmid pGPCDH1. The obtained transformant was cultured by the same method as in Example 23. The culture solution was sampled with time, and the carboxymethyl cellulose (CMC)-decomposing activity was measured. As a result, it was found that a transformant could be obtained, which had a suppressed endoglucanase activity that was approximately 20% less than that of the host cells.

[Example 60] Treatment of woodchips with transformant having suppressed activity of endoglucanase belonging to glycolytic enzyme family 5

Woodchips made from Eucalyptus lumbers were treated with the transformant strain having a suppressed activity of endoglucanase belonging to glycolytic enzyme family 5 selected in Example 59 according to the method described in Example 25.

[Example 61] Production of mechanical pulp using transformant having suppressed activity of endoglucanase belonging to glycolytic enzyme family 5

Woodchips made from radiata pine lumbers were treated with the transformant obtained in Example 59 in the same manner as in Example 25. Analysis of the paper strength or the like was carried out on the treated woodchips by the method described in Example 26.

As shown in Table 24 below, use of a transformant with a suppressed activity of endoglucanase belonging to glycolytic enzyme family 5 could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 24

| Effects of woodchip treatment with microorganisms having suppressed activity of endoglucanase belonging to glycolytic enzyme family 5 on mechanical pulp | | | |
|---|---|---|---|
| | Control | Transformant | Wild type strain |
| Chip yield (%) | 99.8 | 98.2 | 94.7 |
| Refining energy (Kw·h/ton) | 2560 | 1910 | 1840 |
| Tear index (mN·$m^2$/g) | 7.92 | 8.05 | 6.95 |
| Burst index (kPa·$m^2$/g) | 1.35 | 1.34 | 1.21 |

[Example 62] Cooking of woodchips treated with transformant having suppressed activity of endoglucanase belonging to glycolytic enzyme family 5

The woodchips obtained in Example 60 were cooked by the same method as in Example 27.

[Example 63] Measurement of Ka value, preparation of handsheets used for physical tests of pulp, and physical tests of handmade pulp sheets

Measurement of the kappa value or the like was carried out on the pulp obtained in Example 62 in the same manner as described in Example 28. As shown in Table 25 below, when woodchips were treated with a transformant or wild type strain, the Ka value was decreased after cooking, the Screened yield was increased, and the Screened rejects was decreased. In addition, as shown in Table 26 below, when the transformant was compared with the wild type strain, the transformant did not cause decrease in paper strength.

Table 25

| Effects of woodchip treatment with microorganisms having suppressed activity of endoglucanase belonging to glycolytic enzyme family 5 on cooking | | | |
|---|---|---|---|
| | Control | Transformant | Wild type strain |
| Ka value after cooking | 20.1 | 17.9 | 17.7 |
| Screened yield (%) | 45.7 | 46.8 | 47.3 |
| Screened rejects (%) | 1.20 | 0.95 | 0.84 |

Table 26

| Effects of woodchip treatment with microorganisms having suppressed activity of endoglucanase belonging to glycolytic enzyme family 5 on paper strength<br>Whiteness degree = 86, and CSF (Canadian Shopper Freeness) = 410 ml | | | |
|---|---|---|---|
| | Control | Transformant | Wild type strain |
| PFI (rev) | 2,600 | 2,300 | 2,200 |
| Tear index (mN·$m^2$/g) | 9.4 | 9.4 | 8.5 |

Table 26 (continued)

| Effects of woodchip treatment with microorganisms having suppressed activity of endoglucanase belonging to glycolytic enzyme family 5 on paper strength<br>Whiteness degree = 86, and CSF (Canadian Shopper Freeness) = 410 ml | | | |
|---|---|---|---|
| | Control | Transformant | Wild type strain |
| Breaking length (km) | 8.62 | 8.55 | 7.34 |
| Burst index ($kPa \cdot m^2/g$) | 6.76 | 7.36 | 7.54 |
| Folding endurance (logT) | 2.31 | 2.26 | 2.19 |

Note) Control means a pulp sheet produced by a woodchip treatment wherein no microorganism treatment was carried out.

[Example 64] Selection of transformant having suppressed activity of engoglucanase belonging to glycolytic enzyme family 9

Transformation was carried out as described in the transformation method in the above Example 22 using the plasmid pGPPCEG9AM produced in Example 21 instead of the plasmid pGPCDH1. The obtained transformant was cultured by the same method as in Example 23. The culture solution was sampled with time, and the carboxymethyl cellulose (CMC)-decomposing activity was measured. As a result, it was found that a transformant could be obtained, which had a suppressed endoglucanase activity that was approximately 30% less than that of the host cells.

[Example 65] Treatment of woodchips with transformant having suppressed activity of endoglucanase belonging to glycolytic enzyme family 9

Woodchips made from Eucalyptus lumbers were treated with the transformant strain having a suppressed activity of endoglucanase belonging to glycolytic enzyme family 9 selected in Example 64 according to the method described in Example 25.

[Example 66] Production of mechanical pulp using transformant having suppressed activity of endoglucanase belonging to glycolytic enzyme family 9

Woodchips made from radiata pine lumbers were treated with the transformant obtained in Example 64 in the same manner as in Example 25. Analysis of the paper strength or the like was carried out on the treated woodchips by the method described in Example 26.

As shown in Table 27 below, use of a transformant with a suppressed activity of endoglucanase belonging to glycolytic enzyme family 9 could control reduction in chip yield and could reduce refining energy. In addition, both tearing strength and bursting strength were increased. In contrast, when woodchips were treated with a wild type strain, effects of reducing refining energy could be obtained, but paper strength was decreased.

Table 27

| Effects of woodchip treatment with microorganisms having suppressed activity of endoglucanase belonging to glycolytic enzyme family 9 on mechanical pulp | | | |
|---|---|---|---|
| | Control | Transformant | Wild type strain |
| Chip yield (%) | 99.8 | 98.1 | 94.7 |
| Refining energy (Kw·h/ton) | 2560 | 1860 | 1840 |
| Tear index ($mN \cdot m^2/g$) | 7.92 | 7.98 | 6.95 |
| Burst index ($kPa \cdot m^2/g$) | 1.35 | 1.36 | 1.21 |

[Example 67] Cooking of woodchips treated with transformant having suppressed activity of endoglucanase belonging to glycolytic enzyme family 9

The woodchips obtained in Example 65 were cooked by the same method as in Example 27.

[Example 68] Measurement of Ka value, preparation of handsheets used for physical tests of pulp, and physical tests of handmade pulp sheets

Measurement of the kappa value or the like was carried out on the pulp obtained in Example 67 in the same manner as described in Example 28. As shown in Table 28 below, when woodchips were treated with a transformant or wild type strain, the Ka value was decreased after cooking, the Screened yield was increased, and the Screened rejects was decreased. In addition, as shown in Table 29 below, when the transformant was compared with the wild type strain, the transformant did not cause decrease in paper strength.

Table 28

| Effects of woodchip treatment with microorganisms having suppressed activity of endoglucanase belonging to glycolytic enzyme family 9 on cooking | | | |
|---|---|---|---|
| | Control | Transformant | Wild type strain |
| Ka value after cooking | 20.1 | 18.1 | 17.7 |
| Screened yield (%) | 45.7 | 46.7 | 47.3 |
| Screened rejects (%) | 1.20 | 0.86 | 0.84 |

Table 29

| Effects of woodchip treatment with microorganisms having suppressed activity of endoglucanase belonging to glycolytic enzyme family 9 on paper strength<br>Whiteness degree = 86, and CSF (Canadian Shopper Freeness) = 410 ml | | | |
|---|---|---|---|
| | Control | Transformant | Wild type strain |
| PFI (rev) | 2,600 | 2,200 | 2,200 |
| Tear index (mN·$m^2$/g) | 9.4 | 9.6 | 8.5 |
| Breaking length (km) | 8.62 | 8.44 | 7.34 |
| Burst index (kPa·$m^2$/g) | 6.76 | 7.45 | 7.54 |
| Folding endurance (logT) | 2.31 | 2.25 | 2.19 |
| Note) Control means a pulp sheet produced by a woodchip treatment wherein no microorganism treatment was carried out. | | | |

[Example 69] Cellobiose dehydrogenase activity

1. Summary of measurement method

Cellobiose dehydrogenase activity was measured as follows. A solution was produced by mixing 250 ul of 0.67 mM dichlorophenolindophenol (manufactured by Sigma Chemical Company), 100 ul of 3.33 mM cellobiose (manufactured by Kanto Kagaku), and 100 ul of a 250 mM acetate buffer solution of pH 5, and thereafter, 50 ul of a test solution was added to the mixed solution, followed by reaction at 37°C. After initiation of the reaction, absorbance (optical length: 1 cm) at 550 nm (molar absorption coefficient: 3965 L/mol/cm) as the maximum absorption wavelength of dichlorophenolindophenol, was continuously measured: With regard to the activity unit of cellobiose dehydrogenase, the amount of enzyme necessary for decreasing 1 umol dichlorophenolindophenol per minute under the above conditions was defined as 1 unit (unit: U).

2. Preparation of crude enzyme solution (1)

100 ml of a liquid medium (pH 5.0) containing 1.0% hardwood oxygen-bleached kraft pulp (kappa value: 8.5, whiteness degree: 46.0%), 1.0% peptone, 0.005% $MgSO_4 \cdot 7H_2O$, 0.15% $KH_2PO_4$, and 20 ppb thiamine hydrochloride was placed in a 500 ml-volume Erlenmeyer flask, and the flask was then closed with a paper, followed by steam sterilization at 121°C for 15 minutes. A *Coriolus hirsutus* IFO4917 strain was inoculated into the resultant product using an inoculating loop, followed by a rotary shaking culture at 27°C (amplitude: 25 mm, 120 reciprocations/minute). After completion of the culture, the culture product was subjected to centrifugation (10,000 rpm × 10 minutes) to separate the culture supernatant, thereby obtaining a crude enzyme solution of cellobiose dehydrogenase. The activity of the enzyme was measured under the above conditions. As a result, it was found that the cellobiose dehydrogenase activity in the culture supernatant was 0.06 U/ml at 72 hours after initiation of the culture.

3. Preparation of crude enzyme solution (2)

100 ml of a liquid medium (pH 5.0) containing 1.0% Avicel (manufactured by Funakoshi Co., Ltd.), 1.0% peptone, 0.005%, 0.15% $KH_2PO_4$, and 20 ppb thiamine hydrochloride was placed in a 500 ml-volume Erlenmeyer flask, and the flask was then closed with a paper, followed by steam sterilization at 121°C for 15 minutes. A *Coriolus hirsutus* IFO4917 strain was inoculated into the resultant product using an inoculating loop, followed by a rotary shaking culture at 27°C (amplitude: 25 mm, 120 reciprocations/minute). After completion of the culture, the culture product was subjected to centrifugation (10,000 rpm × 10 minutes) to separate the culture supernatant, thereby obtaining a crude enzyme solution of cellobiose dehydrogenase. The activity of the enzyme was measured under the above conditions. As a result, it was found that the cellobiose dehydrogenase activity in the culture supernatant was 0.07 U/ml at 72 hours after initiation of the culture.

4. Purification of cellobiose dehydrogenase (1)

The crude enzyme solution obtained in Example 1 or 2 was subjected to ammonium sulfate fractionation, and 80% deposit fraction was then recovered by centrifugation (20,000 rpm × 10 minutes). Thereafter, the obtained fraction was dissolved in a 20 mM phosphate buffer solution (pH 6.0). The obtained crude enzyme solution was subjected to hydrophobic chromatography, using Resource 15PHE (diameter 1.6 × 3 cm; manufactured by Amersham) equilibrated with a 20 mM phosphate buffer solution (pH 6.0) containing 1 M ammonium sulfate.

Adsorption fractions were eluted with a 20 mM phosphate buffer solution (pH 6.0) in a concentration gradient of ammonium sulfate from 1 M to 0 M. The obtained fractions were fractionated into 9 ml fractions, and thus, active fractions were obtained. The obtained fractions were then subjected to gel filtration chromatography, using HiLoad26/60 Superdex 200 (diameter 2.6 × 60 cm; manufactured by Amersham) equilibrated with a 20 mM phosphate buffer solution (pH 6.0) containing 100 mM sodium chloride. The chromatography was carried out at a flow rate of 1.5 ml/minute, the samples were fractionated into 3 ml fractions, and the active fractions were obtained.

These fractions were collected and subjected to ion exchange chromatography, using POROS HQ (diameter 4.6 × 10 cm; manufactured by ABI) equilibrated with a 20 mM phosphate buffer solution (pH 6.0). Adsorbed fractions were eluted with the above buffer solution containing sodium chloride in a cosncentration gradient from 0 M to 1 M. The obtained fractions were fractionated into 9 ml fractions, and thus, active fractions were obtained.

The above active fractions were subjected to SDS polyacrylamide electrophoresis. As a result, it could be confirmed that the fractions were uniformly purified. The yield of the purified enzyme was 4.9% with respect to the culture solution, and the specific activity was 10.5 U/mg.

5. Purification of cellobiose dehydrogenase (2)

When the crude enzyme solution obtained in Example 1 or 2 were frozen and then melted, glucan-like substances were deposited. These glucan-like substances were eliminated by centrifugation (20,000 rpm × 10 minutes), and ammonium sulfate was then added such that the concentration of ammonium sulfate became 1 M. The obtained crude enzyme solution was subjected to hydrophobic chromatography, using Resource 15PHE (diameter 1.6 × 3 cm; manufactured by Amersham) equilibrated with a 20 mM phosphate buffer solution (pH 6.0) containing 1 M ammonium sulfate.

Adsorption fractions were eluted with a 20 mM phosphate buffer solution (pH 6.0) in a concentration gradient of ammonium sulfate from 1 M to 0 M. The obtained fractions were fractionated into 9 ml fractions, and thus, active fractions were obtained. The obtained fractions were then subjected to gel filtration chromatography, using HiLoad26/60 Superdex 200 (diameter 2.6 × 60 cm; manufactured by Amersham) equilibrated with a 20 mM phosphate buffer solution (pH 6.0) containing 100 mM sodium chloride. The chromatography was carried out at a flow rate of 1.5 ml/minute, the samples were fractionated into 3 ml fractions, and the active fractions were obtained. These fractions were collected and subjected to ion exchange chromatography, using monoQ HR 5/5 (diameter 0.5 × 5 cm; manufactured by Amersham) equilibrated with a 20 mM phosphate buffer solution (pH 6.0). Adsorbed fractions were eluted with the above buffer solution containing sodium chloride in a concentration gradient from 0 M to 0.4 M. The obtained fractions were fractionated into 1 ml fractions, and thus, active fractions were obtained.

The above active fractions were subjected to SDS polyacrylamide electrophoresis. As a result, it could be confirmed that the fractions were uniformly purified. The yield of the purified enzyme was 16.6% with respect to the culture solution, and the specific activity was 10.5 U/mg.

Industrial Applicability

The present invention provides a gene encoding cellulolytic enzyme derived from Basidiomycete, a transformant transformed with a recombinant vector containing the above gene or an antisense gene of the above gene, and a use thereof. Host cells having a suppressed cellulolytic enzyme activity are prepared by genetic recombination using an antisense gene of the above gene encoding cellulolytic enzyme, and the host cells having a suppressed cellulolytic enzyme activity are used in treatment of woodchips, so as to realize a pulp production method that is excellent in yield and paper strength.

All publications, patents and patent applications cited herein are incorporated herein by reference in their entirety.

## Claims

1. A method for treating woodchips, comprising the steps of:

   preparing a DNA encoding an antisense RNA substantially complementary to the whole or a part of a transcription product of a cellulolytic enzyme gene derived from Basidiomycete;
   preparing a vector comprising (a) said DNA, or (b) a recombinant DNA comprising said DNA and a DNA fragment having a promoter activity, wherein said DNA binds to said DNA fragment such that an antisense RNA of the cellulolytic enzyme gene is generated as a result of transcription;
   transforming host cells with said vector, so as to prepare the host cells having a suppressed cellulolytic enzyme activity; and
   inoculating said host cells having a suppressed cellulolytic enzyme activity into woodchips to treat them.

2. The method according to claim 1, wherein the cellulolytic enzyme gene comprises one or more genes selected from the group consisting of respective genes encoding a cellobiose dehydrogenase, cellobiohydrolase I, cellobiohydrolase II, an endoglucanase belonging to glycolytic enzyme family 61, an endoglucanase belonging to glycolytic enzyme family 12, an endoglucanase belonging to glycolytic enzyme family 5, and an endoglucanase belonging to glycolytic enzyme family 9.

3. The method according to claim 2, wherein the cellobiose dehydrogenase gene is an isolated cellobiose dehydrogenase gene comprising any one of the following nucleotide sequences (a) to (c):

   (a) a nucleotide sequence as shown in SEQ ID No. 1 or 3;
   (b) a nucleotide sequence hybridizing with a nucleotide sequence comprising a nucleotide sequence complementary to the nucleotide sequence according to (a) under stringent conditions, and encoding a protein having a cellobiose dehydrogenase enzyme activity; and
   (c) a nucleotide sequence comprising deletion, substitution or addition of one or more nucleotides with respect to SEQ ID No. 1 or 3, and encoding a protein having a cellobiose dehydrogenase enzyme activity.

4. The method according to claim 2, wherein the cellobiohydrolase I gene is an isolated cellobiohydrolase I gene comprising any one of the following nucleotide sequences (a) to (c):

   (a) a nucleotide sequence as shown in SEQ ID No. 7, 9, or 11;
   (b) a nucleotide sequence hybridizing with a nucleotide sequence comprising a nucleotide sequence complementary to the nucleotide sequence according to (a) under stringent conditions, and encoding a protein having a cellobiohydrolase I gene enzyme activity; and
   (c) a nucleotide sequence comprising deletion, substitution or addition of one or more nucleotides with respect to SEQ ID No. 7, 9 or 11, and encoding a protein having a cellobiohydrolase I gene enzyme activity.

5. The method according to claim 2, wherein the cellobiohydrolase II gene is an isolated cellobiohydrolase II gene comprising any one of the following nucleotide sequences (a) to (c):

   (a) a nucleotide sequence as shown in SEQ ID No. 14;
   (b) a nucleotide sequence hybridizing with a nucleotide sequence comprising a nucleotide sequence complementary to the nucleotide sequence according to (a) under stringent conditions, and encoding a protein having a cellobiohydrolase II gene enzyme activity; and
   (c) a nucleotide sequence comprising deletion, substitution or addition of one or more nucleotides with respect to SEQ ID No. 14, and encoding a protein having a cellobiohydrolase II gene enzyme activity.

6. The method according to claim 2, wherein the endoglucanase gene belonging to glycolytic enzyme family 61 is an isolated endoglucanase gene belonging to glycolytic enzyme family 61 comprising any one of the following nucleotide sequences (a) to (c):

   (a) a nucleotide sequence as shown in SEQ ID No. 18;
   (b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 61; and
   (c) a nucleotide sequence comprising deletion, substitution or addition of one or more nucleotides with respect to SEQ ID No. 18, and encoding a protein having an activity of endoglucanase enzyme belonging to glycolytic enzyme family 61.

**7.** The method according to claim 2, wherein the endoglucanase gene belonging to glycolytic enzyme family 12 is an isolated endoglucanase gene belonging to glycolytic enzyme family 12 comprising any one of the following nucleotide sequences (a) to (c):

(a) a nucleotide sequence as shown in SEQ ID No. 20;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 12; and
(c) a nucleotide sequence comprising deletion, substitution or addition of one or more nucleotides with respect to SEQ ID No. 20, and encoding a protein having an activity of endoglucanase enzyme belonging to glycolytic enzyme family 12.

**8.** The method according to claim 2, wherein the endoglucanase gene belonging to glycolytic enzyme family 5 is an isolated endoglucanase gene belonging to glycolytic enzyme family 5 comprising any one of the following nucleotide sequences (a) to (c):

(a) a nucleotide sequence as shown in SEQ ID No. 24;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 5; and
(c) a nucleotide sequence comprising deletion, substitution or addition of one or more nucleotides with respect to SEQ ID No. 24, and encoding a protein having an activity of endoglucanase enzyme belonging to glycolytic enzyme family 5.

**9.** The method according to claim 2, wherein the endoglucanase gene belonging to glycolytic enzyme family 9 is an isolated endoglucanase gene belonging to glycolytic enzyme family 9 comprising any one of the following nucleotide sequences (a) to (c):

(a) a nucleotide sequence as shown in SEQ ID No. 28;
(b) a nucleotide sequence hybridizing with a nucleotide sequence comprising a nucleotide sequence complementary to the nucleotide sequence according to (a) under stringent conditions, and encoding a protein having an activity of endoglucanase enzyme belonging to glycolytic enzyme family 9; and
(c) a nucleotide sequence comprising deletion, substitution or addition of one or more nucleotides with respect to SEQ ID No. 28, and encoding a protein having an activity of endoglucanase enzyme belonging to glycolytic enzyme family 9.

**10.** The method according to any one of claims 1 to 9, wherein Basidiomycete is *Coriolus hirsutus* or *Phanerochaete chrysosporium*.

**11.** The method according to any one of claims 1 to 10, wherein host cells are *Coriolus hirsutus*.

**12.** A woodchip obtained by the method according to any one of claims 1 to 11.

**13.** A method for producing a pulp using the woodchip according to claim 12.

**14.** A pulp obtained by the method according to claim 13.

Fig. 1

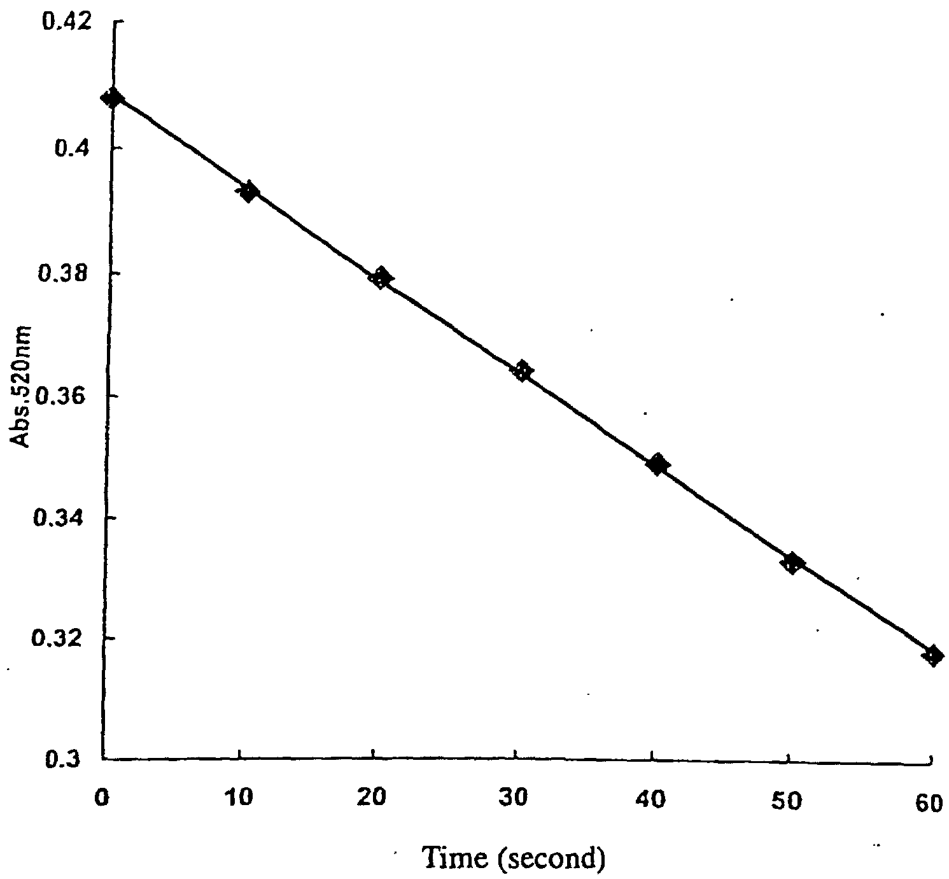
0.42
0.4
0.38
0.36
0.34
0.32
0.3
Abs.520nm
0
10
20
30
40
50
60
Time (second)

Fig. 2

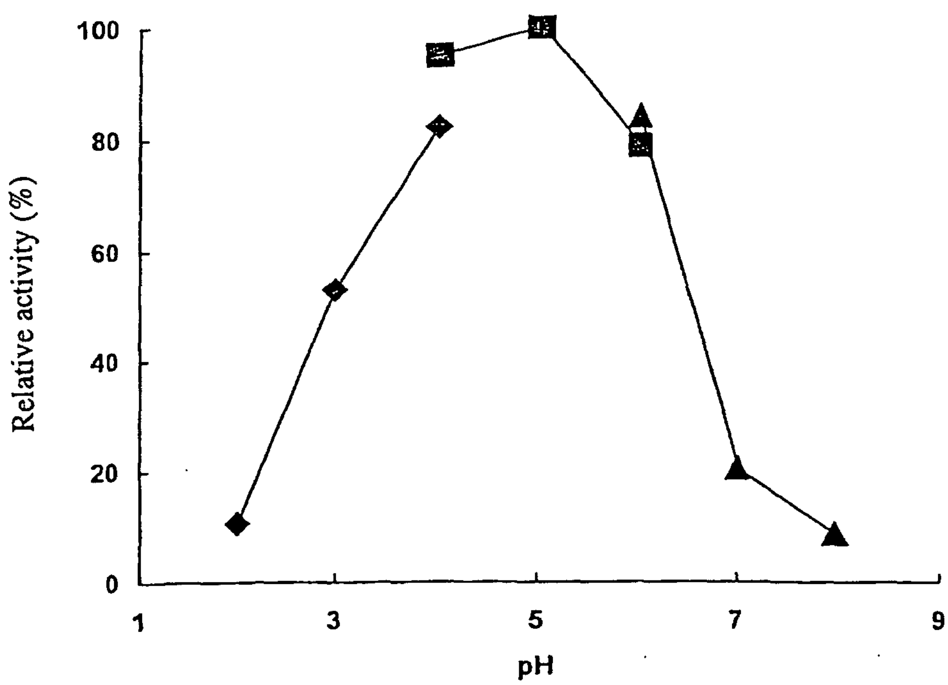

Fig. 3

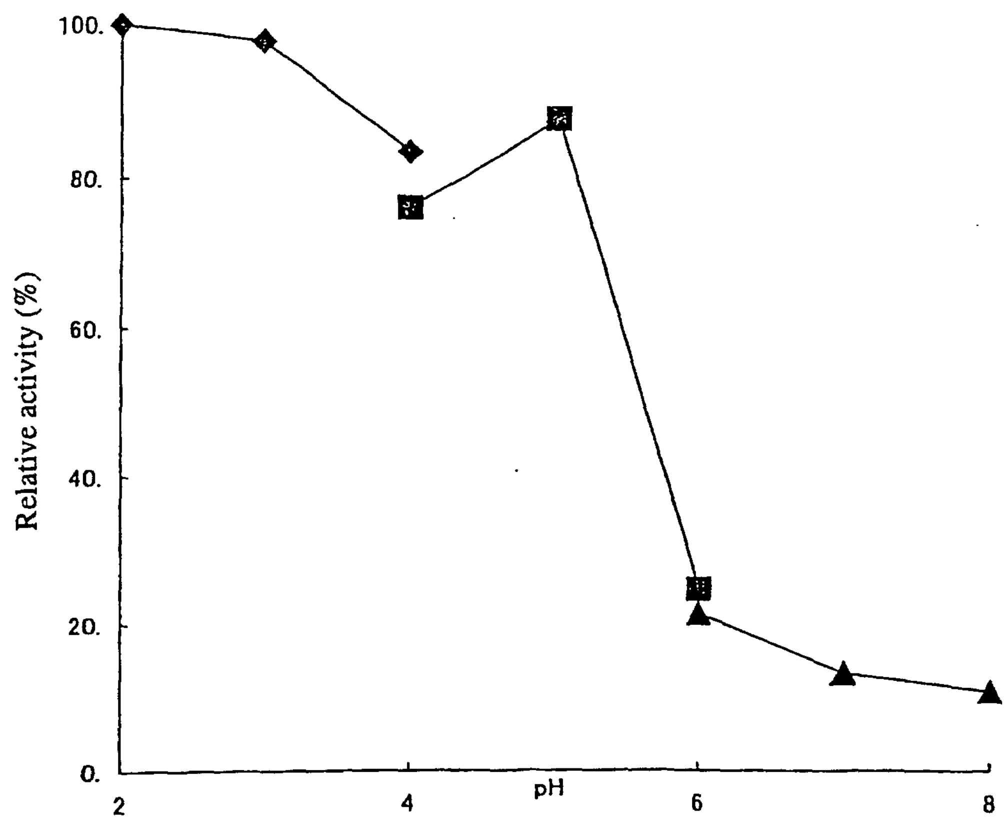

Fig. 4

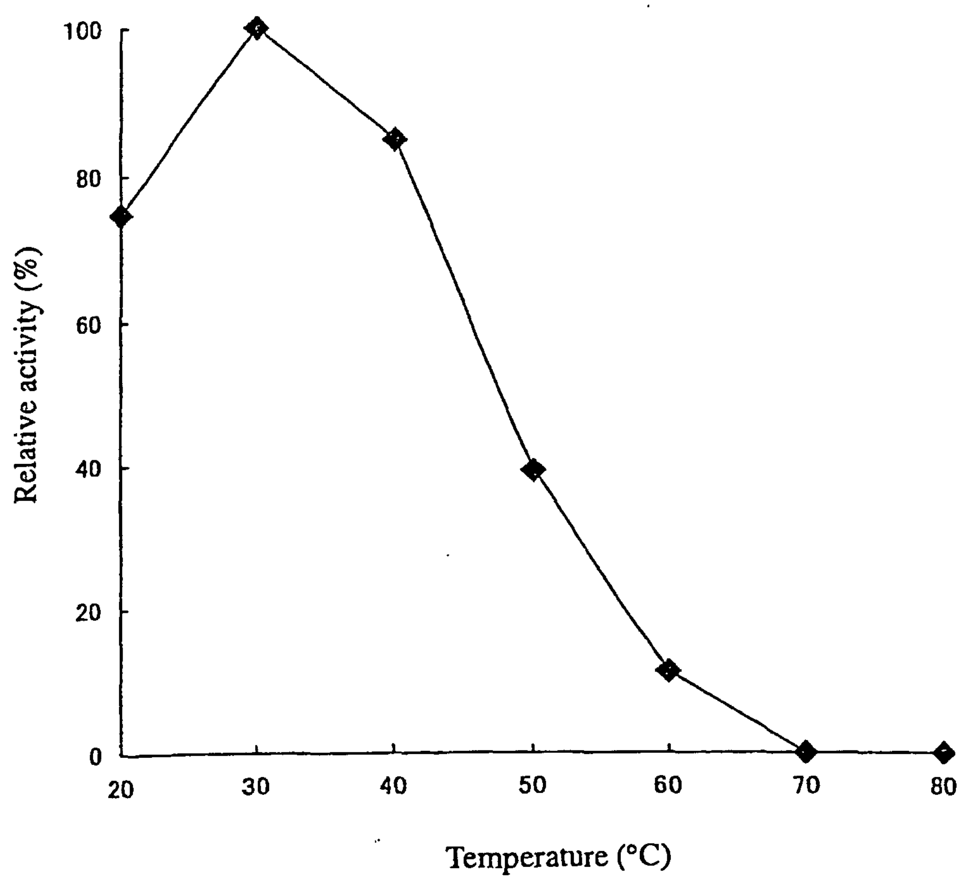
100
80
60
40
20
0
Relative activity (%)
20
30
40
50
60
70
80
Temperature (°C)

Fig. 5

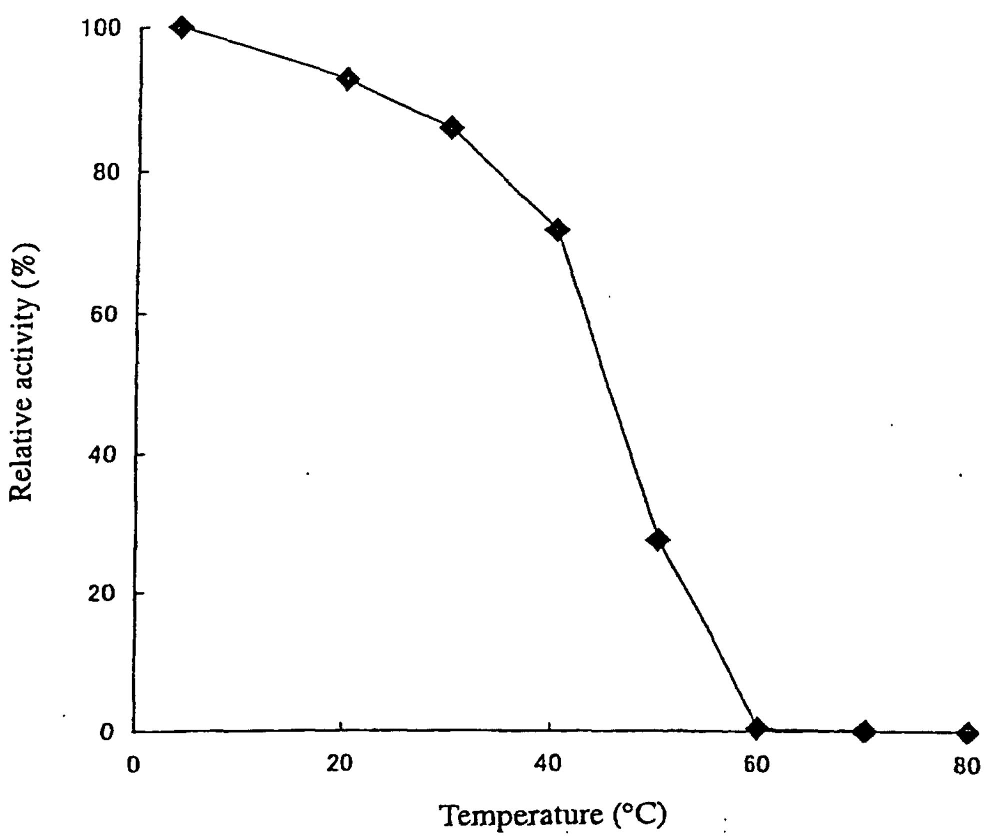

## INTERNATIONAL SEARCH REPORT

International application No.
PCT/JP03/02058

A. CLASSIFICATION OF SUBJECT MATTER
Int.Cl[7] C12N15/09, C12N9/42, C12P1/02, D21C3/00

According to International Patent Classification (IPC) or to both national classification and IPC

B. FIELDS SEARCHED

Minimum documentation searched (classification system followed by classification symbols)
Int.Cl[7] C12N15/09, C12N9/42, C12P1/02, D21C3/00

Documentation searched other than minimum documentation to the extent that such documents are included in the fields searched

Electronic data base consulted during the international search (name of data base and, where practicable, search terms used)
SwissProt/PIR/Geneseq, GenBank/EMBL/DDBJ/Geneseq, WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| X<br>A | EP 1029922 A2 (OJI Paper Co.), 23 August, 2000 (23.08.00), & US 6387688 B1 & JP 2000-342275 A | 12-14<br>1-11 |
| A | JP 11-127863 A (Mori Sangyo Kabushiki Kaisha), 18 May, 1999 (18.05.99), (Family: none) | 1-14 |
| A | Dumonceaux, T.J. et al., Cloning and sequencing of a gene encoding cellobiose dehydrogenase from Trametes versicolor., Gene, Vol.210, No.2, pages 211 to 219, (1998) | 1-14 |
| A | Moukha, S.M. et al., Cloning and analysis of Pycnoporus cinnabarinus cellobiose dehydrogenase., Gene, Vol.234, No.1, pages 23 to 33, (1999) | 1-14 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 30 April, 2003 (30.04.03) | 27 May, 2003 (27.05.03) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP03/02058

C (Continuation). DOCUMENTS CONSIDERED TO BE RELEVANT

| Category* | Citation of document, with indication, where appropriate, of the relevant passages | Relevant to claim No. |
|---|---|---|
| A | Sims, P. et al., The identification, molecular cloning and characterisation of a gene from Phanerochaete chrysosporium that shows strong homology to the exo-cellobiohydrolase I gene from Trichoderma reesei., Gene, Vol.74, No.2, pages 411 to 422, (1988) | 1-14 |
| A | Yague, E. et al., Correlation of exons with functional domains and folding regions in a cellulase from Agaricus bisporus., Curr.Genet., Vol.30, No.1, pages 56 to 61, (1996) | 1-14 |
| A | JP 2000-106887 A (YASOKAWA, D.), 18 April, 2000 (18.04.00), (Family: none) | 1-14 |

Form PCT/ISA/210 (continuation of second sheet) (July 1998)

SEQUENCE LISTING

<110> OJI PAPER CO., LTD.

<120> Cellulolytic enzyme and use thereof

<130> PH-1724-PCT

<160> 52

<170> PatentIn Ver. 3.1

<210> 1

<211> 3420

<212> DNA

<213> Coriolus hirsutus

<220>

<223> Inventor: Akira, Tsukamoto ; Seiji, Nakagame ; Mari, Kabuto ; Jun Sugiura ; Hisako Sakaguchi ; Atsushi Furujyo

<400> 1

catgtcctgt cggctccttg aatgctcggg tctttctcgc gataccgaag tgctgggcaa 60

cccggggacg cgtataaagt ccaaaaaatc ggtctttgac ggtgagcgcg acactacgac 120

tgcccgccat gaagttcaag agtctcctgt tgtccgtgtt gccgttggtc ggctctggta 180

tgttgcggcc ttccatccga caccgagacg agacgctgac agtaacacgc cacgaccagt 240

ctactcccag gtcgccgcac cctaccagga cgccggcaac ggcttcgtct ttgacggtgt 300

cactgatcca gtgcatagcg tcacgtatgg aatcgtcctc cctcaggcgg cctccagctc 360

ggagttcatt ggcgagatcg tcgcgccaaa cgacgcacaa tggatcggtt tggctcttgg 420

aggagccatg atcggcgacc tgcttctcgt cgcatggcca tatgagaaca aaattatttt 480
ctcccctcgc tacgcgacgt gagtatatgc tgttacatgt atgcagacgc tacgggctaa 540
atacgccaat ctcacagcgg gtacacgctg ccggcggtct acgaaggccc aaccattacc 600
acactcccgt ccagttcgat caactcgacg cactggaagt tcgtgttccg ctgccagaac 660
tgcacatgtg cgtacctcac attacgtatg acgtctccaa ctaaacctct tcacagcctg 720
ggatggcgga agcattgacc cctccggcac tggcgtcttc gcgtgggcgt actcgaacgt 780
cgcagtagat acccccgccg atcccaacag cagcttcgcc gagcacaccg actgtaagcg 840
atcatctctg aaccatggta ctgaatcact catggtatat cgcagtcggc ttcttcggcg 900
tcaacttccc cgatgctcag aactcgaact accaaagcta cctccagggc aacgccggca 960
ctccccctcc cacatccgtc cctagcggcc cttccagcac tacgactact actggtccta 1020
cggcaaccgt gagggcttcc acttcgctgt gcaggacgtt gctaacggtc tgtacaggct 1080
acgccgtttg actacatcgt cgttggtgcc ggcccaggtg gtctcatcgc tgccgatcgc 1140
ttgtcggagg cgggcaagaa ggtccttctt cttgagcgtg gtggaccttc gactgcagag 1200
accggcggca cttacgatgt cccatgggcc aagtccgcta acgtgagttg aatacccttg 1260
aatcgataat gcgcacaccg actgactccc atccatggta gctcacaaaa ttcgatgtcc 1320
cgggattgtt cgagacgctg ttcaccgaca cgaacccatt ctggtggtgc aagggtgggt 1380
cggtttctgg aagcgcatgt caacgtcgct aagaaagcct tctagacacc aacttctttg 1440
ctggatgcat tctcggtggc ggtaccacgg tcaacggagc gtaagtgcat tgactctgcc 1500
gtgcccaagc agccctcctg acgacaatct acagtcttta ctggtacccc aacaacaatg 1560
acttctccac cgccagcgga tggccgagca gctggaccaa ccaccagccg ttcaccaaca 1620
agctgaagca gcgtctgccg agcacagacc acccctccac cgacggccag cgctacctcg 1680
aacagtccgc gaacgtcgtc cagcagctgc tccagagcca gggctaccgg caggtcacga 1740
tcaacgacga cccggactcc aaggaccacg tcttcggcta cagcgcgttt gacttcctca 1800
acgggcagcg cgccggtccc gtcgcgacgt acttccagac cgcgctcgcg cgcaagaact 1860
tcgtgtaccg cgacaacgtg ctcgtcacgc aggtcatccg caacggctcg acgatcacgg 1920
gcgtgcgcac gaacgacctc accatcgggc ccgacggcat cgtgcccctc aacccgaacg 1980
gccgcgtcat ccttgctggc ggctcgttcg ggaccccgcg catcctgttc caaagcggca 2040
tcgggccgac ggacatgctc caggtcgtgc agggcaacgc gcaggccgcg gcgaacctgc 2100
ccccgcagaa ccagtggatc aacctcccgg tcggccaggc cgtgtctgac aacccgtcga 2160

tcaacgtgag tgacgctgca tacgcgttca agcccgccgg cctgaggctg acatggctcg 2220
tagttggtct tcactcaccc gagcatcgac gcgtacgaca actgggcgac cgtatggtcg 2280
aacccaaggc aggcggacgc tcagcagtac ctgcagagcc gctccggcgt gttggcgggc 2340
gcgtccccga agtacgttcg acatcgcgcc tggagtcttg caggtgtctg accagtcctc 2400
tctacaggct gaacttctgg agggcctacg gcggcagtga cggtatcacc cgctacgtat 2460
gtctatgttc gtcttgatct ccggtgctac gacctgacat tggccgtagg cgcaaggaac 2520
tgtccgtcct ggcgcagcat ccgtgaacac ctccgttgcg tacaacgcga gccagatctt 2580
cacgatcacc ctctacctgt gggtaccagc ccgaccgtat gtggactgtg cagctaaccg 2640
tgcaccacta caggtccaac ggtatccagt cgcgcggtcg catcggcgtg gacgccgccc 2700
tgaacgcgaa ggcgctcgtc aacccctggc tcaccaacgc cgtcgacaag acgatcctgc 2760
tgcaggccct gcacgacgtc gtctccacac tgaataacgg taaggccact tctccgtacc 2820
tgcctgcgcg cgcgccgctc atgcctctcc ttcctccagt ccaaggcctg acgatgatca 2880
cccccgacca caccatgacg atcgagcagt acgtcgacgc ctacgacccg gtgagtcccg 2940
cccgcagcat cccggcgaaa taaaaaacgg acgctgacgc ccccgtccca cgcaggcgac 3000
gatgtgctcc aaccactggg tgggcgccgc gaagatcggc acaagcccgt ccacggccgt 3060
cgtcgacgag aacgcgaagg tgttcaacac ggacaacctg gtacgtttcc ctgccctttt 3120
tcttcccgtg ccctccgctg acgcggcctt cctgcagttc atcgtcgatg cgtccatcat 3180
cccgtctctg ccggtcggga acccgcaggg cctgctcatg tctgcggccg agcaggccgt 3240
gtcgaagatc ctcgcgctcg ccggaggacc gtgaggcagg gggttcaaaa gcatttggag 3300
cgctgctatg gtagaccatg aagcgggatg ggtcctgtcg atatgagaca cgatgtatat 3360
attatatatt ctgcacggtt ttcttcttcc tggaagcctg atgaggctct cgacgtgcca 3420

<210> 2

<211> 768

<212> PRT

<213> Coriolus hirsutus

<400> 2

Met Lys Phe Lys Ser Leu Leu Leu Ser Val Leu Pro Leu Val Gly Ser
1 5 10 15

Val Tyr Ser Gln Val Ala Ala Pro Tyr Gln Asp Ala Gly Asn Gly Phe
20 25 30

Val Phe Asp Gly Val Thr Asp Pro Val His Ser Val Thr Tyr Gly Ile
35 40 45

Val Leu Pro Gln Ala Ala Ser Ser Ser Glu Phe Ile Gly Glu Ile Val
50 55 60

Ala Pro Asn Asp Ala Gln Trp Ile Gly Leu Ala Leu Gly Gly Ala Met
65 70 75 80

Ile Gly Asp Leu Leu Leu Val Ala Trp Pro Tyr Glu Asn Lys Ile Ile
85 90 95

Phe Ser Pro Arg Tyr Ala Thr Gly Tyr Thr Leu Pro Ala Val Tyr Glu
100 105 110

Gly Pro Thr Ile Thr Thr Leu Pro Ser Ser Ser Ile Asn Ser Thr His
115 120 125

Trp Lys Phe Val Phe Arg Cys Gln Asn Cys Thr Ser Trp Asp Gly Gly
130 135 140

Ser Ile Asp Pro Ser Gly Thr Gly Val Phe Ala Trp Ala Tyr Ser Asn
145 150 155 160

Val Ala Val Asp Thr Pro Ala Asp Pro Asn Ser Ser Phe Ala Glu His
165 170 175

Thr Asp Phe Gly Phe Phe Gly Val Asn Phe Pro Asp Ala Gln Asn Ser
180 185 190

Asn Tyr Gln Ser Tyr Leu Gln Gly Asn Ala Gly Thr Pro Pro Pro Thr
195 200 205

Ser Val Pro Ser Gly Pro Ser Ser Thr Thr Thr Thr Thr Gly Pro Thr
210 215 220

Ala Thr Ala Thr Pro Phe Asp Tyr Ile Val Val Gly Ala Gly Pro Gly
225 230 235 240

Gly Leu Ile Ala Ala Asp Arg Leu Ser Glu Ala Gly Lys Lys Val Leu
245 250 255

Leu Leu Glu Arg Gly Gly Pro Ser Thr Ala Glu Thr Gly Gly Thr Tyr
260 265 270

Asp Val Pro Trp Ala Lys Ser Ala Asn Leu Thr Lys Phe Asp Val Pro
275 280 285

Gly Leu Phe Glu Thr Leu Phe Thr Asp Thr Asn Pro Phe Trp Trp Cys
290 295 300

Lys Asp Thr Asn Phe Phe Ala Gly Cys Ile Leu Gly Gly Gly Thr Thr

305 310 315 320

Val Asn Gly Ala Leu Tyr Trp Tyr Pro Asn Asn Asn Asp Phe Ser Thr

325 330 335

Ala Ser Gly Trp Pro Ser Ser Trp Thr Asn His Gln Pro Phe Thr Asn

340 345 350

Lys Leu Lys Gln Arg Leu Pro Ser Thr Asp His Pro Ser Thr Asp Gly

355 360 365

Gln Arg Tyr Leu Glu Gln Ser Ala Asn Val Val Gln Gln Leu Leu Gln

370 375 380

Ser Gln Gly Tyr Arg Gln Val Thr Ile Asn Asp Asp Pro Asp Ser Lys

385 390 395 400

Asp His Val Phe Gly Tyr Ser Ala Phe Asp Phe Leu Asn Gly Gln Arg

405 410 415

Ala Gly Pro Val Ala Thr Tyr Phe Gln Thr Ala Leu Ala Arg Lys Asn

420 425 430

Phe Val Tyr Arg Asp Asn Val Leu Val Thr Gln Val Ile Arg Asn Gly

435 440 445

Ser Thr Ile Thr Gly Val Arg Thr Asn Asp Leu Thr Ile Gly Pro Asp

450 455 460

Gly Ile Val Pro Leu Asn Pro Asn Gly Arg Val Ile Leu Ala Gly Gly
465 470 475 480

Ser Phe Gly Thr Pro Arg Ile Leu Phe Gln Ser Gly Ile Gly Pro Thr
485 490 495

Asp Met Leu Gln Val Val Gln Gly Asn Ala Gln Ala Ala Ala Asn Leu
500 505 510

Pro Pro Gln Asn Gln Trp Ile Asn Leu Pro Val Gly Gln Ala Val Ser
515 520 525

Asp Asn Pro Ser Ile Asn Leu Val Phe Thr His Pro Ser Ile Asp Ala
530 535 540

Tyr Asp Asn Trp Ala Thr Val Trp Ser Asn Pro Arg Gln Ala Asp Ala
545 550 555 560

Gln Gln Tyr Leu Gln Ser Arg Ser Gly Val Leu Ala Gly Ala Ser Pro
565 570 575

Lys Leu Asn Phe Trp Arg Ala Tyr Gly Gly Ser Asp Gly Ile Thr Arg
580 585 590

Tyr Ala Gln Gly Thr Val Arg Pro Gly Ala Ala Ser Val Asn Thr Ser
595 600 605

Val Ala Tyr Asn Ala Ser Gln Ile Phe Thr Ile Thr Leu Tyr Leu Ser
610 615 620

Asn Gly Ile Gln Ser Arg Gly Arg Ile Gly Val Asp Ala Ala Leu Asn
625 630 635 640

Ala Lys Ala Leu Val Asn Pro Trp Leu Thr Asn Ala Val Asp Lys Thr
645 650 655

Ile Leu Leu Gln Ala Leu His Asp Val Val Ser Thr Leu Asn Asn Val
660 665 670

Gln Gly Leu Thr Met Ile Thr Pro Asp His Thr Met Thr Ile Glu Gln
675 680 685

Tyr Val Asp Ala Tyr Asp Pro Ala Thr Met Cys Ser Asn His Trp Val
690 695 700

Gly Ala Ala Lys Ile Gly Thr Ser Pro Ser Thr Ala Val Val Asp Glu
705 710 715 720

Asn Ala Lys Val Phe Asn Thr Asp Asn Leu Phe Ile Val Asp Ala Ser
725 730 735

Ile Ile Pro Ser Leu Pro Val Gly Asn Pro Gln Gly Leu Leu Met Ser
740 745 750

Ala Ala Glu Gln Ala Val Ser Lys Ile Leu Ala Leu Ala Gly Gly Pro
755 760 765

<210> 3

<211> 3480

<212> DNA

<213> Coriolus hirsutus

<400> 3

agcgcacgcg gcgcgtacca aatgagcgtt catgtcctgt cggctccttg aatgctcggg 60

tctttctcgc ggtaccgaag tgctgggcaa cccggggacg cgtataaagt ccaaaaaatg 120

ggtcttgaac ggtgagcacg acactacgac cgcccgccat gaagctcaag agcctcctgt 180

tgtccgtgtt gccgttggtc ggctctggta tgttgcagcc ttctatctga catcgagacg 240

agacgctgac agtaacgcac cacgaacagt ctacccccag gtcgccgcac cctaccagga 300

cgccggcaac ggcttcgtct ttgacggtgt cactgaccca gtgcatagcg tcacctatgg 360

aatcgtcctc cctcaggcgg cctccagctc ggagttcatt ggcgagatcg tcgcgccaaa 420

cgacgcacaa tggatcggtt tggctcttgg aggagccatg atcggcgacc tgcttctcgt 480

cgcatggcca tatgagaaca aaatcatttt ctcccctcgc tacgcgacgt gagtatatgc 540

tgttacatgc atgcagacgc tcggggctaa atacgccaat attacagcgg gtacaccctg 600

ccggcggtct acgacggccc aaccattacc acactcccgt ccagttcggt caactcgacg 660

cactggaagt tcgtgtttcg ctgccagaac tgcacatgtg cgtacctcac atttcgtacg 720

acgtctccaa ctaaacctct tcacagcctg ggatggcgga agcattgacc cctccggcac 780

tggcgtcttc gcgtgggcgt actcgaacgt cgcagttgat acccccgccg atcccaacag 840

cagcttcgcc gagcacaccg actgtaagca atcatctctt aatcccggtg ccgaatcact 900

catggtatat cgcagtcggc ttcttcggcg tcaacttccc cgatgctcag aactcgaact 960

accaaaacta cctccagggc aacgccggca ccccccctcc cacgtccgtc cctagcggcc 1020

cttccagcac tacgactact actggtccta cggcaactgt gagcgcttcc acttcactgt 1080

gcagaacgtc gctaactttc tgtataggct acgccgtttg actacatcgt cgttggtgcc 1140

ggcccaggtg gtctcatcgc tgccgatcgc ctgtcggagg cgggcaagaa ggttcttctt 1200

cttgagcgtg gtggaccttc gacagcagag accggcggca cttacgatgc cccatgggcc 1260

aagtccgcta acgtgagttg aataccettg aatcgataat gcgcacaccg actgactccc 1320

atccatggta gctcacaaaa ttcgatgtcc cgggattgtt cgagacgctg ttcaccgaca 1380

cgaacccatt ctggtggtgc aagggtgggt cggtttctgg aagcgcatgt caacgtcgct 1440
gagaaagcgt tctagatacc aacttctttg ctggatgcat tctcggtggc ggtaccacgg 1500
tcaacggagc gtaagtgcat cgactctgcc gtgtccaagc agtcctccta acgacaatct 1560
acagtcttta ctggtacccc aacaacaatg acttctccac ggccagcgga tggccgagca 1620
gctgggccaa ccaccagccg ttcaccagca agctgaagca gcgtctgccg agcacagacc 1680
acccctccac cgacggccag cgctacctcg aacagtccgc gaacgtcgtc cagcagctgc 1740
ttcaaagcca gggctaccgg caggtcacga tcaacgacga cccggactcc aaggaccacg 1800
tcttcggcta cagcgcgttc gacttcctca acgggcagcg cgccggcccc gttgcgacgt 1860
acttccagac cgcgctcgcg cgcaagaact tcgtgtaccg cgacaacgtg ctcgtcacgc 1920
aggtcatccg caacggctcg acgatcaccg gcgtgcgcac gaacgacctc accatcgggc 1980
ccgacggcat cgtgcccctc aacccgaacg gccgcgtcat cctcgctggc ggctcgttcg 2040
ggaccccgcg catcctgttc caaagcggca tcgggccgac ggacatgctc caggtcgtgc 2100
agggcaacgc gcaggctgcg gcgaacctgc ccccgcagag ccagtggatc gacctcccgg 2160
tcggccaggc cgtgtctgac aacccgtcga tcaacgtgag tgacgctgta tacgtgctct 2220
agcccgccgg cctgaggctg acatggctcg tagttggtct tcacgcaccc gagcatcgac 2280
gcgtacgaca actgggccac cgtgtggtcg aaccccaggc aggcggacgc tcagcagtat 2340
ctgcagagcc gctccggcgt gttggcgggc gcgtccccaa agtacgttcg acatcgtgtc 2400
cggagtcttg caggtgtctg accagtcctc tctacaggct gaacttctgg agggcctacg 2460
gcggcagtga cggcatcacc cgctacgtat gtctatgtcc gtcttcatca atggaaccgc 2520
gatctgacat tatccgtagg cgcaaggaac cgtccgtcct ggcgcagcat ccgtgaacac 2580
ctccgttgcg tacaacgcga gccagatctt cacgatcacc ctctacctgt gggtaccaac 2640
ccggtcgtat gtataccgtg cagctgaccg tgcgccacca caggtccaac ggtatccagt 2700
cgcgcggtcg cattggtgtg gacgccgccc tgaacgcgaa ggcgctcgtc aacccctggc 2760
tcaccaacgc cgtcgacaag acgatcctgc tgcaggccct gcacgacgtc gtctccacac 2820
tgaacaacgg taaggccgcc cctacatgcc cgcctgcgcg cgccgctcat gccgctcctt 2880
cctccagtcc aaggcctgac gatgatcacc cccgaccaca ccatgacgat cgagcagtac 2940
gtcgacgcct acgacccggt gagtcccgtc cgcagcatcc ccgcaaaaga aaaaaaacga 3000
acgctgacgc ccccgtccca cgcaggcgac gatgtgctcc aaccactggg tgggcgccgc 3060
gaagatcggc acgagcccgt ccacggccgt cgtcgacgag aacgcgaagg tgttcaacac 3120

ggacaacctg gtgcgttccc cttcgttatg taactcacca cctcccctgg ccaccgccgc 3180

tgacaggatc gacgtttctg catcgcagtt catcgtcgac gcgtccatca tcccgtctct 3240

gccggtcggg aacccgcagg ggttgctcat gtccgcggcc gagcaggccg tgtcgaagat 3300

cctcgcgctc gccggaggac cgtgagggag ggggttcaaa agcctttgga gcgctgctat 3360

ggtggaccct gaagcgggat gggttctgtc gatatgagac acgatgtaat attatattct 3420

gcatgaattt cttcttcctg cagcctaatg cggactgtct ctcgatgtgc taacgagagc 3480

<210> 4

<211> 768

<212> PRT

<213> Coriolus hirsutus

<400> 4

Met Lys Leu Lys Ser Leu Leu Leu Ser Val Leu Pro Leu Val Gly Ser
1 5 10 15

Val Tyr Pro Gln Val Ala Ala Pro Tyr Gln Asp Ala Gly Asn Gly Phe
20 25 30

Val Phe Asp Gly Val Thr Asp Pro Val His Ser Val Thr Tyr Gly Ile
35 40 45

Val Leu Pro Gln Ala Ala Ser Ser Ser Glu Phe Ile Gly Glu Ile Val
50 55 60

Ala Pro Asn Asp Ala Gln Trp Ile Gly Leu Ala Leu Gly Gly Ala Met
65 70 75 80

Ile Gly Asp Leu Leu Leu Val Ala Trp Pro Tyr Glu Asn Lys Ile Ile
85 90 95

Phe Ser Pro Arg Tyr Ala Thr Gly Tyr Thr Leu Pro Ala Val Tyr Asp
100 105 110

Gly Pro Thr Ile Thr Thr Leu Pro Ser Ser Ser Val Asn Ser Thr His
115 120 125

Trp Lys Phe Val Phe Arg Cys Gln Asn Cys Thr Ser Trp Asp Gly Gly
130 135 140

Ser Ile Asp Pro Ser Gly Thr Gly Val Phe Ala Trp Ala Tyr Ser Asn
145 150 155 160

Val Ala Val Asp Thr Pro Ala Asp Pro Asn Ser Ser Phe Ala Glu His
165 170 175

Thr Asp Phe Gly Phe Phe Gly Val Asn Phe Pro Asp Ala Gln Asn Ser
180 185 190

Asn Tyr Gln Asn Tyr Leu Gln Gly Asn Ala Gly Thr Pro Pro Pro Thr
195 200 205

Ser Val Pro Ser Gly Pro Ser Ser Thr Thr Thr Thr Thr Gly Pro Thr
210 215 220

Ala Thr Ala Thr Pro Phe Asp Tyr Ile Val Val Gly Ala Gly Pro Gly
225 230 235 240

Gly Leu Ile Ala Ala Asp Arg Leu Ser Glu Ala Gly Lys Lys Val Leu
245 250 255

Leu Leu Glu Arg Gly Gly Pro Ser Thr Ala Glu Thr Gly Gly Thr Tyr
260 265 270

Asp Ala Pro Trp Ala Lys Ser Ala Asn Leu Thr Lys Phe Asp Val Pro
275 280 285

Gly Leu Phe Glu Thr Leu Phe Thr Asp Thr Asn Pro Phe Trp Trp Cys
290 295 300

Lys Asp Thr Asn Phe Phe Ala Gly Cys Ile Leu Gly Gly Gly Thr Thr
305 310 315 320

Val Asn Gly Ala Leu Tyr Trp Tyr Pro Asn Asn Asn Asp Phe Ser Thr
325 330 335

Ala Ser Gly Trp Pro Ser Ser Trp Ala Asn His Gln Pro Phe Thr Ser
340 345 350

Lys Leu Lys Gln Arg Leu Pro Ser Thr Asp His Pro Ser Thr Asp Gly
355 360 365

Gln Arg Tyr Leu Glu Gln Ser Ala Asn Val Val Gln Gln Leu Leu Gln
370 375 380

Ser Gln Gly Tyr Arg Gln Val Thr Ile Asn Asp Asp Pro Asp Ser Lys

385 390 395 400

Asp His Val Phe Gly Tyr Ser Ala Phe Asp Phe Leu Asn Gly Gln Arg

405 410 415

Ala Gly Pro Val Ala Thr Tyr Phe Gln Thr Ala Leu Ala Arg Lys Asn

420 425 430

Phe Val Tyr Arg Asp Asn Val Leu Val Thr Gln Val Ile Arg Asn Gly

435 440 445

Ser Thr Ile Thr Gly Val Arg Thr Asn Asp Leu Thr Ile Gly Pro Asp

450 455 460

Gly Ile Val Pro Leu Asn Pro Asn Gly Arg Val Ile Leu Ala Gly Gly

465 470 475 480

Ser Phe Gly Thr Pro Arg Ile Leu Phe Gln Ser Gly Ile Gly Pro Thr

485 490 495

Asp Met Leu Gln Val Val Gln Gly Asn Ala Gln Ala Ala Ala Asn Leu

500 505 510

Pro Pro Gln Ser Gln Trp Ile Asp Leu Pro Val Gly Gln Ala Val Ser

515 520 525

Asp Asn Pro Ser Ile Asn Leu Val Phe Thr His Pro Ser Ile Asp Ala

530 535 540

Tyr Asp Asn Trp Ala Thr Val Trp Ser Asn Pro Arg Gln Ala Asp Ala
545 550 555 560

Gln Gln Tyr Leu Gln Ser Arg Ser Gly Val Leu Ala Gly Ala Ser Pro
565 570 575

Lys Leu Asn Phe Trp Arg Ala Tyr Gly Gly Ser Asp Gly Ile Thr Arg
580 585 590

Tyr Ala Gln Gly Thr Val Arg Pro Gly Ala Ala Ser Val Asn Thr Ser
595 600 605

Val Ala Tyr Asn Ala Ser Gln Ile Phe Thr Ile Thr Leu Tyr Leu Ser
610 615 620

Asn Gly Ile Gln Ser Arg Gly Arg Ile Gly Val Asp Ala Ala Leu Asn
625 630 635 640

Ala Lys Ala Leu Val Asn Pro Trp Leu Thr Asn Ala Val Asp Lys Thr
645 650 655

Ile Leu Leu Gln Ala Leu His Asp Val Val Ser Thr Leu Asn Asn Val
660 665 670

Gln Gly Leu Thr Met Ile Thr Pro Asp His Thr Met Thr Ile Glu Gln
675 680 685

Tyr Val Asp Ala Tyr Asp Pro Ala Thr Met Cys Ser Asn His Trp Val
690 695 700

Gly Ala Ala Lys Ile Gly Thr Ser Pro Ser Thr Ala Val Val Asp Glu
705 710 715 720

Asn Ala Lys Val Phe Asn Thr Asp Asn Leu Phe Ile Val Asp Ala Ser
725 730 735

Ile Ile Pro Ser Leu Pro Val Gly Asn Pro Gln Gly Leu Leu Met Ser
740 745 750

Ala Ala Glu Gln Ala Val Ser Lys Ile Leu Ala Leu Ala Gly Gly Pro
755 760 765

<210> 5
<211> 28
<212> DNA
<213> Artificial Sequence

<220>
<223> Synthetic DNA

<400> 5
taygaraaya avatthttn 19

<210> 6
<211> 19
<212> DNA

<213> Artificial Sequence

<220>

<223> Synthetic DNA

<400> 6

gayatcaagt tyatcratgg 20

<210> 7

<211> 1496

<212> DNA

<213> Coriolus hirsutus

<400> 7

atgttcccca cagtctccct cctcgcgttc tccctccttg cgaccgtcta cggtcagcaa 60
gtcggcaccc tgacggcgga gaaccacccc cgcctcaccg tccagcagtg cacggccaag 120
aacaactgcc agacccagca gcactccgtc gtgctcgact ccaactggcg ctggctccac 180
gccaccaccg gcagcaacaa ctgctacacc ggcaacacct gggatgcgtt gctctgcccc 240
gatgcgacca cctgcgccaa gaactgcgcg gtcgacggcg ccgactatgc cggtatgtcc 300
cgttcttgcg gctgcgctgc ctgcgacgcg ccagttgtct aacacacgcg ctcgctaggc 360
acctacggca tcaccacgaa cggcaacgcg ctcacgctca agttcgtgca gcagggcccg 420
tactcgaaga acatcggctc gcgcgtgtac ctcatggacg cgcaggacca gaagtacgag 480
ctcttcaacc tgaagaacca ggagttcacg ttcgacgtcg acatgtcgaa cctcccctgc 540
ggcctcaacg gcgcgctcta cttcgtcgag atggacgccg acggcggcgc gtcccgcttc 600
ccgaccaaca aggccggcgc gaagtacgga accggctact gcgacaccca gtgcccgcag 660
gacatcaagt tcatcaacgg cgtggtaagc accgacctcc ccgctgcccg actccccgct 720
gcgtgctcac atccgccccg caggccaacc tcgaggggtg ggcgggctcg ccctctgacc 780
cgaactcggg caccggcagc ttcggcacgt gctgcaacga gatggacgtg tgggaggcga 840

acaagaacgg cgccgcgttc acgccgcacg tctgctccgt cacgagccag acgcgctgcg 900
agggcacgca gtgcggcgac ggcgacgagc gctacgacgg cctctgcgac aaggacggct 960
gcgacttcaa ctcgttccgc aagggcgacc agaccttcct cgggccgggc aagaccgtcg 1020
acacgaacgc gaagttcacg gtcgtcacgc agttcctgac gaacaacaac cagacgtccg 1080
gccagctgtc cgagatccgc cgcctgtacg tgcagaacgg gcgggtgatc gcgaactcga 1140
agacgaacgt gcccgggctc ggcgccttcg actcgatcac ggaccagttc tgcaacgcgc 1200
agaagcaggt gttcggcgac gacaacacgt tcgagaagct cggtgggctc aacacgatgg 1260
gccaggcctt ccagcgcggc atggcgctcg tcatgtccat ctgggacgac cacgccgcgg 1320
gcatgctctg gctcgacgcc gactacccca ccgacgcgcc cgcgaccaac cccggtgtct 1380
cccgtggccc gtgctcggcc acctctggcg accccgcgac gatcgagaac agcgaggcga 1440
gctcgtccgt caccttctcg aacatcaagg tcggccccat cggctcgacg ttctga 1496

<210> 8
<211> 456
<212> PRT
<213> Coriolus hirsutus

<400> 8

Met Phe Pro Thr Val Ser Leu Leu Ala Phe Ser Leu Leu Ala Thr Val
1 5 10 15

Tyr Gly Gln Gln Val Gly Thr Leu Thr Ala Glu Asn His Pro Arg Leu
20 25 30

Thr Val Gln Gln Cys Thr Ala Lys Asn Asn Cys Gln Thr Gln Gln His
35 40 45

Ser Val Val Leu Asp Ser Asn Trp Arg Trp Leu His Ala Thr Thr Gly

50 55 60

Ser Asn Asn Cys Tyr Thr Gly Asn Thr Trp Asp Ala Leu Leu Cys Pro
65 70 75 80

Asp Ala Thr Thr Cys Ala Lys Asn Cys Ala Val Asp Gly Ala Asp Tyr
85 90 95

Ala Gly Thr Tyr Gly Ile Thr Thr Asn Gly Asn Ala Leu Thr Leu Lys
100 105 110

Phe Val Gln Gln Gly Pro Tyr Ser Lys Asn Ile Gly Ser Arg Val Tyr
115 120 125

Leu Met Asp Ala Gln Asp Gln Lys Tyr Glu Leu Phe Asn Leu Lys Asn
130 135 140

Gln Glu Phe Thr Phe Asp Val Asp Met Ser Asn Leu Pro Cys Gly Leu
145 150 155 160

Asn Gly Ala Leu Tyr Phe Val Glu Met Asp Ala Asp Gly Gly Ala Ser
165 170 175

Arg Phe Pro Thr Asn Lys Ala Gly Ala Lys Tyr Gly Thr Gly Tyr Cys
180 185 190

Asp Thr Gln Cys Pro Gln Asp Ile Lys Phe Ile Asn Gly Val Ala Asn
195 200 205

Leu Glu Gly Trp Ala Gly Ser Pro Ser Asp Pro Asn Ser Gly Thr Gly
210 215 220

Ser Phe Gly Thr Cys Cys Asn Glu Met Asp Val Trp Glu Ala Asn Lys
225 230 235 240

Asn Gly Ala Ala Phe Thr Pro His Val Cys Ser Val Thr Ser Gln Thr
245 250 255

Arg Cys Glu Gly Thr Gln Cys Gly Asp Gly Asp Glu Arg Tyr Asp Gly
260 265 270

Leu Cys Asp Lys Asp Gly Cys Asp Phe Asn Ser Phe Arg Xaa Gly Asp
275 280 285

Gln Thr Phe Leu Gly Pro Gly Lys Thr Val Asp Thr Asn Ala Lys Phe
290 295 300

Thr Val Val Thr Gln Phe Leu Thr Asn Asn Asn Gln Thr Ser Gly Gln
305 310 315 320

Leu Ser Glu Ile Arg Arg Leu Tyr Val Gln Asn Gly Arg Val Ile Ala
325 330 335

Asn Ser Lys Thr Asn Val Pro Gly Leu Gly Ala Phe Asp Ser Ile Thr
340 345 350

Asp Gln Phe Cys Asn Ala Gln Lys Gln Val Phe Gly Asp Asp Asn Thr
355 360 365

Phe Glu Lys Leu Gly Gly Leu Asn Thr Met Gly Gln Ala Phe Gln Arg
370 375 380

Gly Met Ala Leu Val Met Ser Ile Trp Asp Asp His Ala Ala Gly Met
385 390 395 400

Leu Trp Leu Asp Ala Asp Tyr Pro Pro Thr Arg Pro Arg Pro Thr Pro
405 410 415

Val Val Ser Arg Gly Pro Cys Ser Ala Thr Ser Gly Asp Pro Ala Thr
420 425 430

Ile Glu Asn Ser Glu Ala Ser Ser Ser Val Thr Phe Ser Asn Ile Lys
435 440 445

Val Gly Pro Ile Gly Ser Thr Phe
450 455

<210> 9
<211> 1488
<212> DNA
<213> Coriolus hirsutus

<400> 9
atgttccccg cagtcgccct cctcgctctc tccttcttcg ccatcgccta cggccagcag 60
gtcggcacac tcacggcgga gaaccacccg aagatcacgg tccagcagtg cacgggcaag 120
aacagctgcc agaccctgca gcgctcggtc gtgctcgact ccaactggcg ctggctccac 180

```
tcgaccagcg gcagcaacaa ctgctacacc ggcaacacct gggacagctc cctctgcccc 240
gaccccacca cctgcgcgaa gaactgcgcg cttgacggcg ccgactatgc tggtgagctt 300
actcgtcctt tgggtctgac gggcgagcgt gctaaccata tttgatgcgt taggcaccta 360
cggcatcacc acgagcggta accagctcag tctcaagttc gtaacgcacg gccagtactc 420
gaccaacatc ggctcgcgcg tgtacctcct cgacggtagc gactccaagt accagcagtt 480
caacctgaag aaccaggagt tcacgttcga catcgacatg tcgaagctcc cgtgcggcct 540
caacggcgcg ctctacttcg tcgagatgga cgccgacggt ggtctctccc gcttcccctc 600
caacaaggct ggcgcgaagt acggcaccgg ctactgcgat acccagtgcc cgcacgacat 660
caagttcatc aacggcgagg tgagttttca atgagtcaat cttcgtgtgt gccggtgctc 720
acacgtccct tacaggccaa cgtcctcggc tggacgccct cagacagcga cccgaacgcg 780
ggcagcggcc agtacggcac gtgctgcaac gagatggaca tctgggaggc gaactcgatg 840
ggcgcggcgg taactccgca cgtctgctcc gtcacgagcc agacgcgctg ctcgggcacg 900
gactgcggcg acggcgacaa ccgctacaac ggcatctgcg acaaggacgg ctgcgacttc 960
aactcgtggc gcatgggcga ccagacgttc ctcgggccgg gcaagaccgt caacacgaac 1020
cagaagttca cggtcgtgac gcagttcctg acgaacaaca accagacgtc gggcacgctc 1080
tccgagatcc gccgcctgta cgtgcagaac gggaaggtga tcgcgaactc gaagacgaag 1140
atccccggca tggacgcgta cgactcgatc accgacgcgt tctgcaacgc gcagaagcag 1200
gcgttcggcg acaacaactc gttcgagagg ctcggcgggc tcaaggcgat gggcgccgcc 1260
ttcgacaagg gcatgagcct cgtcatgtcc atctgggacg accacgaggc gaagatgctc 1320
tggctcgaca gcgagtaccc cctcgacaag gacgcttcca cgcccggtgt ctctcgcggc 1380
ccctgcgcgc ggacctccgg cgagccgaag gacgtcgagt ccaacagccc cgacgcgacc 1440
gtcgtcttct ccaacatcaa gtacggcccc atcggctcga cctactaa             1488
```

<210> 10

<211> 456

<212> PRT

<213> Coriolus hirsutus

<400> 10

Met Phe Pro Ala Val Ala Leu Leu Ala Leu Ser Phe Phe Ala Ile Ala
1               5                   10                  15

Tyr Gly Gln Gln Val Gly Thr Leu Thr Ala Glu Asn His Pro Lys Ile
            20                  25                  30

Thr Val Gln Gln Cys Thr Gly Lys Asn Ser Cys Gln Thr Leu Gln Arg
        35                  40                  45

Ser Val Val Leu Asp Ser Asn Trp Arg Trp Leu His Ser Thr Ser Gly
    50                  55                  60

Ser Asn Asn Cys Tyr Thr Gly Asn Thr Trp Asp Ser Ser Leu Cys Pro
65                  70                  75                  80

Asp Pro Thr Thr Cys Ala Lys Asn Cys Ala Leu Asp Gly Ala Asp Tyr
                85                  90                  95

Ala Gly Thr Tyr Gly Ile Thr Thr Ser Gly Asn Gln Leu Ser Leu Lys
            100                 105                 110

Phe Val Thr His Gly Gln Tyr Ser Thr Asn Ile Gly Ser Arg Val Tyr
        115                 120                 125

Leu Leu Asp Gly Ser Asp Ser Lys Tyr Gln Gln Phe Asn Leu Lys Asn
    130                 135                 140

Gln Glu Phe Thr Phe Asp Ile Asp Met Ser Lys Leu Pro Cys Gly Leu

145 150 155 160

Asn Gly Ala Leu Tyr Phe Val Glu Met Asp Ala Asp Gly Gly Leu Ser
165 170 175

Arg Phe Pro Ser Asn Lys Ala Gly Ala Lys Tyr Gly Thr Gly Tyr Cys
180 185 190

Asp Thr Gln Cys Pro His Asp Ile Lys Phe Ile Asn Gly Glu Ala Asn
195 200 205

Val Leu Gly Trp Thr Pro Ser Asp Ser Asp Pro Asn Ala Gly Ser Gly
210 215 220

Gln Tyr Gly Thr Cys Cys Asn Glu Met Asp Ile Trp Glu Ala Asn Ser
225 230 235 240

Met Gly Ala Ala Val Thr Pro His Val Cys Ser Val Thr Ser Gln Thr
245 250 255

Arg Cys Ser Gly Thr Asp Cys Gly Asp Gly Asp Asn Arg Tyr Asn Gly
260 265 270

Ile Cys Asp Lys Asp Gly Cys Asp Phe Asn Ser Trp Arg Met Gly Asp
275 280 285

Gln Thr Phe Leu Gly Pro Gly Lys Thr Val Asn Thr Asn Gln Lys Phe
290 295 300

Thr Val Val Thr Gln Phe Leu Thr Asn Asn Asn Gln Thr Ser Gly Thr
305 310 315 320

Leu Ser Glu Ile Arg Arg Leu Tyr Val Gln Asn Gly Lys Val Ile Ala
325 330 335

Asn Ser Lys Thr Lys Ile Pro Gly Met Asp Ala Tyr Asp Ser Ile Thr
340 345 350

Asp Ala Phe Cys Asn Ala Gln Lys Gln Ala Phe Gly Asp Asn Asn Ser
355 360 365

Phe Glu Arg Leu Gly Gly Leu Lys Ala Met Gly Ala Ala Phe Asp Lys
370 375 380

Gly Met Ser Leu Val Met Ser Ile Trp Asp Asp His Glu Ala Lys Met
385 390 395 400

Leu Trp Leu Asp Ser Glu Tyr Pro Leu Asp Lys Asp Ala Ser Thr Pro
405 410 415

Gly Val Ser Arg Gly Pro Cys Ala Arg Thr Ser Gly Glu Pro Lys Asp
420 425 430

Val Glu Ser Asn Ser Pro Asp Ala Thr Val Val Phe Ser Asn Ile Lys
435 440 445

Tyr Gly Pro Ile Gly Ser Thr Tyr
450 455

<210> 11

<211> 1485

<212> DNA

<213> Coriolus hirsutus

<400> 11

atgttcccca ccgccgccct cctctcgctc tccttcgcgg cgatcgccta cggccagcag 60

gtcggcacgc tcaccgccga gtcgcacccc aagctcagcg tgcagcaatg caccgccggc 120

ggcagctgcc agaccctgca gcgctccgtc gtcctcgact ccaactggcg ttggctccac 180

tctacctcgg gctcgaccaa ctgctacacc ggcaacacct gggacgcgtc cctctgcccc 240

gaccccacca cgtgcgcggc aaactgtgcc ctcgatggcg ctgactactc tggtccgcgt 300

tgatctcacc tgaagaccct tgtgcatact caacaatccc tctaggcacc tacggtatca 360

ccaccagcgg caacgagctc aacctcaggt tcgtcacgaa gggccagtac tccaccaaca 420

tcggctcccg cgtctacctt ctctccgagg acgacagcac gtacgagatg ttcaacctca 480

acaaccagga gttcacgttc gacgtcgaca tgtcgaacct cccgtgcggc ctcaacggcg 540

cgctctactt cgtggagatg gacaaggacg gtggctcatc ccgcttcccc accaacaagg 600

ccggctccaa gtacggtacc ggctactgcg acacccagtg cccgcacgat atcaagttca 660

tcaacggcga ggtaagatgc cgcgtctcaa cgcagtaaaa ccatgctgac tcgctcttcc 720

gcacgccagg ccaacgtcct cggctgggag ggctccccga acgacccgaa cgcgggaacc 780

ggccagtacg gaacgtgctg caacgagatg gacatctggg aggcgaacca gaacggcgcg 840

gcggtcacgc cgcacgtctg ctccgtcgac ggccagacgc gctgcgaggg cacggactgc 900

ggcgacggcg acgagcggta cgacggcatc tgcgacaagg acggctgcga cttcaactcg 960

taccgcatgg gcgaccagtc cttcctcggc ctcggcaaga ccgtcgacac ctcgaagaag 1020

ttcaccgtcg tcacccagtt cctcaccgcg gacaacacga cgaccggcca gctcacggag 1080

atccgccggc tgtacgtgca ggacggcaag gtcatcgcga actcgaagac gaacatcccc 1140

ggcctcgact cgttcgactc catcaccgac gacttctgca acgcgcagaa ggaggtcttc 1200

ggcgacacca actcgttcga gaagctcggc ggcctcgcgg agatgggcaa ggccttccag 1260

aagggcatgg tcctcgtcat gagcatctgg gacgaccacg ccgccaacat gctctggctc 1320
gacagcgact accccaccga cgccgacccg tcgaagccag gtgtcgcccg tggcccgtgc 1380
ccgaccagct ctggcgtccc caccgatgtc gagtcgcaga gccccaacgc gaacgtcatc 1440
ttctccaaca tcaagaccgg ccccattggc tcgacctacg cttga 1485

<210> 12
<211> 457
<212> PRT
<213> Coriolus hirsutus

<400> 12
Met Phe Pro Thr Ala Ala Leu Leu Ser Leu Ser Phe Ala Ala Ile Ala
1 5 10 15

Tyr Gly Gln Gln Val Gly Thr Leu Thr Ala Glu Ser His Pro Lys Leu
20 25 30

Ser Val Gln Gln Cys Thr Ala Gly Gly Ser Cys Gln Thr Leu Gln Arg
35 40 45

Ser Val Val Leu Asp Ser Asn Trp Arg Trp Leu His Ser Thr Ser Gly
50 55 60

Ser Thr Asn Cys Tyr Thr Gly Asn Thr Trp Asp Ala Ser Leu Cys Pro
65 70 75 80

Asp Pro Thr Thr Cys Ala Ala Asn Cys Ala Leu Asp Gly Ala Asp Tyr
85 90 95

Ser Gly Thr Tyr Gly Ile Thr Thr Ser Gly Asn Glu Leu Asn Leu Arg
100 105 110

Phe Val Thr Lys Gly Gln Tyr Ser Thr Asn Ile Gly Ser Arg Val Tyr
115 120 125

Leu Leu Ser Glu Asp Asp Ser Thr Tyr Glu Met Phe Asn Leu Asn Asn
130 135 140

Gln Glu Phe Thr Phe Asp Val Asp Met Ser Asn Leu Pro Cys Gly Leu
145 150 155 160

Asn Gly Ala Leu Tyr Phe Val Glu Met Asp Lys Asp Gly Gly Ser Ser
165 170 175

Arg Phe Pro Thr Asn Lys Ala Gly Ser Lys Tyr Gly Thr Gly Tyr Cys
180 185 190

Asp Thr Gln Cys Pro His Asp Ile Lys Phe Ile Asn Gly Glu Ala Asn
195 200 205

Val Leu Gly Trp Glu Gly Ser Pro Asn Asp Pro Asn Ala Gly Thr Gly
210 215 220

Gln Tyr Gly Thr Cys Cys Asn Glu Met Asp Ile Trp Glu Ala Asn Gln
225 230 235 240

Asn Gly Ala Ala Val Thr Pro His Val Cys Ser Val Asp Gly Gln Thr

245 250 255

Arg Cys Glu Gly Thr Asp Cys Gly Asp Gly Asp Glu Arg Tyr Asp Gly

260 265 270

Ile Cys Asp Lys Asp Gly Cys Asp Phe Asn Ser Tyr Arg Met Gly Asp

275 280 285

Gln Ser Phe Leu Gly Leu Gly Lys Thr Val Asp Thr Ser Lys Lys Phe

290 295 300

Thr Val Val Thr Gln Phe Leu Thr Ala Asp Asn Thr Thr Thr Gly Gln

305 310 315 320

Leu Thr Glu Ile Arg Arg Leu Tyr Val Gln Asp Gly Lys Val Ile Ala

325 330 335

Asn Ser Lys Thr Asn Ile Pro Gly Leu Asp Ser Phe Asp Ser Ile Thr

340 345 350

Asp Asp Phe Cys Asn Ala Gln Lys Glu Val Phe Gly Asp Thr Asn Ser

355 360 365

Phe Glu Lys Leu Gly Gly Leu Ala Glu Met Gly Lys Ala Phe Gln Lys

370 375 380

Gly Met Val Leu Val Met Ser Ile Trp Asp Asp His Ala Ala Asn Met

385 390 395 400

Leu Trp Leu Asp Ser Asp Tyr Pro Thr Asp Ala Asp Pro Ser Lys Pro
405 410 415

Gly Val Ala Arg Gly Pro Cys Pro Thr Ser Ser Gly Val Pro Thr Asp
420 425 430

Val Glu Ser Gln Ser Pro Asn Ala Asn Val Ile Phe Ser Asn Ile Lys
435 440 445

Thr Gly Pro Ile Gly Ser Thr Tyr Ala
450 455

<210> 13
<211> 21
<212> DNA
<213> Artificial Sequence

<220>
<223> Synthetic DNA

<400> 13
cagtggggig actggtgcaa c 21

<210> 14
<211> 1704
<212> DNA
<213> Coriolus hirsutus

<400> 14

atgtccaagt tcgcgacgct cctcgctctc ctgactgtcg tcccctccct ggcttacgcc 60
caggcgtcgc tgtacggaca gtgcggtggt attggcttct gtatgtctcg ttttcacgtg 120
cttctgtgag atgcgtactc accaccgtat cctcgcagcg ggtccgacga cttgtgttgc 180
cggcgccgtt tgcacgaagc agaacgacta ctactcgcag tgcctgtatg tcgtgtcaac 240
cagttatgag ataaagcaac ttgctaactt attacgacag tccgggcgcc gctgcaccga 300
ccactgttgc accgaccacc acacctaacg cgcccacatc tgcacctggt ggtggctcgc 360
ccacatcttc tgctccgagc gccccttcga gcactcctgc tgctggcaac cccttcgacg 420
gttttgaggt acagtgttct cgaacaccgc aaatcttcgc atgctcaccc atgtatacaa 480
cagatctacc tgagccctta ctacgccaag gaggtcgctg ctgccgctgc cgccatcacc 540
gatcccacgc tgaagagcaa ggccgcaagc gttgctaaca tcccgacttt cacatggctc 600
gactccgtgt cgaaggtgcc tgacctcggc acgtacctcg ccgacgcgtc ctcgatccag 660
agcagcaccg gcaagaagca gctcgtgccg atcgtcgtgt acgacctgcc cgaccgcgac 720
tgcgcggcga aggcgtccaa cggcgagttc agcatcgcgg acggcggcgc ggcgaagtac 780
aaggactaca tcgaccagat cgtcgcgcag atcaagcagt tccccgacgt gcgcgtcgtc 840
gcggtcatcg agcccgactc gctcgcgaac ctcgtcacga acctgaacgt gcagaagtgc 900
gcgaacgcgg aggcgacgta caaggccagc gttacgtacg cgctccagca gctctcgtcc 960
gtcggcgtgt accagtacat ggacgccggc cacgccggct ggctcggctg gcccgccaac 1020
atccagcccg cggcgaccct tttcgcggag atgttcaaga gcgcgaactc gtcgcctttc 1080
gtccgcggtc tcgccactag tgagcactca cctagacaca gcgatgtgga tggccactaa 1140
cggagcgtcg cagacgtcgc caactacaac gccctgaccg ccgcctcccc cgacccgatc 1200
acccagaaca accccaacta cgacgagtcc cactacatta acgtgagtcc ctcctgctca 1260
gcgctgttca gccacaatgc tcacattgca cgcacgcacc caggcgctcg gcccgatgct 1320
caagtccgcc ggcttccccg cgcagttcgt cgtcgaccag ggccgcgccg gccagcagaa 1380
cctccgccag cagtggggcg actggtgcaa catcaagggc gccggcttcg gcacgcgccc 1440
gacgacgaac accggcaacc cgctcatcga cgcgatcatc tgggtgaagc ccggaggcga 1500
gtccgacggc acctcgaaca gctcgtcgcc ccgctacgac agcacgtgct ctctggtacg 1560
tcttcctcgt cctcctgcat ctgggggtg cgtgtgctta ccactgcggt gcagtccgac 1620

gcgacggtcc ccgcacccga ggccggtacc tggttccagg cgtacttcga gaccctcgtg 1680

tccaaggcca acccgccgct gtga 1704

<210> 15

<211> 453

<212> PRT

<213> Coriolus hirsutus

<400> 15

Met Ser Lys Phe Ala Thr Leu Leu Ala Leu Leu Thr Val Val Pro Ser
1 5 10 15

Leu Ala Tyr Ala Gln Ala Ser Leu Tyr Gly Gln Cys Gly Gly Ile Gly
20 25 30

Phe Ser Gly Pro Thr Thr Cys Val Ala Gly Ala Val Cys Thr Lys Gln
35 40 45

Asn Asp Tyr Tyr Ser Gln Cys Leu Pro Gly Ala Ala Ala Pro Thr Thr
50 55 60

Val Ala Pro Thr Thr Thr Pro Asn Ala Pro Thr Ser Ala Pro Gly Gly
65 70 75 80

Gly Ser Pro Thr Ser Ser Ala Pro Ser Ala Pro Ser Ser Thr Pro Ala
85 90 95

Ala Gly Asn Pro Phe Asp Gly Phe Glu Ile Tyr Leu Ser Pro Tyr Tyr

100 105 110

Ala Lys Glu Val Ala Ala Ala Ala Ala Ala Ile Thr Asp Pro Thr Leu
115 120 125

Lys Ser Lys Ala Ala Ser Val Ala Asn Ile Pro Thr Phe Thr Trp Leu
130 135 140

Asp Ser Val Ser Lys Val Pro Asp Leu Gly Thr Tyr Leu Ala Asp Ala
145 150 155 160

Ser Ser Ile Gln Ser Ser Thr Gly Lys Lys Gln Leu Val Pro Ile Val
165 170 175

Val Tyr Asp Leu Pro Asp Arg Asp Cys Ala Ala Lys Ala Ser Asn Gly
180 185 190

Glu Phe Ser Ile Ala Asp Gly Gly Ala Ala Lys Tyr Lys Asp Tyr Ile
195 200 205

Asp Gln Ile Val Ala Gln Ile Lys Gln Phe Pro Asp Val Arg Val Val
210 215 220

Ala Val Ile Glu Pro Asp Ser Leu Ala Asn Leu Val Thr Asn Leu Asn
225 230 235 240

Val Gln Lys Cys Ala Asn Ala Glu Ala Thr Tyr Lys Ala Ser Val Thr
245 250 255

Tyr Ala Leu Gln Gln Leu Ser Ser Val Gly Val Tyr Gln Tyr Met Asp
260 265 270

Ala Gly His Ala Gly Trp Leu Gly Trp Pro Ala Asn Ile Gln Pro Ala
275 280 285

Ala Thr Leu Phe Ala Glu Met Phe Lys Ser Ala Asn Ser Ser Pro Phe
290 295 300

Val Arg Gly Leu Ala Thr Asn Val Ala Asn Tyr Asn Ala Leu Thr Ala
305 310 315 320

Ala Ser Pro Asp Pro Ile Thr Gln Asn Asn Pro Asn Tyr Asp Glu Ser
325 330 335

His Tyr Ile Asn Ala Leu Gly Pro Met Leu Lys Ser Ala Gly Phe Pro
340 345 350

Ala Gln Phe Val Val Asp Gln Gly Arg Ala Gly Gln Gln Asn Leu Arg
355 360 365

Gln Gln Trp Gly Asp Trp Cys Asn Ile Lys Gly Ala Gly Phe Gly Thr
370 375 380

Arg Pro Thr Thr Asn Thr Gly Asn Pro Leu Ile Asp Ala Ile Ile Trp
385 390 395 400

Val Lys Pro Gly Gly Glu Ser Asp Gly Thr Ser Asn Ser Ser Ser Pro
405 410 415

Arg Tyr Asp Ser Thr Cys Ser Leu Ser Asp Ala Thr Val Pro Ala Pro
420 425 430

Glu Ala Gly Thr Trp Phe Gln Ala Tyr Phe Glu Thr Leu Val Ser Lys
435 440 445

Ala Asn Pro Pro Leu
450

<210> 16
<211> 17
<212> DNA
<213> Artificial

<220>
<223> Synthetic DNA

<400> 16
gtaaaacgac ggccagt 17

<210> 17
<211> 19
<212> DNA
<213> Artificial

<220>

<223> Synthetic DNA

<400> 17

ggaaacagct atgaccatg 19

<210> 18

<211> 1327

<212> cDNA

<213> Coriolus hirsutus

<400> 18

aagcactcta ttgacaccgt catgttctcg tctaccctct ccttcgccgc cctcgcgctc 60
gcgctcgtcg cgcccactgc cgtcaacgct cacggtttca ttcatgaata tgagatcggc 120
ggcaagagct actccggttg gctcccgttc tcggacccct acgagagccc tgtcccgagc 180
cgcatcgagc gcaagatccc gagtgacggc cctatccttg atgtcacttc tcctgacctt 240
gcctgcaaca aggggggcga gtctggcgtc aaggccatcg ccactgcggc agcaggcagc 300
cagatcacct ttgactggaa cagttggccc gcagatcaca tgggcccggt gaccacatac 360
atggcgtctt gcaacggtga ttgcgcgtct ttcgatgcct ccaacgcgaa gtggttcaag 420
attgacgctg ccggctactc gaacggcaag tgggctgcca ccaagctcat tgagaacggc 480
gccaagtgga ccagcaccat tcccagcgag ctcaaggctg gtgaatactt ggtccgtcat 540
gagatcattg ctctccacga cgccggtgcg cctcagttct accccagctg cgctcaggtg 600
aaggtcactg gtggtggtag ccaggttccc tctggttcct ccctcgtgtc cattccgggg 660
ctctacacca ttcaggagtt cccgacatct ggtccgacag cttcaagagc tttgccattc 720
ctggacccgc ggtcgccttc agtggctcca acagcggctc tggcgattct cagcctgctg 780
cctcctcctc tacccacgcc gctacttctt cggcggcctc ccagtctgcg tcctcgacgc 840
aggttcacac ctccgcggag acctccgcgc aggcctcggc gacgtctgtt gcgtcccacg 900
catcttccgc tgcccacact tcctcggccg catccgcgtc gaagccctcg tcgacgggga 960

ccggaaggtg ctcctctaag cgcactcgcc gcggcatggt caagcgcaac gtctctcacc 1020

acgccaagcg ccaccaccat tgatttctct ttcttccttg cgctcttggc tgtctcgaga 1080

tctcgatatg cttcagagaa gcactggtcg acgggatctc aatcgatgtt gatacagatg 1140

ggttgactcc cctccgcgct ctgctcccac cgcgccgggg atagagtcct cgcgcgcggc 1200

ttccttaacg ttattcattc ctcgctccgc ataagtctcc gcatgctatg tatcggtgct 1260

gctagcccgc acgactgccc gacgattgta ccggaataca acgcgctttg tcctttgtga 1320

aaaaaaa 1327

<210> 19

<211> 374

<212> PRT

<213> Coriolus hirsutus

<400> 19

Met Phe Ser Ser Thr Leu Ser Phe Ala Ala Leu Ala Leu Ala Leu Val
1 5 10 15

Ala Pro Thr Ala Val Asn Ala His Gly Phe Ile His Glu Tyr Glu Ile
20 25 30

Gly Gly Lys Ser Tyr Ser Gly Trp Leu Pro Phe Ser Asp Pro Tyr Glu
35 40 45

Ser Pro Val Pro Ser Arg Ile Glu Arg Lys Ile Pro Ser Asp Gly Pro
50 55 60

Ile Leu Asp Val Thr Ser Pro Asp Leu Ala Cys Asn Lys Gly Gly Glu
65 70 75 80

Ser Gly Val Lys Ala Ile Ala Thr Ala Ala Ala Gly Ser Gln Ile Thr
85 90 95

Phe Asp Trp Asn Ser Trp Pro Ala Asp His Met Gly Pro Val Thr Thr
100 105 110

Tyr Met Ala Ser Cys Asn Gly Asp Cys Ala Ser Phe Asp Ala Ser Asn
115 120 125

Ala Lys Trp Phe Lys Ile Asp Ala Ala Gly Tyr Ser Asn Gly Lys Trp
130 135 140

Ala Ala Thr Lys Leu Ile Glu Asn Gly Ala Lys Trp Thr Ser Thr Ile
145 150 155 160

Pro Ser Glu Leu Lys Ala Gly Glu Tyr Leu Val Arg His Glu Ile Ile
165 170 175

Ala Leu His Asp Ala Gly Ala Pro Gln Phe Tyr Pro Ser Cys Ala Gln
180 185 190

Val Lys Val Thr Gly Gly Gly Ser Gln Val Pro Ser Gly Ser Ser Leu
195 200 205

Val Ser Ile Pro Gly Leu Tyr Thr Ile Gln Glu Phe Pro Thr Ser Gly
210 215 220

Pro Thr Ala Ser Arg Ala Leu Pro Phe Leu Asp Pro Arg Ser Pro Ser

225 230 235 240

Val Ala Pro Thr Ala Ala Leu Ala Ile Leu Ser Leu Leu Pro Pro Pro

245 250 255

Leu Pro Thr Pro Leu Leu Leu Arg Arg Pro Pro Ser Leu Arg Pro Arg

260 265 270

Arg Arg Phe Thr Pro Pro Arg Arg Pro Pro Arg Arg Pro Arg Arg Arg

275 280 285

Leu Leu Arg Pro Thr His Leu Pro Leu Pro Thr Leu Pro Arg Pro His

290 295 300

Pro Arg Arg Ser Pro Arg Arg Arg Gly Pro Glu Gly Ala Pro Leu Ser

305 310 315 320

Ala Leu Ala Ala Ala Trp Ser Ser Ala Thr Ser Leu Thr Thr Pro Ser

325 330 335

Ala Thr Thr Ile Asp Phe Ser Phe Phe Leu Ala Leu Leu Ala Val Ser

340 345 350

Arg Ser Arg Tyr Ala Ser Glu Lys His Trp Ser Thr Gly Ser Gln Ser

355 360 365

Met Leu Ile Gln Met Gly

370 374

<210> 20
<211> 735
<212> DNA
<213> Coriolus hirsutus

<400> 20
gcgggcccgt actcgctcct gctcgaccag tggggcaagg acggcgcgac gtccggctcc 60
caatgcgcga acctcatcag cctgagcggc agtaccgtcg cgtggaagac gacctggcag 120
tggacgggcg gctccggcgt gaagagcttc acgaacatcc agctcaacga gggcctcaac 180
aagcagctca gcgcgatcaa gagcatcccc acgacgtggc agtggtcgca gagcgcgtcc 240
gggtcgatcg tcgcggacgt cgcgtacgac ctcttcacgg cgaacaccgc cgggggctcg 300
aacgtgaacg agatcatgat ctggctcgcg aacttcaacg cgggcccgat ctcgatccag 360
tacggcgcgg acggcaagcc cgtgcccgtc gcgtcgaacc tgagcctcgc gggccacacc 420
tggaacctgt acagcggctc gaacggcgcg aacgcggtgt tctcgttcct gcccacgagc 480
ggcacgatta cgagcttcag cggggacgtg aacgtgttcc tccagtactt gacgcagcac 540
cagggcgtca gcacctcgca gttcctcgtc accgcgcaag cgggtacgga gcctacatct 600
ggctctgcga cgctcacgac gtctgcatac agcttggcta tcaactaggg agacgaaaca 660
tgtacattca gaacttgtgc cgacaggaat cacattactt cagacttccc gaaaaaaaaa 720
aaaaaaaaaa aaaaa 735

<210> 21
<211> 216
<212> PRT
<213> Coriolus hirsutus

<400> 21
Ala Gly Pro Tyr Ser Leu Leu Leu Asp Gln Trp Gly Lys Asp Gly Ala

1 5 10 15

Thr Ser Gly Ser Gln Cys Ala Asn Leu Ile Ser Leu Ser Gly Ser Thr

20 25 30

Val Ala Trp Lys Thr Thr Trp Gln Trp Thr Gly Gly Ser Gly Val Lys

35 40 45

Ser Phe Thr Asn Ile Gln Leu Asn Glu Gly Leu Asn Lys Gln Leu Ser

50 55 60

Ala Ile Lys Ser Ile Pro Thr Thr Trp Gln Trp Ser Gln Ser Ala Ser

65 70 75 80

Gly Ser Ile Val Ala Asp Val Ala Tyr Asp Leu Phe Thr Ala Asn Thr

85 90 95

Ala Gly Gly Ser Asn Val Asn Glu Ile Met Ile Trp Leu Ala Asn Phe

100 105 110

Asn Ala Gly Pro Ile Ser Phe Gln Tyr Gly Ala Asp Gly Lys Pro Val

115 120 125

Pro Val Ala Ser Asn Leu Ser Leu Ala Gly His Thr Trp Asn Leu Tyr

130 135 140

Ser Gly Ser Asn Gly Ala Asn Ala Val Phe Ser Phe Leu Pro Thr Ser

145 150 155 160

Gly Thr Ile Thr Ser Phe Ser Gly Asp Val Asn Val Phe Leu Gln Tyr

165 170 175

Leu Thr Gln His Gln Gly Val Ser Thr Ser Gln Phe Leu Val Thr Ala

180 185 190

Gln Ala Gly Thr Glu Pro Thr Ser Gly Ser Ala Thr Leu Thr Thr Ser

195 200 205

Ala Tyr Ser Leu Ala Ile Asn Xaa

210 215

<210> 22

<211> 21

<212> DNA

<213> Artificial

<220>

<223> Synthetic DNA

<400> 22

atgaagttac ttcttgctct c 21

<210> 23

<211> 21

<212> DNA

<213> Artificial

<220>

<223> Synthetic DNA

<400> 23

tcacaggaag ggttcgagtg c 21

<210> 24

<211> 1889

<212> DNA

<213> Phanerochaete chrysosporium

<400> 24

atgaagtact tcttgctctc agtagcagcg acgcttgccc ttagtgcacc tgcgcttggt 60
gtcgctgtct ggggccaatg cggtgtaagc atatgtgttc atatgagagg cgtacgagaa 120
ggttgaccgc gcaacaggga atcggcttta gtgggagtac tacttgcgat gctggcaatc 180
actgcgttta tctgaacgat tgtgaggtca atgtcatttc aatgccatga tttgctgacc 240
gatatccttg cagattattc gcaatgccag cccggcgcgg cgacgaccac ggtccggtcg 300
acaagtacca ttgcttcaac gacatctacc gcaccatcaa gcagcaatag cctttgctcc 360
ggcagccgca cgaagttcaa gtttttcggt gtcaatgagt ctggtgctga gttcggtaat 420
ctcaacatcc caggtgtcct aggcacggac tacacctggc cttcgccgtc cagcatcgac 480
gtaagtgata catcatatca gagctgctaa gcaggttgct gatagtgatg cactagttct 540
tcatgggcaa gggattcaac accttccgca ttccgttcct catggagcgt atgagccctc 600
ccgcgacggg cctcaccggt ccgtttgatc agacctatct gagcggtctt cagacggtac 660
gccaaacgta atagctggac cttgcgggga gtaaggctga ccatcacctt acggcagatt 720
gtcagctata tcaccggaaa ggggggctat gcgctcgtag accgtgagtg acggctcgca 780
cgcaagtagt aggaggtcat ctgatagtga aggactgcag cgcacaactt tatgatctac 840
aatggcacaa ccattagcga cacaaacgcg tgagcactcc tttagtttcg tctctgcgtt 900
agctccgttc tgatgataca acaacacagc ttccagactt gtacgtgtgc tctgcctgaa 960
cccgcttggc cgttgatcat catagtgatg cgttctcaca gggtggcaca acctcgccac 1020
cgtcttcgta cgtctgtttc catgcgtgaa gcctgcaagg gtcgcccctg atcggtttta 1080
tagaaatcca accccaatgt cgtctttggt gaatgagttc ggcgtgtgtg ctactcttag 1140
caacgagctt acagcttggc agatgtcatg aacgagccgc acgacattcc cgcacagacg 1200
gtcttcaacc tggtgagttg cggccgagtt tggggacttc tgtcaaactc attcggcgtt 1260
gtttccccct agaaccaagc cgctatcaac ggaatccgtg ctgcgggtgc cacctcgcaa 1320
cttatccttg ttgagggcac tagctacact ggcgcctgga gtgagatcag acccttcaca 1380
cccgcagaac ctgcccattg attgattgtt tcgcagcgtg gacgacttct tccggtaatg 1440
gtgctgtctt tggtgctatc caagatccca acaacaatgt tgccatcggt gagtgcggga 1500
agcgtaccgg tctgccaagt gctcacgggt aagcgtggga aagagatgca ccagtacctc 1560
gacagcgacg gctccggcac gtcccccaca tgcgtttcgt ccacgatcgg tgcggagcgt 1620
ctccaagcgg cgacacagtg gctgcagcag aacaacctga aaggcttcct gggtgagatc 1680

ggagctggtt cgaacggtgc gtcggatcat gtcctgtgtt gcagcctgtg ctgaccgtag 1740

tcgttcaaac cagccgactg catcagtgcc gttcagggtg cgctgtgcga gatgcaacag 1800

tccggtgtct ggctcggcgc tctctggtgg gccgcaggcc catggtgggg cacggtacgt 1860

gactgacctc ttgctgcgtg tgcacgtgca gactgctcat tgctgccaca gtatttccaa 1920

tcgatcgagc ccccgagtgg cgccgcgatc ccctccatcc tcccgcaggc actcgaaccc 1980

ttcctgtga 1989

<210> 25

<211> 386

<212> PRT

<213> Phanerochaete chrysosporium

<400> 25

Met Leu Lys Tyr Ala Ser Ile Ala Leu Ala Leu Ala Thr Leu Gly Val
1 5 10 15

Ala Gln Gln Gln Gln Trp Gly Gln Cys Gly Gly Ile Gly Trp Thr Gly
20 25 30

Ala Thr Thr Cys Val Ala Gly Ser Val Cys Ser Val Leu Asn Pro Tyr
35 40 45

Tyr Ser Gln Cys Ile Pro Gly Ala Ala Thr Val Thr Ser Ser Ser Ala
50 55 60

Pro Ser Thr Pro Thr Pro Pro Ala Gly Ala Leu Pro Arg Leu Gly Gly
65 70 75 80

Val Asn Thr Ala Gly Tyr Asp Phe Ser Val Ala Thr Asp Gly Ser Phe
85 90 95

Thr Gly Thr Gly Val Ser Pro Pro Val Ser Gln Phe Ser His Phe Ser
100 105 110

Ser Gln Gly Ala Asn Leu Tyr Arg Ile Leu Phe Ala Trp Gln Leu Met
115 120 125

Thr Pro Thr Leu Gly Gly Thr Ile Ser Gln Ser Phe Leu Ser Arg Tyr
130 135 140

Asp Gln Thr Val Gln Ala Ala Leu Asn Ser Gly Pro Asn Val Phe Val
145 150 155 160

Ile Ile Asp Leu His Asn Tyr Ala Arg Trp Asn Gly Gly Ile Ile Ala
165 170 175

Gln Gly Gly Pro Thr Asp Ala Gln Phe Gln Ser Ile Trp Thr Gln Leu
180 185 190

Ala Gln Lys Tyr Gly Ser Asn Gln Arg Val Ile Phe Gly Ile Met Asn
195 200 205

Glu Pro His Asp Ile Pro Ser Ile Ser Thr Trp Val Asn Ser Val Gln
210 215 220

Gly Ala Val Asn Ala Ile Arg Ala Ala Gly Ala Thr Asn Tyr Leu Leu
225 230 235 240

Leu Pro Gly Ser Ser Trp Ser Ser Ala Gln Ala Phe Pro Thr Glu Ala
245 250 255

Gly Pro Leu Leu Val Lys Val Thr Asp Pro Leu Gly Gly Thr Ser Lys
260 265 270

Leu Ile Phe Asp Val His Lys Tyr Leu Asp Ser Asp Asn Ser Gly Thr
275 280 285

His Pro Asp Cys Thr Thr Asp Asn Val Gln Val Leu Gln Thr Leu Val
290 295 300

Gln Phe Leu Gln Ala Asn Gly Asn Arg Gln Ala Ile Leu Ser Glu Thr
305 310 315 320

Gly Gly Gly Asn Thr Ser Ser Cys Glu Ser Leu Leu Ala Asn Glu Leu
325 330 335

Ala Tyr Val Lys Ser Ala Tyr Pro Thr Leu Ala Gly Phe Ser Val Trp
340 345 350

Ala Ala Gly Ala Phe Asp Thr Thr Tyr Val Leu Thr Val Thr Pro Asn
355 360 365

Ala Asp Gly Ser Asp Gln Pro Leu Trp Val Asp Ala Val Lys Pro Asn
370 375 380

Leu Pro

385

<210> 26

<211> 20

<212> DNA

<213> Artificial Sequence

<220>

<223> Synthetic DNA

<400> 26

atgatacctc tccgctctgc 20

<210> 27

<211> 20

<212> DNA

<213> Artificial Sequence

<220>

<223> Synthetic DNA

<400> 27

tatcttcctg atgcgattcc 20

<210> 28

<211> 2138

<212> DNA

<213> Phanerochaete chrysosporium

<400> 28

atgatacctc tccgctctgc agtcgcgtcc tcactacttc tcgccagtct cggggctgcc 60
cagctcccgc tgccgaaccc tccgtgggtg ccgccgaacg caacgttcgg gacgcaccct 120
agcaatcctt ctgacgggtc cgggaacccg cactggacga atatcctcgg gagcacgctc 180
tacttctatg aagaacagcg gagcggaaag ctgcccgtta cgaaccgcgt cccgtggcga 240
aacgatagtg cgacggacga cggcagggac gtcggactgg acctgagtgg cggctactat 300
gatgctggag gttcgtattt gagtacgctc tcccgagagg tcgtctaacc ttccggtgta 360
gactacatca agtatacctt tcctatggtg cgtaacacgc agcgtactgg ttctgaaagc 420
taatgtcaat ggcgacccag tccttctctg ttatgtctat atgctggggc gcgctggatt 480
atggcaaggg taaggcaaga atttctcgtt cgctgccctc cattgaaacc cgtctgcgtt 540
aggttatgac ctcgcaaacc aaaccgcgta tctcgacgac atgctccggt ggagcctaga 600
ctggctaatg aaggtatggt caaggatata cttgcatcga cttacctact tgtctgacga 660
tgcatgtcaa ggcccatcct gacccaaaca cgctgtatgt acaaatcggg gatgcagatc 720
tggacaacgc atattgggga ggcgacagag gcatacctac accaagaact tcatatgcaa 780
tcaacagtac caggtgcgcg ctttgtcgcg ttatcctcta ggcaatacta agattttgct 840
tagccccggt acagacgccg ccgcacaagc cgccgcggct ttcgctgcct gttctgcatt 900
atacaacaat cgaacattgt cgcagcccgc acctaacggt ataacaaata catcttacgc 960
atcgacgctc cttcagcatg cgcaacaact gtatcatttt gccacaaact cttctatacc 1020
tcaagttacc taccagacgt ccgtgccgtc ggtcgccgac gcatacgcat cctccggatt 1080
ccaagacgag ctagccatcg cggctctgtt tatctcgctt gcaggaaatt ctagcgacgc 1140
ttactcgcaa gcgtctcagc tttaccgcaa gcaagggctt tctaagcact tggaagatga 1200
cgctgtgttc aactgggatg agaagtcacc cggcgtcgcc cttcttgcgg cgcagattgc 1260
gcagaagtat cctcagctcg cgaacggcac tggtgtggat tggaagagcg acctgaacaa 1320
ctacttcgat cgcatcgtca acaacagtgg cagatcattc ctaacatcag gtaagcgtgt 1380
caatctctta tcccagttct tcgtgctgac agttcgctct aggcggtctg ctttattatc 1440
caggcgattc agacgacgct acccttaacc cggcattgaa cgctgccatg ctcctcttgc 1500

gctatgccga ctcgggcttt gcatccagca gtgagaagca gtctgtttac cgccagttcg 1560
cccagtctca aatcgactat ttcttgggca ataacccaat gactggtggg tagtgttttc 1620
ttgacattat gtctatccgc gtcttactcg ttgcagtacc gtatatggtt ggcgtgcacc 1680
cgaacgcgcc atcaaaccct cattctgcct tggctacggg tgcttcaccc caggacatcg 1740
cgaacatcga cacggtccca gagcacgagg cttacgtcct ttatggcgga gtagttggag 1800
ggccgaatga tgacgacctc ttttgggacc tacggagcga ttgggtggag aacgaggttg 1860
ggctggacta cgttgccccg gtcgtgacca tcgcggcgcg ggaactcgtc agtggagcag 1920
gcgacccttg gtacacacag ctgcaggttg ggtcgtacga ggagcggaga ccgggtggcc 1980
agccttgtga tgctgcaatc tctgcgggat gtcgcggcca tgattggaga gtgggcaaga 2040
tcgtcatggg tgtcttggtc ggagtgactg gtctcgtggt attgtctctt ggtaccgtgt 2100
ggatggtgtt ggcatatagg aatcgcatca ggaagata 2138

<210> 29
<211> 592
<212> PRT
<213> Phanerochaete chrysosporium

<400> 29

Met Ile Pro Leu Arg Ser Ala Val Ala Ser Ser Leu Leu Leu Ala Ser
1 5 10 15

Leu Gly Ala Ala Gln Leu Pro Leu Pro Asn Pro Pro Trp Val Pro Leu
20 25 30

Asn Ala Thr Phe Gly Thr His Pro Ser Asn Pro Ser Asp Gly Ser Gly
35 40 45

Asn Pro His Trp Thr Asn Phe Leu Glu Asn Thr Leu Tyr Phe Tyr Glu
50 55 60

Glu Gln Arg Ser Gly Lys Leu Pro Val Thr Asn Arg Val Pro Trp Arg
65 70 75 80

Asn Asp Ser Ala Thr Asp Asp Gly Arg Asp Val Gly Leu Asp Leu Ser
85 90 95

Gly Gly Tyr Tyr Asp Ala Gly Asp Tyr Ile Lys Tyr Thr Phe Pro Met
100 105 110

Ser Phe Ser Val Met Ser Ile Cys Trp Gly Ala Leu Asp Tyr Gly Lys
115 120 125

Gly Tyr Asp Leu Ala Asn Gln Thr Ala Tyr Leu Asp Asp Met Leu Arg
130 135 140

Trp Ser Leu Asp Trp Leu Met Lys Ala His Pro Asp Pro Asn Thr Leu
145 150 155 160

Tyr Val Gln Val Gly Asp Ala Asp Leu Asp Asn Ala Tyr Trp Gly Gly
165 170 175

Asp Arg Gly Ile Pro Thr Pro Arg Thr Ser Tyr Ala Ile Asn Ser Thr
180 185 190

Ser Pro Gly Thr Asp Ala Ala Ala Gln Ala Ala Ala Ala Phe Ala Ala
195 200 205

Cys Ser Ala Leu Tyr Asn Asn Arg Thr Leu Ser Gln Pro Ala Pro Asn
210 215 220

Gly Ile Thr Ser Thr Ser Tyr Ala Ser Thr Leu Leu Gln His Ala Gln
225 230 235 240

Gln Leu Tyr Asn Phe Ala Thr Asn Ser Ser Val Pro Gln Val Thr Tyr
245 250 255

Gln Ala Ser Glu Pro Ser Val Ala Asp Ala Tyr Ala Ser Ser Gly Phe
260 265 270

Gln Asp Glu Leu Ala Ile Ala Ala Leu Phe Ile Ser Leu Ala Gly Asn
275 280 285

Ser Ser Asp Ala Tyr Pro Gln Ala Ser Gln Val Tyr Arg Lys Gln Gly
290 295 300

Leu Ser Lys His Leu Glu Asp Asp Ala Val Phe Asn Trp Asp Glu Lys
305 310 315 320

Ser Pro Gly Val Ala Leu Leu Ala Ala Gln Ile Ala Gln Lys Tyr Pro
325 330 335

Glu Leu Ala Asn Gly Thr Gly Val Asp Trp Lys Ser Asp Leu Asn Asn
340 345 350

Tyr Phe Asp Arg Ile Val Ser Asn Ser Gly Arg Ser Phe Leu Thr Ser

355 360 365

Gly Gly Leu Leu Tyr Tyr Pro Gly Asp Ser Asp Asp Ala Thr Leu Asn

370 375 380

Pro Ala Leu Asn Ala Ala Met Leu Leu Leu Arg Tyr Ala Asp Ser Gly

385 390 395 400

Leu Ala Ser Ser Ser Glu Lys Gln Ser Ala Tyr Arg Gln Phe Ala Gln

405 410 415

Ser Gln Ile Asp Tyr Phe Leu Gly Asn Asn Pro Met Thr Val Gln Tyr

420 425 430

Met Val Gly Val His Pro Asn Ala Pro Ser Asn Pro His Ser Ala Leu

435 440 445

Ala Thr Gly Ala Thr Pro Gln Asp Ile Ala Asn Ile Asp Thr Val Pro

450 455 460

Glu His Glu Ala Tyr Val Leu Tyr Gly Gly Val Val Gly Gly Pro Asn

465 470 475 480

Asp Asp Asp Leu Phe Trp Asp Leu Arg Ser Asp Trp Val Glu Ser Glu

485 490 495

Val Gly Leu Asp Tyr Val Ala Pro Val Val Thr Ile Ala Ala Arg Glu

500 505 510

Leu Val Ser Gly Ala Gly Asp Pro Trp Tyr Thr Gln Leu Gln Ala Gly
515 520 525

Ser Tyr Glu Glu Arg Arg Pro Gly Gly Gln Pro Cys Asp Ala Ala Ile
530 535 540

Ser Ala Gly Cys Arg Gly His Asp Trp Arg Val Gly Lys Ile Val Met
545 550 555 560

Gly Ala Leu Val Gly Val Thr Gly Leu Val Val Leu Ser Leu Gly Thr
565 570 575

Val Trp Met Val Leu Ala Tyr Arg Asn Arg Ile Arg Lys Ile
580 585 590

<210> 30
<211> 18
<212> DNA
<213> Artificial

<220>
<223> Synthetic DNA

<400> 30
catggtgtgt ggtggatg 18

<210> 31

<211> 19

<212> DNA

<213> Artificial

<220>

<223> Synthetic DNA

<400> 31

aagttcaaga gtctcctgt 19

<210> 32

<211> 20

<212> DNA

<213> Artificial

<220>

<223> Synthetic DNA

<400> 32

ggtacagtac ttatctgtat 20

<210> 33

<211> 30

<212> DNA

<213> Artificial

<220>

<223> Synthetic DNA

<400> 33
tctagattta ctggtacccc aacaacaatg 30

<210> 34
<211> 31
<212> DNA
<213> Artificial

<220>
<223> Synthetic DNA

<400> 34
ccatgggttg atcgacgggt tgtcagacac g 31

<210> 35
<211> 17
<212> DNA
<213> Artificial

<220>
<223> Synthetic DNA

<400> 35
tctagagtca cctccgt 17

<210> 36

<211> 16

<212> DNA

<213> Artificial

<220>

<223> Synthetic DNA

<400> 36

aagcttgggt actgtg 16

<210> 37

<211> 24

<212> DNA

<213> Artificial

<220>

<223> Synthetic DNA

<400> 37

tctagagcca acctcgaggg gtgg 24

<210> 38

<211> 24

<212> DNA

<213> Artificial

<220>

<223> Synthetic DNA

<400> 38

ccatgggaac gtcgagccga tggg 24

<210> 39

<211> 24

<212> DNA

<213> Artificial

<220>

<223> Synthetic DNA

<400> 39

tctagagcca acgtcctcgg ctgg 24

<210> 40

<211> 24

<212> DNA

<213> Artificial

<220>

<223> Synthetic DNA

<400> 40

ccatgggtag gtcgagccga tggg 24

<210> 41

<211> 24

<212> DNA

<213> Artificial

<220>

<223> Synthetic DNA

<400> 41

tctagagcca acgtcctcgg ctgg 24

<210> 42

<211> 25

<212> DNA

<213> Artificial

<220>

<223> Synthetic DNA

<400> 42

ccatggagcg taggtcgagc caatg 25

<210> 43

<211> 24

<212> DNA

<213> Artificial

<220>

<223> Synthetic DNA

<400> 43

tctagaatct acctgagccc ttac 24

<210> 44

<211> 24

<212> DNA

<213> Artificial

<220>

<223> Synthetic DNA

<400> 44

ccatggctca ctagtggcga gacc 24

<210> 45

<211> 25

<212> DNA

<213> Artificial

<220>

<223> Synthetic DNA

<400> 45
tctagagctc acggtttcat tcatg 25

<210> 46
<211> 25
<212> DNA
<213> Artificial

<220>
<223> Synthetic DNA

<400> 46
ccatggggtg tagagccccg gaatg 25

<210> 47
<211> 24
<212> DNA
<213> Artificial

<220>
<223> Synthetic DNA

<400> 47
tctagagcgg gcccgtactc gctc 24

<210> 48

<211> 24

<212> DNA

<213> Artificial

<220>

<223> Synthetic DNA

<400> 48

ccatgggtaa tgtgattcct gtcg 24

<210> 49

<211> 24

<212> DNA

<213> Artificial

<220>

<223> Synthetic DNA

<400> 49

tctagaatga agtacttctt gctc 24

<210> 50

<211> 24

<212> DNA

<213> Artificial

<220>

<223> Synthetic DNA

<400> 50
ccatggcgtt tggcgtaccg tctg 24

<210> 51
<211> 24
<212> DNA
<213> Artificial

<220>
<223> Synthetic DNA

<400> 51
tctagacccc ggtacagacg ccgc 24

<210> 52
<211> 24
<212> DNA
<213> Artificial

<220>
<223> Synthetic DNA

<400> 52
ccatgggatg ttaggaatga tctg 24